# EUROPEAN PATENT APPLICATION

(11) **EP 4 545 534 A1**
(43) Date of publication of application: **30.04.2025**
(21) Application number: 23826517.7
(22) Date of filing: 21.06.2023
(51) Int. Cl.: C07D 495/04, C07D 495/14, C07D 493/04, C07D 417/04, C07D 413/04, C07D 409/04, A61K 31/381, A61P 25/18, A61P 25/22, A61P 25/28

(54) **HETEROCYCLIC COMPOUND AS TAAR1 LIGAND AGONIST**

(30) Priority: 24.06.2022 CN 202210723387; 09.02.2023 CN 202310089008
(71) Applicant: Shandong Luye Pharmaceutical Co., Ltd., Yantai, Shandong 264670 (CN)
(72) Inventor: LU, Jing, Yantai, Shandong 264670 (CN); ZHANG, Jianzhao, Yantai, Shandong 264670 (CN); YE, Liang, Yantai, Shandong 264670 (CN); ZHENG, Lixia, Yantai, Shandong 264670 (CN); DAI, Yusen, Yantai, Shandong 264670 (CN); WANG, Wenyan, Yantai, Shandong 264670 (CN); TIAN, Jingwei, Yantai, Shandong 264670 (CN); WANG, Yunjie, Yantai, Shandong 264670 (CN); YU, Pengfei, Yantai, Shandong 264670 (CN); LEI, Hui, Yantai, Shandong 264670 (CN); WANG, Lin, Yantai, Shandong 264670 (CN)
(74) Representative: karo IP
(86) International application number: PCT/CN2023/101737
(87) International publication number: WO 2023/246872

(57) **Abstract**

Provided are a heterocyclic compound used as a TAAR1 agonist, a preparation method of the compound, a pharmaceutical composition containing the compound, and a use of the compound as a TAAR1 agonist in preventing and/or treating various CNS-related diseases. Each substituent in the general formula (IA) is the same as defined in the description.

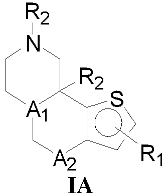

## Description

### TECHNICAL FIELD

The present disclosure relates to a heterocyclic compound and a preparation method therefor, as well as use thereof as a TAAR1 agonist for the treatment of related diseases.

### BACKGROUND

The trace amine-associated receptors (TAARs) are a family of endogenous biogenic amine-activated G protein-coupled receptors, and 6 TAARs and 3 pseudogenes have been identified in humans. Of all TAARs, TAAR1 is most studied, and is widely expressed in the mammalian brain, particularly in the limbic and monoaminergic regions. TAAR1 can modulate presynaptic dopaminergic neurotransmission function, and can also induce the activation of G protein-coupled inwardly rectifying potassium ion channel and reduce the discharge frequency of neuron. TAAR1 may be a potential therapeutic target for treating psychiatric diseases such as schizophrenia, depression, Parkinson's disease psychosis, behavioral and psychological symptoms of dementia, anxiety, migraine and attention deficit and hyperactivity disorder (ADHD).

At present, there are 4 drugs acting on TAAR1, that is, amphetamine, RG-7906, RO-5263397, and SEP-363856. Amphetamine was approved by the FDA for the treatment of attention deficit and hyperactivity disorder (adhd) in 2016, but this compound has strong excitability to the central nervous system. Administering it in the short term may cause fast heart beat, increased blood pressure, mood swings, and reduced fatigue, while in the long term, may cause adverse reactions such as insomnia, restlessness, delusion and hallucination, violent and assaultive actions, thus being classified as a controlled drug. RG-7906, RO-5263397, and SEP-363856 under development are all TAAR1 selective agonists, where RG-7906 is in phase II clinical trials, RO-5263397 is in phase I pause, and the most rapidly progressing SEP-363856 is in phase III clinical trials. Therefore, there is a need to develop a new TAAR1 agonist that meet patient needs with better safety and efficacy.

### SUMMARY

In one embodiment of the present disclosure, provided is a compound of formula (IA), a pharmaceutically acceptable salt thereof or a stereoisomer thereof: 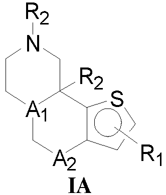 wherein,
A₁ is selected from the group consisting of CR₂ or N;
A₂ is selected from the group consisting of CR₂ or O;
R₁ is selected from the group consisting of H, halogen, C₁₋₆ alkyl, or C₁₋₆ alkoxy;
each R₂ is independently selected from the group consisting of H or C₁₋₆ alkyl.

In some embodiments of the present disclosure, in the compound of formula (IA), the pharmaceutically acceptable salt thereof or the stereoisomer thereof,
A₁ is selected from the group consisting of CR₂ or N;
A₂ is selected from the group consisting of CR₂ or O;
R₁ is selected from the group consisting of H, F, Cl, Br, I, methyl, ethyl, n-propyl, isopropyl, n-butyl, isobutyl, s-butyl, t-butyl, methoxy, ethoxy, n-propoxy, or isopropoxy;
each R₂ is independently selected from the group consisting of H, methyl, or ethyl.

In some embodiments of the present disclosure, in the compound of formula (IA), the pharmaceutically acceptable salt thereof or the stereoisomer thereof,
A₁ is selected from the group consisting of CR₂ or N;
A₂ is selected from the group consisting of CR₂ or O;
R₁ is selected from the group consisting of H, F, methyl, or methoxy;
each R₂ is independently selected from the group consisting of H or methyl.

In one embodiment of the present disclosure, provided is a compound of formula (IA-1) or (IA-2), or a pharmaceutically acceptable salt thereof: 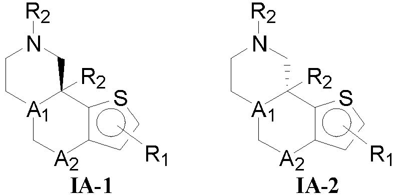
A₁ is selected from the group consisting of CR₂ or N;
A₂ is selected from the group consisting of CR₂ or O;
R₁ is selected from the group consisting of H, F, methyl, or methoxy;
R₂ is independently selected from the group consisting of H or methyl.

In one embodiment of the present disclosure, provided is a compound of formula (IA-3) or (IA-4), or a pharmaceutically acceptable salt thereof: 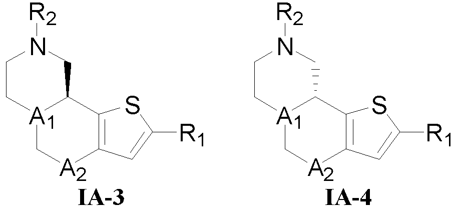 wherein,
A₁ is selected from the group consisting of CH or N;
A₂ is selected from the group consisting of CH₂ or O;
R₁ is selected from the group consisting of H, halogen, C₁₋₆ alkyl, or C₁₋₆ alkoxy;
R₂ is selected from the group consisting of H, C₁₋₆ alkyl, or the following formula: 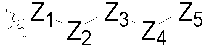
wherein,
Z₁ is selected from the group consisting of a bond or C₁₋₃ alkyl;
Z₂ is selected from the group consisting of a bond, -O-, -NH-, -S-, -(C=O)-, -O(C=O)-, or -(C=O)O-;
Z₃ is selected from the group consisting of a bond, C₁₋₆ alkyl, phenyl, -CH(NHAc)-CH₂CH₂-, -CH(CH₃)-NHC(=O)-CH(CH₃)-, -CH(CH₃)-OC(=O)-, or -CH₂CH₂CH₂CH₂CH(NH₂)-;
Z₄ is selected from the group consisting of a bond, -O-, -NH-, -S-, -(C=O)-, -O(C=O)-, or -(C=O)O-;
Z₅ is selected from the group consisting of H, C₁₋₁₂ alkyl, -P(=O)(OH)₂,

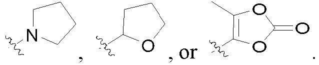

In some embodiments of the present disclosure, in the compound of formula (IA-3) or (IA-4), the pharmaceutically acceptable salt thereof or the stereoisomer thereof,
A₁ is selected from the group consisting of CH or N;
A₂ is selected from the group consisting of CH₂ or O;
R₁ is selected from the group consisting of H, F, methyl, or methoxy;
R₂ is selected from the group consisting of H, methyl, or a group of the following formula: 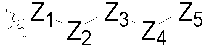
wherein,
Z₁ is selected from the group consisting of a bond, methyl, ethyl, n-propyl, and isopropyl;
Z₂ is selected from the group consisting of a bond, -O-, -NH-, -S-, -(C=O)-, -O(C=O)-, or -(C=O)O-;
Z₃ is selected from the group consisting of a bond, methyl, ethyl, n-propyl, isopropyl, n-butyl, isobutyl, s-butyl, t-butyl, n-pentyl, n-hexyl, phenyl, -CH(NHAc)-CH₂CH₂-, -CH(CH₃)-NHC(=O)-CH(CH₃)-, -CH(CH₃)-OC(=O)-, and -CH₂CH₂CH₂CH₂CH(NH₂)-;
Z₄ is selected from the group consisting of a bond, -O-, -NH-, -S-, -(C=O)-, -O(C=O)-, or -(C=O)O-;
Z₅ is selected from the group consisting of H, methyl, ethyl, n-propyl, isopropyl, n-butyl, isobutyl, s-butyl, t-butyl, n-pentyl, n-hexyl, n-heptyl, n-octyl, n-nonyl, n-decyl, n-undecyl, n-dodecyl, -P(=O)(OH)₂, 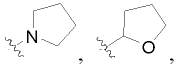 or

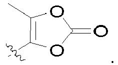

In some embodiments of the present disclosure, in the compound of formula (IA-3) or (IA-4), the pharmaceutically acceptable salt thereof or the stereoisomer thereof,
A₁ is selected from the group consisting of CH or N;
A₂ is selected from the group consisting of CH₂ or O;
R₁ is selected from the group consisting of H, F, methyl, or methoxy;
R₂ is selected from the group consisting of H, methyl, isopropyl, OH,

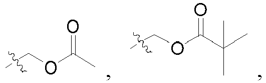

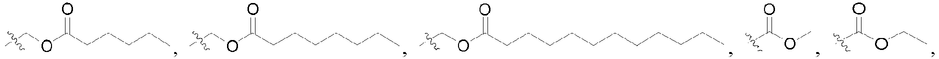

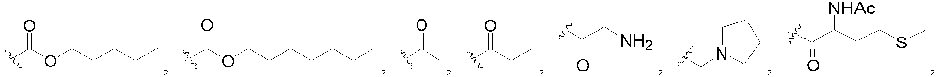

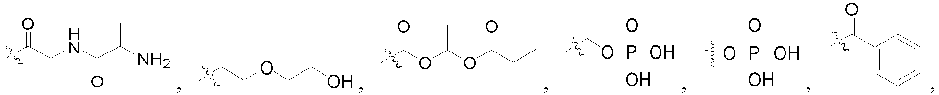

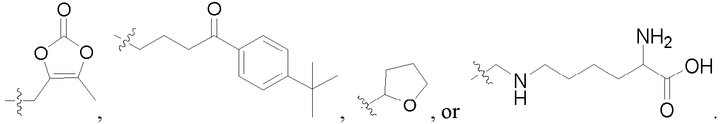

In some embodiments of the present disclosure, the compound of formula (IA-3) or (IA-4), the pharmaceutically acceptable salt thereof or the stereoisomer thereof is a prodrug of the compound of formula (IA-1) or (IA-2), the pharmaceutically acceptable salt thereof or the stereoisomer thereof.

In one embodiment of the present disclosure, provided is a compound of formula (IB), a pharmaceutically acceptable salt thereof or a stereoisomer thereof: 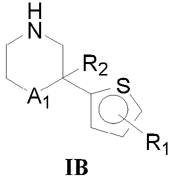 wherein,
(1) A₁ is selected from the group consisting of O or S;
   R₁ is selected from the group consisting of F, C₁₋₆ alkoxy, C₃₋₆ cycloalkoxy, C₁₋₆ haloalkoxy, C₃₋₆ cycloalkyl, or C₁₋₆ alkyl-(C=O)-;
   each R₂ is independently selected from the group consisting of H or C₁₋₆ alkyl;
      or
(2) A₁ is selected from the group consisting of NH;
   R₁ is selected from the group consisting of C₁₋₆ alkoxy, C₃₋₆ cycloalkoxy, C₁₋₆ haloalkoxy, C₃₋₆ cycloalkyl, or C₁₋₆ alkyl-(C=O)-;
   each R₂ is independently selected from the group consisting of H or C₁₋₆ alkyl;
      or
(3) A₁ is selected from the group consisting of CH₂;
   R₁ is selected from the group consisting of C₂₋₆ alkoxy, C₃₋₆ cycloalkoxy, C₁₋₆ haloalkoxy, C₃₋₆ cycloalkyl, or C₁₋₆ alkyl-(C=O)-;
   each R₂ is independently selected from the group consisting of H or C₁₋₆ alkyl.

In some embodiments of the present disclosure, in the compound of formula (IB), the pharmaceutically acceptable salt thereof or the stereoisomer thereof,
A₁ is selected from the group consisting of O or S;
R₁ is selected from the group consisting of F, methoxy, ethoxy, n-propoxy, isopropoxy, cyclopropoxy, cyclobutoxy, fluoromethoxy, chloromethoxy, difluoromethoxy, dichloromethoxy, trifluoromethoxy, trichloromethoxy, 2-fluoroethoxy, 2,2-difluoroethoxy, 2,2-dichloroethoxy, 2,2,2-trifluoroethoxy, 2,2,2-trichloroethoxy, pentafluoroethoxy, pentachloroethoxy, cyclopropyl, cyclobutyl, cyclopentyl, cyclohexyl, methyl-(C=O)-, or ethyl-(C=O)-;
R₂ is independently selected from the group consisting of H or methyl.

In some embodiments of the present disclosure, in the compound of formula (IB), the pharmaceutically acceptable salt thereof or the stereoisomer thereof,
A₁ is selected from the group consisting of NH;
R₁ is selected from the group consisting of methoxy, ethoxy, n-propoxy, isopropoxy, cyclopropoxy, cyclobutoxy, fluoromethoxy, chloromethoxy, difluoromethoxy, dichloromethoxy, trifluoromethoxy, trichloromethoxy, 2-fluoroethoxy, 2,2-difluoroethoxy, 2,2-dichloroethoxy, 2,2,2-trifluoroethoxy, 2,2,2-trichloroethoxy, pentafluoroethoxy, pentachloroethoxy, cyclopropyl, cyclobutyl, cyclopentyl, cyclohexyl, methyl-(C=O)-, or ethyl-(C=O)-;
R₂ is independently selected from the group consisting of H or methyl.

In some embodiments of the present disclosure, in the compound of formula (IB), the pharmaceutically acceptable salt thereof or the stereoisomer thereof,
A₁ is selected from the group consisting of CH₂;
R₁ is selected from the group consisting of ethoxy, n-propoxy, isopropoxy, cyclopropoxy, cyclobutoxy, fluoromethoxy, chloromethoxy, difluoromethoxy, dichloromethoxy, trifluoromethoxy, trichloromethoxy, 2-fluoroethoxy, 2,2-difluoroethoxy, 2,2-dichloroethoxy, 2,2,2-trifluoroethoxy, 2,2,2-trichloroethoxy, pentafluoroethoxy, pentachloroethoxy, cyclopropyl, cyclobutyl, cyclopentyl, cyclohexyl, methyl-(C=O)-, or ethyl-(C=O)-;
R₂ is independently selected from the group consisting of H or methyl.

In one embodiment of the present disclosure, provided is a compound of formula (IB-1) or (IB-2), or a pharmaceutically acceptable salt thereof: 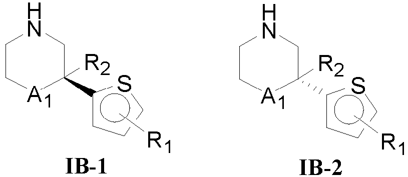 wherein,
(1) A₁ is selected from the group consisting of O or S;
   R₁ is selected from the group consisting of F, methoxy, ethoxy, n-propoxy, isopropoxy, cyclopropoxy, cyclobutoxy, fluoromethoxy, chloromethoxy, difluoromethoxy, dichloromethoxy, trifluoromethoxy, trichloromethoxy, 2-fluoroethoxy, 2,2-difluoroethoxy, 2,2-dichloroethoxy, 2,2,2-trifluoroethoxy, 2,2,2-trichloroethoxy, pentafluoroethoxy, pentachloroethoxy, cyclopropyl, cyclobutyl, cyclopentyl, cyclohexyl, methyl-(C=O)-, or ethyl-(C=O)-;
   each R₂ is independently selected from the group consisting of H or methyl;
      or
(2) A₁ is selected from the group consisting of NH;
   R₁ is selected from the group consisting of methoxy, ethoxy, n-propoxy, isopropoxy, cyclopropoxy, cyclobutoxy, fluoromethoxy, chloromethoxy, difluoromethoxy, dichloromethoxy, trifluoromethoxy, trichloromethoxy, 2-fluoroethoxy, 2,2-difluoroethoxy, 2,2-dichloroethoxy, 2,2,2-trifluoroethoxy, 2,2,2-trichloroethoxy, pentafluoroethoxy, pentachloroethoxy, cyclopropyl, cyclobutyl, cyclopentyl, cyclohexyl, methyl-(C=O)-, or ethyl-(C=O)-;
   each R₂ is independently selected from the group consisting of H or methyl;
      or
(3) A₁ is selected from the group consisting of CH₂;
   R₁ is selected from the group consisting of ethoxy, n-propoxy, isopropoxy, cyclopropoxy, cyclobutoxy, fluoromethoxy, chloromethoxy, difluoromethoxy, dichloromethoxy, trifluoromethoxy, trichloromethoxy, 2-fluoroethoxy, 2,2-difluoroethoxy, 2,2-dichloroethoxy, 2,2,2-trifluoroethoxy, 2,2,2-trichloroethoxy, pentafluoroethoxy, pentachloroethoxy, cyclopropyl, cyclobutyl, cyclopentyl, cyclohexyl, methyl-(C=O)-, or ethyl-(C=O)-;
   each R₂ is independently selected from the group consisting of H or methyl.

In one embodiment of the present disclosure, provided is a compound of formula (II), a pharmaceutically acceptable salt thereof or a stereoisomer thereof: 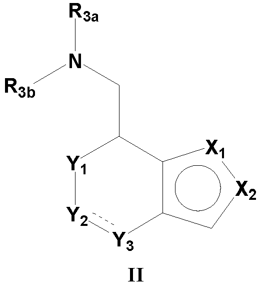 wherein,
X₁ is selected from the group consisting of S or O, and X₂ is selected from the group consisting of CH, or X₂ is selected from the group consisting of S or O, and X₁ is selected from the group consisting of CH;
(1) Y₁ is selected from the group consisting of O; 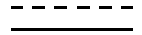
   optionally represents a single bond or a double bond; when 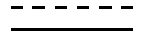 represents a single bond, Y₂ is selected from the group consisting of C(R₅ₐR_{5b}), and Y₃ is selected from the group consisting of C(R₆ₐR_{6b}); when 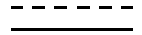 represents a double bond, Y₂ is selected from the group consisting of CR₅ₐ, and Y₃ is selected from the group consisting of CR₆ₐ;
   one of R₃ₐ and R_{3b} is selected from the group consisting of H, and the other is selected from the group consisting of C₁₋₆ alkyl, or R₃ₐ and R_{3b} are both selected from the group consisting of C₁₋₆ alkyl;
   R₅ₐ, R_{5b}, R₆ₐ, and R_{6b} are independently selected from the group consisting of H, C₁₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O), and at least one of R₅ₐ, R_{5b}, R₆ₐ, and R_{6b} is not hydrogen;
      or
(2) Y₁ is selected from the group consisting of NR₄ or S; 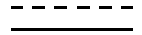
   optionally represents a single bond or a double bond; when 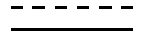 represents a single bond, Y₂ is selected from the group consisting of C(R₅ₐR_{5b}), and Y₃ is selected from the group consisting of C(R₆ₐR_{6b}); when 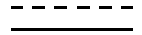 represents a double bond, Y₂ is selected from the group consisting of CR₅ₐ, and Y₃ is selected from the group consisting of CR₆ₐ;
   one of R₃ₐ and R_{3b} is selected from the group consisting of H, and the other is selected from the group consisting of C₁₋₆ alkyl, or R₃ₐ and R_{3b} are both selected from the group consisting of H;
   R₄ is independently selected from the group consisting of H or C₁₋₆ alkyl;
   R₅ₐ, R_{5b}, R₆ₐ, and R_{6b} are independently selected from the group consisting of H, C₁₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O);
      or
(3) Y₃ is selected from the group consisting of O, NR₆, or S; 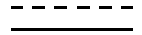
   represents a single bond; Y₁ is selected from the group consisting of C(R₄ₐR_{4b}) or Y₁ is absent, and Y₂ is selected from the group consisting of C(R₅ₐR_{5b});
   R₃ₐ and R_{3b} are independently selected from the group consisting of H or C₁₋₆ alkyl;
   R₄ₐ, R_{4b}, R₅ₐ, and R_{5b} are independently selected from the group consisting of H, C₁₋₆ alkyl, C₁₋₃ alkoxy C₁₋₃ alkyl, or C₆₋₁₀ aryl, or R₄ₐ and R_{4b}, and R₅ₐ and R_{5b} together form oxo (-C=O);
   R₆ is independently selected from the group consisting of H or C₁₋₆ alkyl.

In some embodiments of the present disclosure, in the compound of formula (II), the pharmaceutically acceptable salt thereof or the stereoisomer thereof,
(1) Y₁ is selected from the group consisting of O; 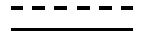
   optionally represents a single bond or a double bond; when 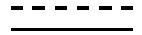 represents a single bond, Y₂ is selected from the group consisting of C(R₅ₐR_{5b}), and Y₃ is selected from the group consisting of C(R₆ₐR_{6b}); when 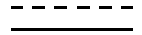 represents a double bond, Y₂ is selected from the group consisting of CR₅ₐ, and Y₃ is selected from the group consisting of CR₆ₐ;
   one of R₃ₐ and R_{3b} is selected from the group consisting of H, and the other is selected from the group consisting of C₁₋₆ alkyl, or R₃ₐ and R_{3b} are both selected from the group consisting of C₁₋₆ alkyl;
   R₅ₐ, R_{5b}, and R₆ₐ are independently selected from the group consisting of H, C₁₋₆ alkyl, C₁₋₃ alkoxy C₁₋₃ alkyl, or C₆₋₁₀ aryl, R_{6b} is independently selected from the group consisting of H, C₂₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O), and at least one of R₅ₐ, R_{5b}, R₆ₐ, and R_{6b} is not hydrogen;
   or R₅ₐ and R_{5b} are independently selected from the group consisting of H, C₁₋₆ alkyl, C₁₋₃ alkoxy C₁₋₃ alkyl, or C₆₋₁₀ aryl, and R₆ₐ and R_{6b} are both selected from the group consisting of C₁₋₆ alkyl;
   or R₅ₐ and R_{5b} are both selected from the group consisting of C₁₋₆ alkyl, and R₆ₐ and R_{6b} are independently selected from the group consisting of H, C₁₋₆ alkyl, C₁₋₃ alkoxy C₁₋₃ alkyl, or C₆₋₁₀ aryl;
      or
(2) Y₁ is selected from the group consisting of NR₄ or S; 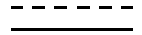
   optionally represents a single bond or a double bond; when 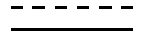 represents a single bond, Y₂ is selected from the group consisting of C(R₅ₐR_{5b}), and Y₃ is selected from the group consisting of C(R₆ₐR_{6b}); when 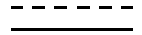 represents a double bond, Y₂ is selected from the group consisting of CR₅ₐ, and Y₃ is selected from the group consisting of CR₆ₐ;
   one of R₃ₐ and R_{3b} is selected from the group consisting of H, and the other is selected from the group consisting of C₁₋₆ alkyl;
   R₄ is independently selected from the group consisting of H or C₁₋₆ alkyl;
   R₅ₐ, R_{5b}, R₆ₐ, and R_{6b} are independently selected from the group consisting of H, C₁₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O);
      or
(3) Y₃ is selected from the group consisting of O, NR₆, or S; 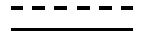
   represents a single bond; Y₁ is selected from the group consisting of C(R₄ₐR_{4b}) or Y₁ is absent, and Y₂ is selected from the group consisting of C(R₅ₐR_{5b});
   R₃ₐ and R_{3b} are independently selected from the group consisting of H or C₁₋₆ alkyl;
   R₄ₐ, R_{4b}, R₅ₐ, and R_{5b} are independently selected from the group consisting of H, C₁₋₆ alkyl, C₁₋₃ alkoxy C₁₋₃ alkyl, or C₆₋₁₀ aryl, or R₄ₐ and R_{4b} together form oxo (-C=O), and at least one of R₄ₐ, R_{4b}, R₅ₐ, and R_{5b} is not hydrogen; R₆ is independently selected from the group consisting of H or C₁₋₆ alkyl.

In some embodiments of the present disclosure, in the compound of formula (II), the pharmaceutically acceptable salt thereof or the stereoisomer thereof, X₁ is selected from the group consisting of S or O, and X₂ is selected from the group consisting of CH; the other variables are as defined in the present disclosure.

In some embodiments of the present disclosure, in the compound of formula (II), the pharmaceutically acceptable salt thereof or the stereoisomer thereof, X₂ is selected from the group consisting of S or O, and X₁ is selected from the group consisting of CH; the other variables are as defined in the present disclosure.

In some embodiments of the present disclosure, in the compound of formula (II), the pharmaceutically acceptable salt thereof or the stereoisomer thereof,
one of R₃ₐ and R_{3b} is selected from the group consisting of H, and the other is selected from the group consisting of methyl, or R₃ₐ and R_{3b} are both selected from the group consisting of methyl;
R₄ is independently selected from the group consisting of H or methyl;
R₄ₐ, R_{4b}, R₅ₐ, R_{5b}, R₆ₐ, and R_{6b} are independently selected from the group consisting of H, methyl, ethyl, n-propyl, isopropyl, methoxymethyl, and phenyl, or R₄ₐ and R_{4b}, R₅ₐ and R_{5b}, and R₆ₐ and R_{6b} together form oxo (-C=O); R₆ is independently selected from the group consisting of H or methyl;
the other variables are as defined in the present disclosure.

In some embodiments of the present disclosure, in the compound of formula (II), the pharmaceutically acceptable salt thereof or the stereoisomer thereof,
X₁ is selected from the group consisting of S or O, and X₂ is selected from the group consisting of CH;
Y₁ is selected from the group consisting of O; 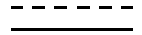
optionally represents a single bond or a double bond; when 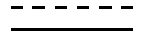 represents a single bond, Y₂ is selected from the group consisting of C(R₅ₐR_{5b}), and Y₃ is selected from the group consisting of C(R₆ₐR_{6b}); when 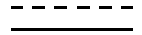 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl, or R₃ₐ and R_{3b} are both selected from the group consisting of methyl;
R₅ₐ, R_{5b}, R₆ₐ, and R_{6b} are independently selected from the group consisting of H, methyl, ethyl, n-propyl, isopropyl, methoxymethyl, and phenyl, or R₅ₐ and R_{5b}, and R₆ₐ and R_{6b} together form oxo (-C=O), and at least one of R₅ₐ, R_{5b}, R₆ₐ, and R_{6b} is not hydrogen.

In some embodiments of the present disclosure, in the compound of formula (II), the pharmaceutically acceptable salt thereof or the stereoisomer thereof,
X₁ is selected from the group consisting of S or O, and X₂ is selected from the group consisting of CH;
Y₁ is selected from the group consisting of O; 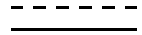
optionally represents a single bond or a double bond; when 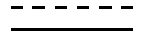 represents a single bond, Y₂ is selected from the group consisting of C(R₅ₐR_{5b}), and Y₃ is selected from the group consisting of C(R₆ₐR_{6b}); when 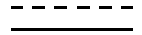 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl, or R₃ₐ and R_{3b} are both selected from the group consisting of methyl;
R₅ₐ, R_{5b}, and R₆ₐ are independently selected from the group consisting of H, methyl, ethyl, n-propyl, isopropyl, methoxymethyl, and phenyl, R_{6b} is independently selected from the group consisting of H, ethyl, n-propyl, isopropyl, methoxymethyl, and phenyl, or R₅ₐ and R_{5b}, and R₆ₐ and R_{6b} together form oxo (-C=O), and at least one of R₅ₐ, R_{5b}, R₆ₐ, and R_{6b} is not hydrogen;
or R₅ₐ and R_{5b} are independently selected from the group consisting of H, methyl, ethyl, n-propyl, isopropyl, methoxymethyl, and phenyl, and R₆ₐ and R_{6b} are both selected from the group consisting of methyl;
or R₅ₐ and R_{5b} are both selected from the group consisting of methyl, and R₆ₐ and R_{6b} are independently selected from the group consisting of H, methyl, ethyl, n-propyl, isopropyl, methoxymethyl, and phenyl.

In some embodiments of the present disclosure, in the compound of formula (II), the pharmaceutically acceptable salt thereof or the stereoisomer thereof,
X₁ is selected from the group consisting of S or O, and X₂ is selected from the group consisting of CH;
Y₁ is selected from the group consisting of NR₄ or S; 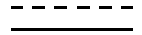
optionally represents a single bond or a double bond; when 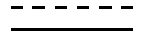 represents a single bond, Y₂ is selected from the group consisting of C(R₅ₐR_{5b}), and Y₃ is selected from the group consisting of C(R₆ₐR_{6b}); when 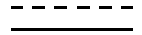 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl, or R₃ₐ and R_{3b} are both selected from the group consisting of H;
R₄ is independently selected from the group consisting of H or methyl;
R₅ₐ, R_{5b}, R₆ₐ, and R_{6b} are independently selected from the group consisting of H, methyl, ethyl, n-propyl, isopropyl, methoxymethyl, and phenyl, or R₅ₐ and R_{5b}, and R₆ₐ and R_{6b} together form oxo (-C=O).

In some embodiments of the present disclosure, in the compound of formula (II), the pharmaceutically acceptable salt thereof or the stereoisomer thereof,
X₁ is selected from the group consisting of S or O, and X₂ is selected from the group consisting of CH;
Y₁ is selected from the group consisting of NR₄ or S; 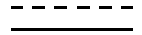
optionally represents a single bond or a double bond; when 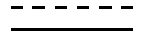 represents a single bond, Y₂ is selected from the group consisting of C(R₅ₐR_{5b}), and Y₃ is selected from the group consisting of C(R₆ₐR_{6b}); when 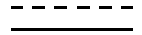 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl;
R₄ is independently selected from the group consisting of H or methyl;
R₅ₐ, R_{5b}, R₆ₐ, and R_{6b} are independently selected from the group consisting of H, methyl, ethyl, n-propyl, isopropyl, methoxymethyl, and phenyl, or R₅ₐ and R_{5b}, and R₆ₐ and R_{6b} together form oxo (-C=O).

In some embodiments of the present disclosure, in the compound of formula (II), the pharmaceutically acceptable salt thereof or the stereoisomer thereof,
X₁ is selected from the group consisting of S or O, and X₂ is selected from the group consisting of CH;
Y₃ is selected from the group consisting of O, NR₆, or S; 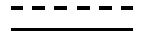
represents a single bond; Y₁ is selected from the group consisting of C(R₄ₐR_{4b}) or Y₁ is absent, and Y₂ is selected from the group consisting of C(R₅ₐR_{5b});
R₃ₐ and R_{3b} are independently selected from the group consisting of H or methyl;
R₄ₐ, R_{4b}, R₅ₐ, and R_{5b} are independently selected from the group consisting of H, methyl, ethyl, n-propyl, isopropyl, methoxymethyl, and phenyl, or R₄ₐ and R_{4b}, and R₅ₐ and R_{5b} together form oxo (-C=O);
R₆ is independently selected from the group consisting of H or methyl.

In some embodiments of the present disclosure, in the compound of formula (II), the pharmaceutically acceptable salt thereof or the stereoisomer thereof,
X₁ is selected from the group consisting of S or O, and X₂ is selected from the group consisting of CH;
Y₃ is selected from the group consisting of O, NR₆, or S; 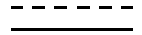 represents a single bond; Y₁ is selected from the group consisting of C(R₄ₐR_{4b}) or Y₁ is absent, and Y₂ is selected from the group consisting of C(R₅ₐR_{5b});
R₃ₐ and R_{3b} are independently selected from the group consisting of H or methyl;
R₄ₐ, R_{4b}, R₅ₐ, and R_{5b} are independently selected from the group consisting of H, methyl, ethyl, n-propyl, isopropyl, methoxymethyl, and phenyl, or R₄ₐ and R_{4b} together form oxo (-C=O), and at least one of R₄ₐ, R_{4b}, R₅ₐ, and R_{5b} is not hydrogen;
R₆ is independently selected from the group consisting of H or methyl.

In some embodiments of the present disclosure, in the compound of formula (II), the pharmaceutically acceptable salt thereof or the stereoisomer thereof,
X₁ is selected from the group consisting of S, and X₂ is selected from the group consisting of CH;
Y₁ is selected from the group consisting of NR₄ or S;
when 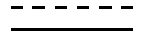 represents a single bond, Y₂ and Y₃ are both selected from the group consisting of CH₂;
one of R₃ₐ and R_{3b} is selected from the group consisting of H, and the other is selected from the group consisting of methyl;
R₄ is selected from the group consisting of H or C₁₋₆ alkyl, preferably H or methyl.

In one embodiment of the present disclosure, provided is a compound of formula (IIA) or (IIB), or a pharmaceutically acceptable salt thereof: 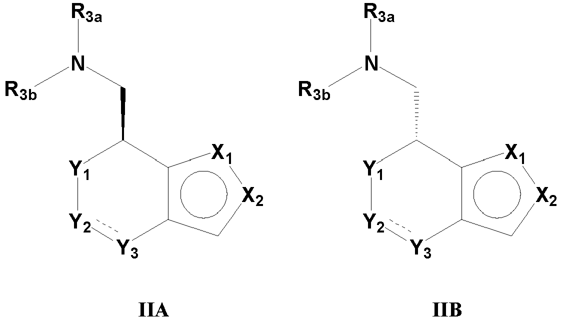 wherein,
X₁ is selected from the group consisting of S or O, and X₂ is selected from the group consisting of CH, or X₂ is selected from the group consisting of S or O, and X₁ is selected from the group consisting of CH;
(1) Y₁ is selected from the group consisting of O; 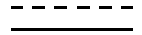
   optionally represents a single bond or a double bond; when 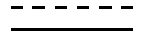 represents a single bond, Y₂ is selected from the group consisting of C(R₅ₐR_{5b}), and Y₃ is selected from the group consisting of C(R₆ₐR_{6b}); when 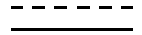 represents a double bond, Y₂ is selected from the group consisting of CR₅ₐ, and Y₃ is selected from the group consisting of CR₆ₐ;
   one of R₃ₐ and R_{3b} is selected from the group consisting of H, and the other is selected from the group consisting of C₁₋₆ alkyl, or R₃ₐ and R_{3b} are both selected from the group consisting of C₁₋₆ alkyl;
   R₅ₐ, R_{5b}, R₆ₐ, and R_{6b} are independently selected from the group consisting of H, C₁₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O), and at least one of R₅ₐ, R_{5b}, R₆ₐ, and R_{6b} is not hydrogen; or
(2) Y₁ is selected from the group consisting of NR₄ or S; 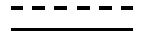
   optionally represents a single bond or a double bond; when 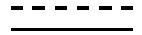 represents a single bond, Y₂ is selected from the group consisting of C(R₅ₐR_{5b}), and Y₃ is selected from the group consisting of C(R₆ₐR_{6b}); when 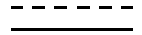 represents a double bond, Y₂ is selected from the group consisting of CR₅ₐ, and Y₃ is selected from the group consisting of CR₆ₐ;
   one of R₃ₐ and R_{3b} is selected from the group consisting of H, and the other is selected from the group consisting of C₁₋₆ alkyl, or R₃ₐ and R_{3b} are both selected from the group consisting of H;
   R₄ is independently selected from the group consisting of H or C₁₋₆ alkyl;
   R₅ₐ, R_{5b}, R₆ₐ, and R_{6b} are independently selected from the group consisting of H, C₁₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O);
      or
(3) Y₃ is selected from the group consisting of O, NR₆, or S; 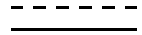
   represents a single bond; Y₁ is selected from the group consisting of C(R₄ₐR_{4b}) or Y₁ is absent, and Y₂ is selected from the group consisting of C(R₅ₐR_{5b});
   R₃ₐ and R_{3b} are independently selected from the group consisting of H or C₁₋₆ alkyl;
   R₄ₐ, R_{4b}, R₅ₐ, and R_{5b} are independently selected from the group consisting of H, C₁₋₆ alkyl, C₁₋₃ alkoxy C₁₋₃ alkyl, or C₆₋₁₀ aryl, or R₄ₐ and R_{4b}, and R₅ₐ and R_{5b} together form oxo (-C=O);
   R₆ is independently selected from the group consisting of H or C₁₋₆ alkyl.

In some embodiments of the present disclosure, in the compound of formula (IIA) or (IIB), the pharmaceutically acceptable salt thereof or the stereoisomer thereof,
(1) Y₁ is selected from the group consisting of O; 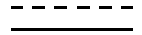
   optionally represents a single bond or a double bond; when 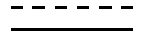 represents a single bond, Y₂ is selected from the group consisting of C(R₅ₐR_{5b}), and Y₃ is selected from the group consisting of C(R₆ₐR_{6b}); when 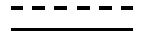 represents a double bond, Y₂ is selected from the group consisting of CR₅ₐ, and Y₃ is selected from the group consisting of CR₆ₐ;
   one of R₃ₐ and R_{3b} is selected from the group consisting of H, and the other is selected from the group consisting of C₁₋₆ alkyl, or R₃ₐ and R_{3b} are both selected from the group consisting of C₁₋₆ alkyl;
   R₅ₐ, R_{5b}, and R₆ₐ are independently selected from the group consisting of H, C₁₋₆ alkyl, C₁₋₃ alkoxy C₁₋₃ alkyl, or C₆₋₁₀ aryl, R_{6b} is independently selected from the group consisting of H, C₂₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O), and at least one of R₅ₐ, R_{5b}, R₆ₐ, and R_{6b} is not hydrogen;
   or R₅ₐ and R_{5b} are independently selected from the group consisting of H, C₁₋₆ alkyl, C₁₋₃ alkoxy C₁₋₃ alkyl, or C₆₋₁₀ aryl, and R₆ₐ and R_{6b} are both selected from the group consisting of C₁₋₆ alkyl;
   or R₅ₐ and R_{5b} are both selected from the group consisting of C₁₋₆ alkyl, and R₆ₐ and R_{6b} are independently selected from the group consisting of H, C₁₋₆ alkyl, C₁₋₃ alkoxy C₁₋₃ alkyl, or C₆₋₁₀ aryl;
      or
(2) Y₁ is selected from the group consisting of NR₄ or S; 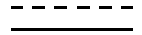
   optionally represents a single bond or a double bond; when 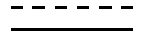 represents a single bond, Y₂ is selected from the group consisting of C(R₅ₐR_{5b}), and Y₃ is selected from the group consisting of C(R₆ₐR_{6b}); when 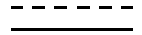 represents a double bond, Y₂ is selected from the group consisting of CR₅ₐ, and Y₃ is selected from the group consisting of CR₆ₐ;
   one of R₃ₐ and R_{3b} is selected from the group consisting of H, and the other is selected from the group consisting of C₁₋₆ alkyl;
   R₄ is independently selected from the group consisting of H or C₁₋₆ alkyl;
   R₅ₐ, R_{5b}, R₆ₐ, and R_{6b} are independently selected from the group consisting of H, C₁₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O);
      or
(3) Y₃ is selected from the group consisting of O, NR₆, or S; 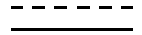
   represents a single bond; Y₁ is selected from the group consisting of C(R₄ₐR_{4b}) or Y₁ is absent, and Y₂ is selected from the group consisting of C(R₅ₐR_{5b});
   R₃ₐ and R_{3b} are independently selected from the group consisting of H or C₁₋₆ alkyl;
   R₄ₐ, R_{4b}, R₅ₐ, and R_{5b} are independently selected from the group consisting of H, C₁₋₆ alkyl, C₁₋₃ alkoxy C₁₋₃ alkyl, or C₆₋₁₀ aryl, or R₄ₐ and R_{4b} together form oxo (-C=O), and at least one of R₄ₐ, R_{4b}, R₅ₐ, and R_{5b} is not hydrogen; R₆ is independently selected from the group consisting of H or C₁₋₆ alkyl.

In some embodiments of the present disclosure, in the compound of formula (IIA) or (IIB), or the pharmaceutically acceptable salt thereof, X₁ is selected from the group consisting of S or O, and X₂ is selected from the group consisting of CH; the other variables are as defined in the present disclosure.

In some embodiments of the present disclosure, in the compound of formula (IIA) or (IIB), or the pharmaceutically acceptable salt thereof, X₂ is selected from the group consisting of S or O, and X₁ is selected from the group consisting of CH; the other variables are as defined in the present disclosure.

In some embodiments of the present disclosure, in the compound of formula (IIA) or (IIB), or the pharmaceutically acceptable salt thereof,
one of R₃ₐ and R_{3b} is selected from the group consisting of H, and the other is selected from the group consisting of methyl, or R₃ₐ and R_{3b} are both selected from the group consisting of methyl;
R₄ is independently selected from the group consisting of H or methyl;
R₄ₐ, R_{4b}, R₅ₐ, R_{5b}, R₆ₐ, and R_{6b} are independently selected from the group consisting of H, methyl, ethyl, n-propyl, isopropyl, methoxymethyl, and phenyl, or R₄ₐ and R_{4b}, R₅ₐ and R_{5b}, and R₆ₐ and R_{6b} together form oxo (-C=O); R₆ is independently selected from the group consisting of H or methyl;
the other variables are as defined in the present disclosure.

In some embodiments of the present disclosure, in the compound of formula (IIA) or (IIB), or the pharmaceutically acceptable salt thereof,
X₁ is selected from the group consisting of S or O, and X₂ is selected from the group consisting of CH;
Y₁ is selected from the group consisting of O; 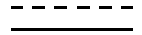
optionally represents a single bond or a double bond; when 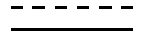 represents a single bond, Y₂ is selected from the group consisting of C(R₅ₐR_{5b}), and Y₃ is selected from the group consisting of C(R₆ₐR_{6b}); when 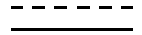 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl, or R₃ₐ and R_{3b} are both selected from the group consisting of methyl;
R₅ₐ, R_{5b}, R₆ₐ, and R_{6b} are independently selected from the group consisting of H, methyl, ethyl, n-propyl, isopropyl, methoxymethyl, and phenyl, or R₅ₐ and R_{5b}, and R₆ₐ and R_{6b} together form oxo (-C=O), and at least one of R₅ₐ, R_{5b}, R₆ₐ, and R_{6b} is not hydrogen.

In some embodiments of the present disclosure, in the compound of formula (IIA) or (IIB), the pharmaceutically acceptable salt thereof or the stereoisomer thereof,
X₁ is selected from the group consisting of S or O, and X₂ is selected from the group consisting of CH;
Y₁ is selected from the group consisting of O; 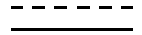
optionally represents a single bond or a double bond; when 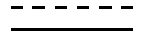 represents a single bond, Y₂ is selected from the group consisting of C(R₅ₐR_{5b}), and Y₃ is selected from the group consisting of C(R₆ₐR_{6b}); when 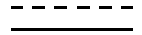 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl, or R₃ₐ and R_{3b} are both selected from the group consisting of methyl;
R₅ₐ, R_{5b}, and R₆ₐ are independently selected from the group consisting of H, methyl, ethyl, n-propyl, isopropyl, methoxymethyl, and phenyl, R_{6b} is independently selected from the group consisting of H, ethyl, n-propyl, isopropyl, methoxymethyl, and phenyl, or R₅ₐ and R_{5b}, and R₆ₐ and R_{6b} together form oxo (-C=O), and at least one of R₅ₐ, R_{5b}, R₆ₐ, and R_{6b} is not hydrogen;
or R₅ₐ and R_{5b} are independently selected from the group consisting of H, methyl, ethyl, n-propyl, isopropyl, methoxymethyl, and phenyl, and R₆ₐ and R_{6b} are both selected from the group consisting of methyl;
or R₅ₐ and R_{5b} are both selected from the group consisting of methyl, and R₆ₐ and R_{6b} are independently selected from the group consisting of H, methyl, ethyl, n-propyl, isopropyl, methoxymethyl, and phenyl.

In some embodiments of the present disclosure, in the compound of formula (IIA) or (IIB), the pharmaceutically acceptable salt thereof or the stereoisomer thereof,
X₁ is selected from the group consisting of S or O, and X₂ is selected from the group consisting of CH;
Y₁ is selected from the group consisting of NR₄ or S; 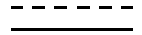
optionally represents a single bond or a double bond; when 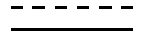 represents a single bond, Y₂ is selected from the group consisting of C(R₅ₐR_{5b}), and Y₃ is selected from the group consisting of C(R₆ₐR_{6b}); when 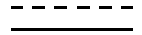 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl, or R₃ₐ and R_{3b} are both selected from the group consisting of H;
R₄ is independently selected from the group consisting of H or methyl;
R₅ₐ, R_{5b}, R₆ₐ, and R_{6b} are independently selected from the group consisting of H, methyl, ethyl, n-propyl, isopropyl, methoxymethyl, and phenyl, or R₅ₐ and R_{5b}, and R₆ₐ and R_{6b} together form oxo (-C=O).

In some embodiments of the present disclosure, in the compound of formula (IIA) or (IIB), the pharmaceutically acceptable salt thereof or the stereoisomer thereof,
X₁ is selected from the group consisting of S or O, and X₂ is selected from the group consisting of CH;
Y₁ is selected from the group consisting of NR₄ or S; 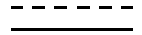
optionally represents a single bond or a double bond; when 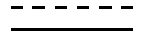 represents a single bond, Y₂ is selected from the group consisting of C(R₅ₐR_{5b}), and Y₃ is selected from the group consisting of C(R₆ₐR_{6b}); when 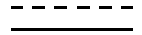 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl;
R₄ is independently selected from the group consisting of H or methyl;
R₅ₐ, R_{5b}, R₆ₐ, and R_{6b} are independently selected from the group consisting of H, methyl, ethyl, n-propyl, isopropyl, methoxymethyl, and phenyl, or R₅ₐ and R_{5b}, and R₆ₐ and R_{6b} together form oxo (-C=O).

In some embodiments of the present disclosure, in the compound of formula (IIA) or (IIB), the pharmaceutically acceptable salt thereof or the stereoisomer thereof,
X₁ is selected from the group consisting of S or O, and X₂ is selected from the group consisting of CH;
Y₃ is selected from the group consisting of O, NR₆, or S; 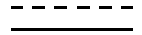
represents a single bond; Y₁ is selected from the group consisting of C(R₄ₐR_{4b}) or Y₁ is absent, and Y₂ is selected from the group consisting of C(R₅ₐR_{5b});
R₃ₐ and R_{3b} are independently selected from the group consisting of H or methyl;
R₄ₐ, R_{4b}, R₅ₐ, and R_{5b} are independently selected from the group consisting of H, methyl, ethyl, n-propyl, isopropyl, methoxymethyl, and phenyl, or R₄ₐ and R_{4b}, and R₅ₐ and R_{5b} together form oxo (-C=O);
R₆ is independently selected from the group consisting of H or methyl.

In some embodiments of the present disclosure, in the compound of formula (IIA) or (IIB), the pharmaceutically acceptable salt thereof or the stereoisomer thereof,
X₂ is selected from the group consisting of S or O, and X₁ is selected from the group consisting of CH;
Y₁ is selected from the group consisting of O; 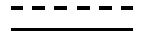
optionally represents a single bond or a double bond; when 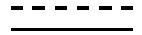 represents a single bond, Y₂ is selected from the group consisting of C(R₅ₐR_{5b}), and Y₃ is selected from the group consisting of C(R₆ₐR_{6b}); when 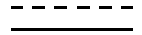 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl, or R₃ₐ and R_{3b} are both selected from the group consisting of methyl;
R₅ₐ, R_{5b}, R₆ₐ, and R_{6b} are independently selected from the group consisting of H, methyl, ethyl, n-propyl, isopropyl, methoxymethyl, and phenyl, or R₅ₐ and R_{5b}, and R₆ₐ and R_{6b} together form oxo (-C=O), and at least one of R₅ₐ, R_{5b}, R₆ₐ, and R_{6b} is not hydrogen.

In some embodiments of the present disclosure, in the compound of formula (IIA) or (IIB), the pharmaceutically acceptable salt thereof or the stereoisomer thereof,
X₂ is selected from the group consisting of S or O, and X₁ is selected from the group consisting of CH;
Y₁ is selected from the group consisting of NR₄ or S; 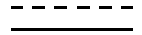
optionally represents a single bond or a double bond; when 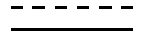 represents a single bond, Y₂ is selected from the group consisting of C(R₅ₐR_{5b}), and Y₃ is selected from the group consisting of C(R₆ₐR_{6b}); when 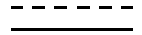 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of methyl, or R₃ₐ and R_{3b} are both selected from the group consisting of H;
R₄ is independently selected from the group consisting of H or methyl;
R₅ₐ, R_{5b}, R₆ₐ, and R_{6b} are independently selected from the group consisting of H, methyl, ethyl, n-propyl, isopropyl, methoxymethyl, and phenyl, or R₅ₐ and R_{5b}, and R₆ₐ and R_{6b} together form oxo (-C=O).

In some embodiments of the present disclosure, in the compound of formula (IIA) or (IIB), the pharmaceutically acceptable salt thereof or the stereoisomer thereof,
X₂ is selected from the group consisting of S or O, and X₁ is selected from the group consisting of CH;
Y₃ is selected from the group consisting of O, NR₆, or S; 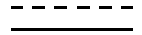
represents a single bond; Y₁ is selected from the group consisting of C(R₄ₐR_{4b}) or Y₁ is absent, and Y₂ is selected from the group consisting of C(R₅ₐR_{5b});
R₃ₐ and R_{3b} are independently selected from the group consisting of H or methyl;
R₄ₐ, R_{4b}, R₅ₐ, and R_{5b} are independently selected from the group consisting of H, methyl, ethyl, n-propyl, isopropyl, methoxymethyl, and phenyl, or R₄ₐ and R_{4b}, and R₅ₐ and R_{5b} together form oxo (-C=O);
R₆ is independently selected from the group consisting of H or methyl.

In some embodiments of the present disclosure, in the compound of formula (IIA) or (IIB), the pharmaceutically acceptable salt thereof or the stereoisomer thereof,
X₁ is selected from the group consisting of S or O, and X₂ is selected from the group consisting of CH;
Y₃ is selected from the group consisting of O, NR₆, or S; 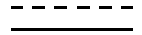 represents a single bond; Y₁ is selected from the group consisting of C(R₄ₐR_{4b}) or Y₁ is absent, and Y₂ is selected from the group consisting of C(R₅ₐR_{5b});
R₃ₐ and R_{3b} are independently selected from the group consisting of H or methyl;
R₄ₐ, R_{4b}, R₅ₐ, and R_{5b} are independently selected from the group consisting of H, methyl, ethyl, n-propyl, isopropyl, methoxymethyl, and phenyl, or R₄ₐ and R_{4b} together form oxo (-C=O), and at least one of R₄ₐ, R_{4b}, R₅ₐ, and R_{5b} is not hydrogen;
R₆ is independently selected from the group consisting of H or methyl.

In some embodiments of the present disclosure, in the compound of formula (IIA) or (IIB), the pharmaceutically acceptable salt thereof or the stereoisomer thereof,
X₁ is selected from the group consisting of S, and X₂ is selected from the group consisting of CH;
Y₁ is selected from the group consisting of NR₄ or S;
when 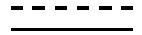 represents a single bond, Y₂ and Y₃ are both selected from the group consisting of CH₂;
one of R₃ₐ and R_{3b} is selected from the group consisting of H, and the other is selected from the group consisting of methyl;
R₄ is selected from the group consisting of H or C₁₋₆ alkyl, preferably H or methyl.

In one embodiment of the present disclosure, provided is a compound of formula (III), a pharmaceutically acceptable salt thereof or a stereoisomer thereof: 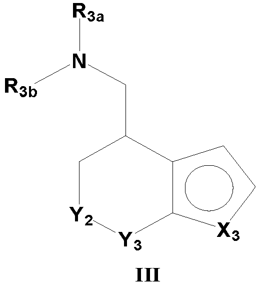 wherein,
X₃ is selected from the group consisting of S or O;
Y₂ is selected from the group consisting of C(R₅ₐR_{5b}), and Y₃ is selected from the group consisting of O, NR₆, or S;
R₃ₐ and R_{3b} are independently selected from the group consisting of H or C₁₋₆ alkyl;
R₅ₐ is independently selected from the group consisting of H, C₁₋₆ alkyl, C₁₋₃ alkoxy-C₁₋₃ alkyl, or C₆₋₁₀ aryl, R_{5b} is independently selected from the group consisting of C₁₋₆ alkyl, C₁₋₃ alkoxy C₁₋₃ alkyl, or C₆₋₁₀ aryl, or R₅ₐ and R_{5b} together form oxo (-C=O);
R₆ is independently selected from the group consisting of H or C₁₋₆ alkyl.

In some embodiments of the present disclosure, in the compound of formula (III), the pharmaceutically acceptable salt thereof or the stereoisomer thereof,
R₃ₐ and R_{3b} are independently selected from the group consisting of H or methyl;
R₅ₐ is independently selected from the group consisting of H, methyl, ethyl, n-propyl, isopropyl, methoxymethyl, and phenyl, R_{5b} is independently selected from the group consisting of methyl, ethyl, n-propyl, isopropyl, methoxymethyl, and phenyl, or R₅ₐ and R_{5b} together form oxo (-C=O);
R₆ is independently selected from the group consisting of H or methyl.

In one embodiment of the present disclosure, provided is a compound of formula (IIIA) or (IIIB), a pharmaceutically acceptable salt thereof or a stereoisomer thereof: 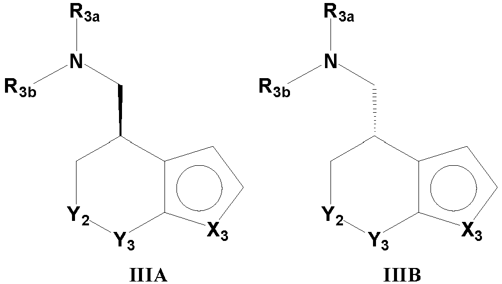 wherein,
X₃ is selected from the group consisting of S or O;
Y₂ is selected from the group consisting of C(R₅ₐR_{5b}), and Y₃ is selected from the group consisting of O, NR₆, or S;
R₃ₐ and R_{3b} are independently selected from the group consisting of H or C₁₋₆ alkyl;
R₅ₐ is independently selected from the group consisting of H, C₁₋₆ alkyl, C₁₋₃ alkoxy-C₁₋₃ alkyl, or C₆₋₁₀ aryl, R_{5b} is independently selected from the group consisting of C₁₋₆ alkyl, C₁₋₃ alkoxy C₁₋₃ alkyl, or C₆₋₁₀ aryl, or R₅ₐ and R_{5b} together form oxo (-C=O);
R₆ is independently selected from the group consisting of H or C₁₋₆ alkyl.

In some embodiments of the present disclosure, in the compound of formula (IIIA) or (IIIB), the pharmaceutically acceptable salt thereof or the stereoisomer thereof,
R₃ₐ and R_{3b} are independently selected from the group consisting of H or methyl;
R₅ₐ is independently selected from the group consisting of H, methyl, ethyl, n-propyl, isopropyl, methoxymethyl, and phenyl, R_{5b} is independently selected from the group consisting of methyl, ethyl, n-propyl, isopropyl, methoxymethyl, and phenyl, or R₅ₐ and R_{5b} together form oxo (-C=O);
R₆ is independently selected from the group consisting of H or methyl.

In another embodiment of the present disclosure, provided are the following compounds, pharmaceutically acceptable salts thereof or stereoisomers thereof:

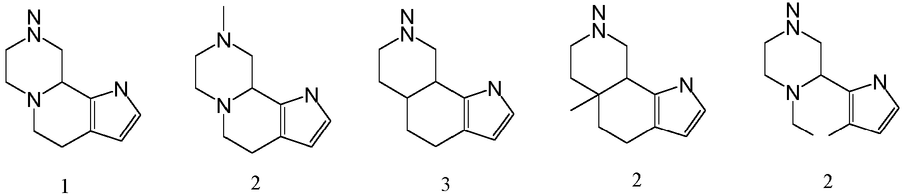

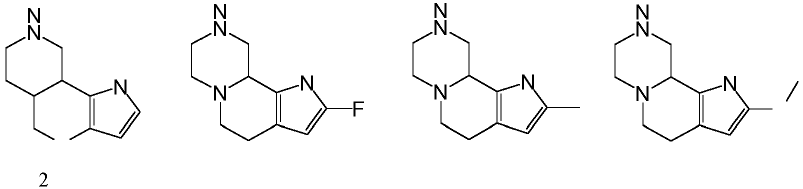

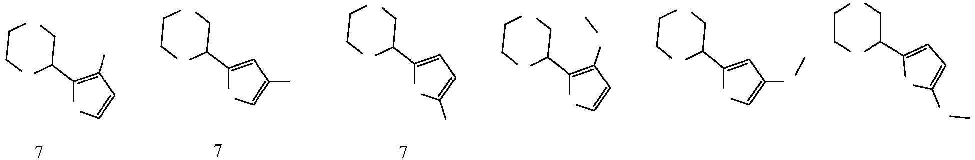

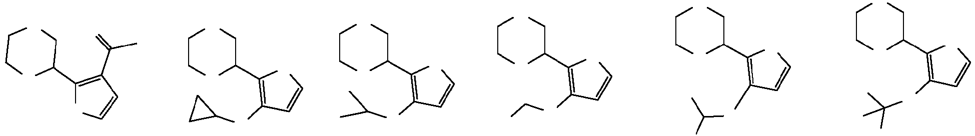

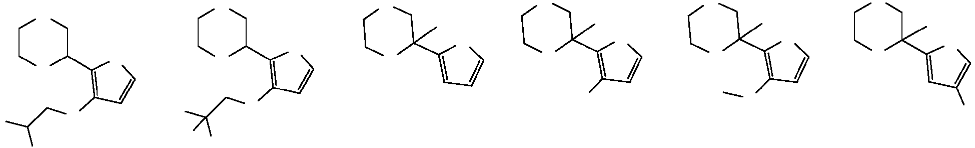

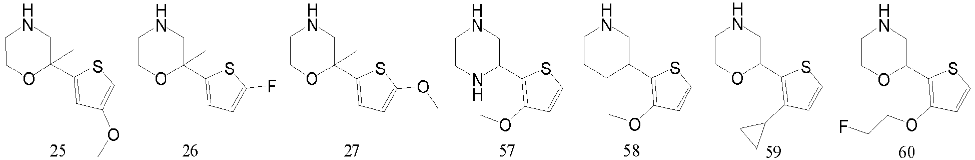

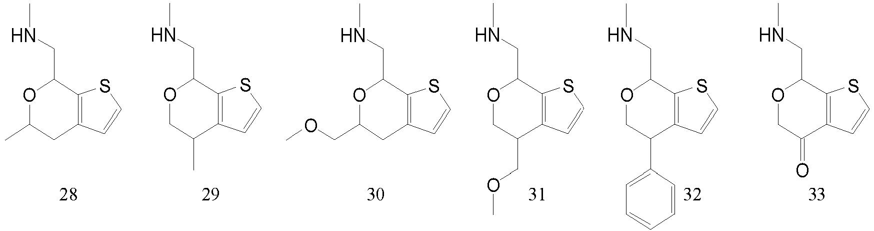

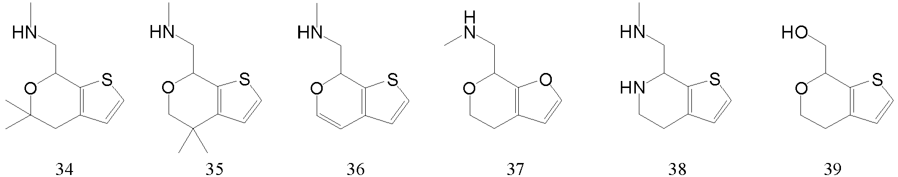

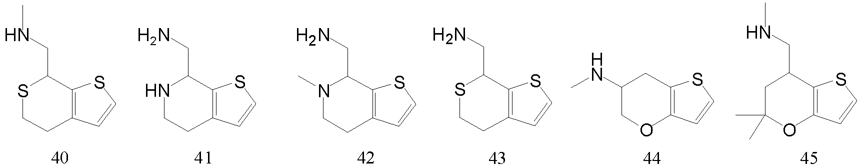

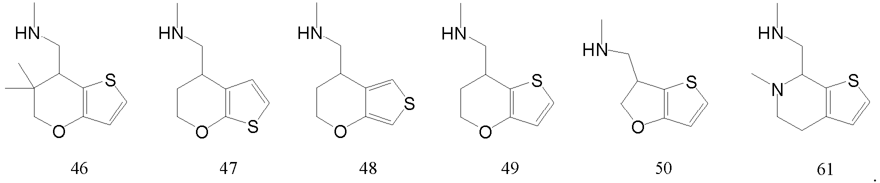

In another embodiment of the present disclosure, provided are the following compounds and stereoisomers or pharmaceutically acceptable salts thereof:

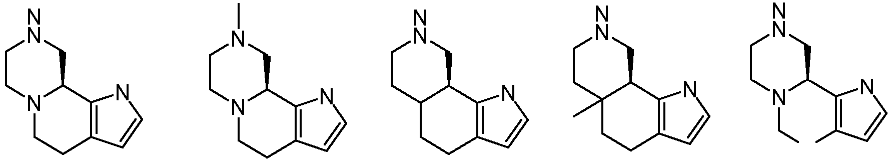

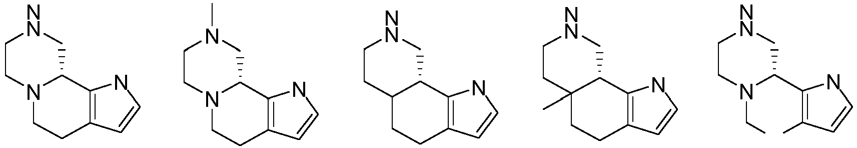

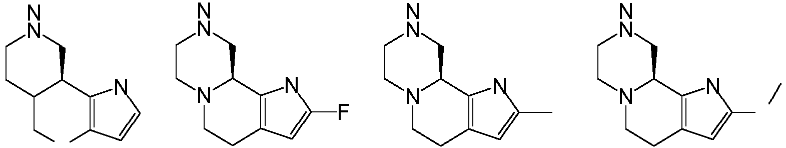

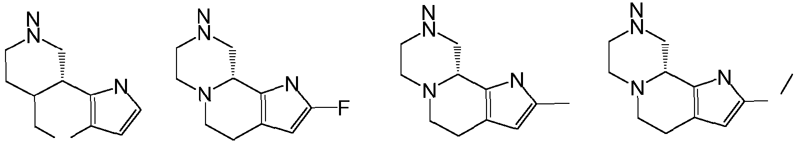

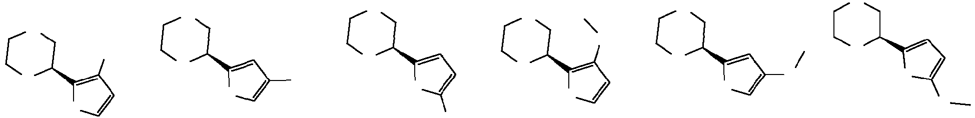

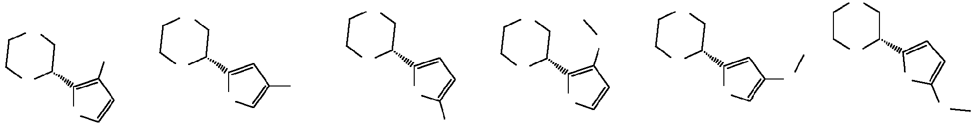

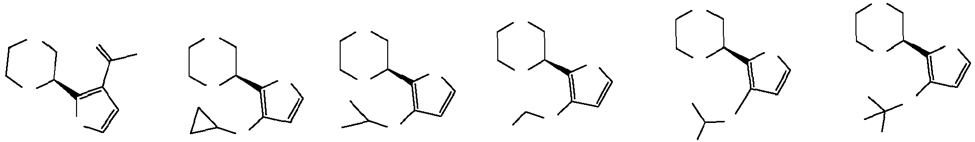

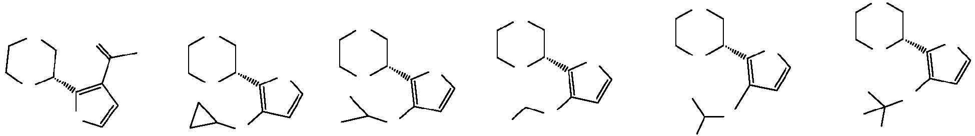

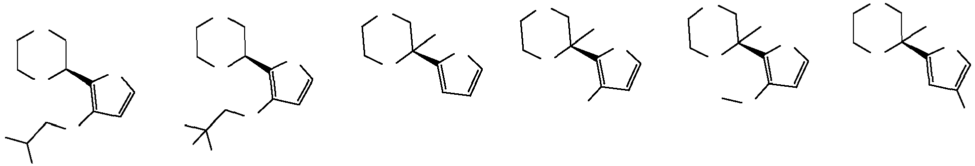

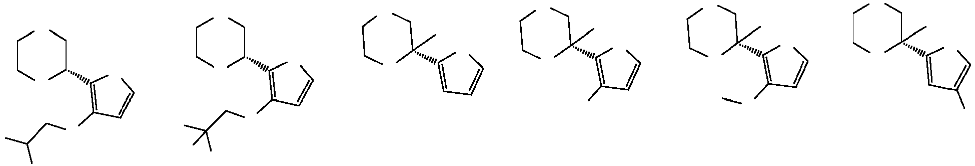

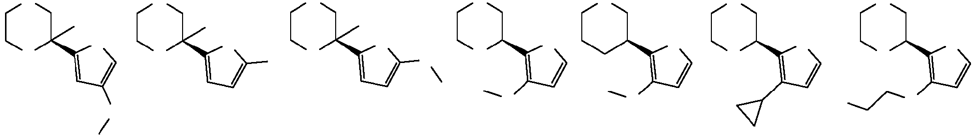

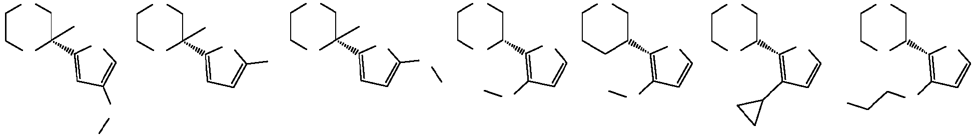

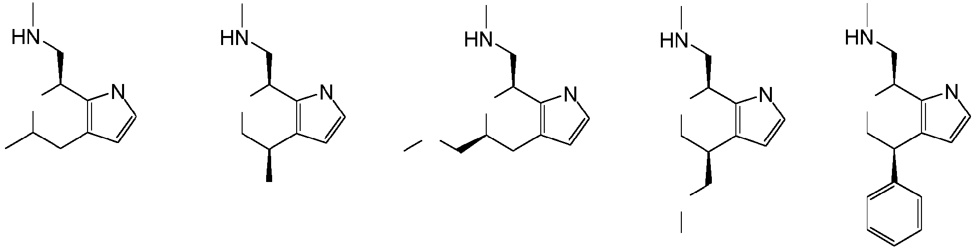

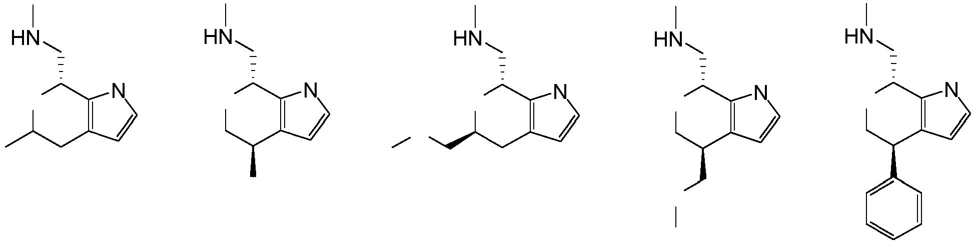

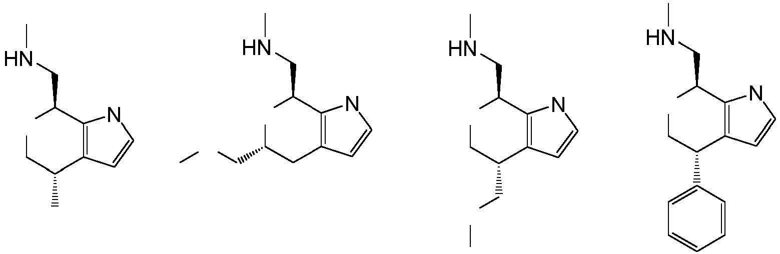

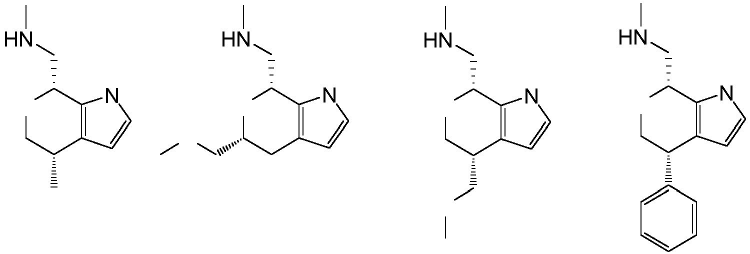

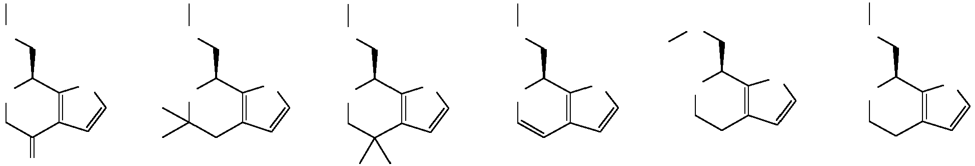

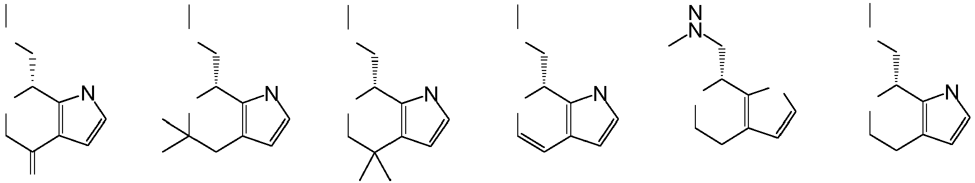

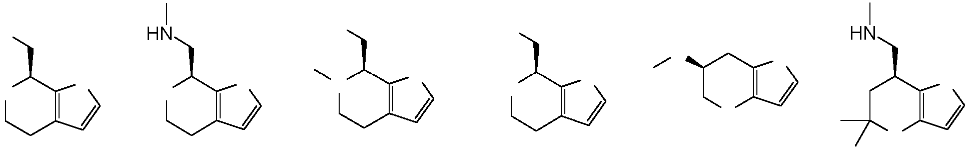

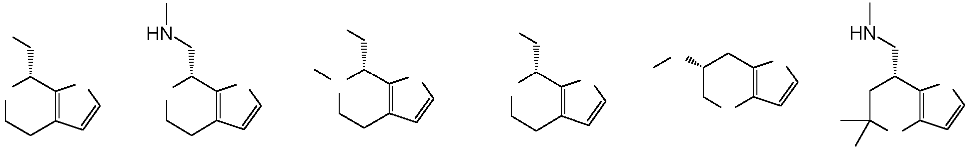

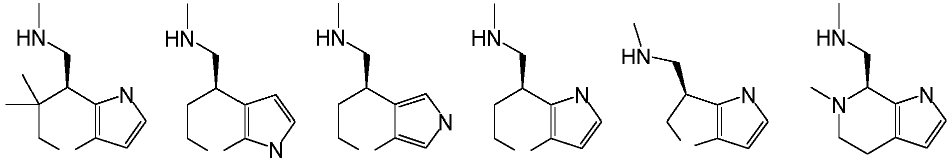

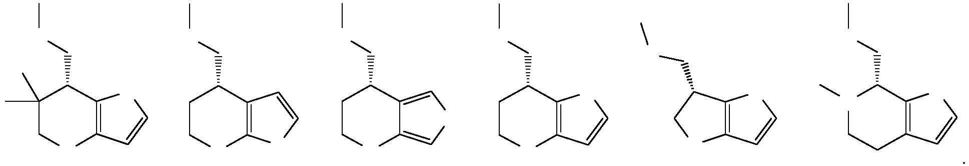

In another embodiment of the present disclosure, provided are the following compounds and stereoisomers or pharmaceutically acceptable salts thereof:

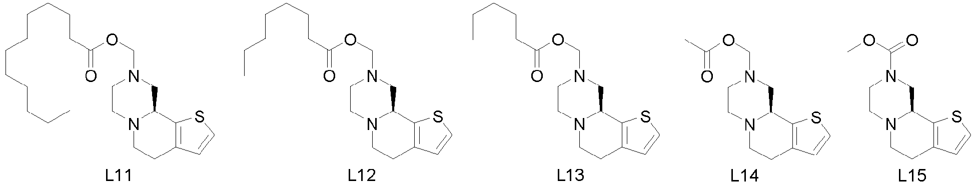

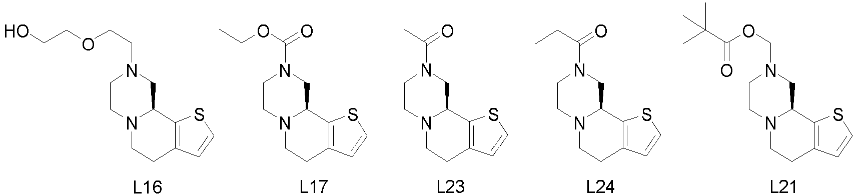

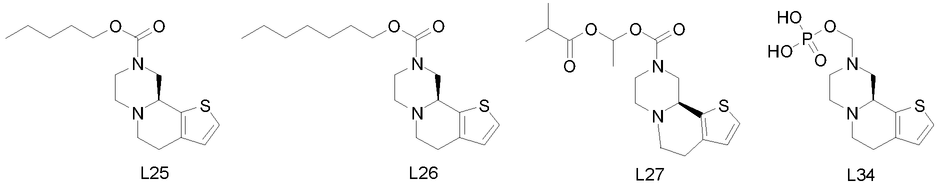

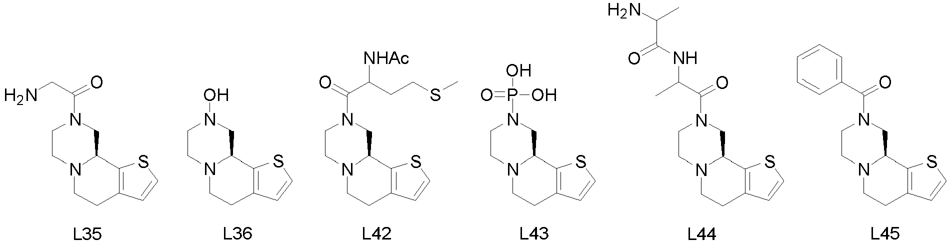

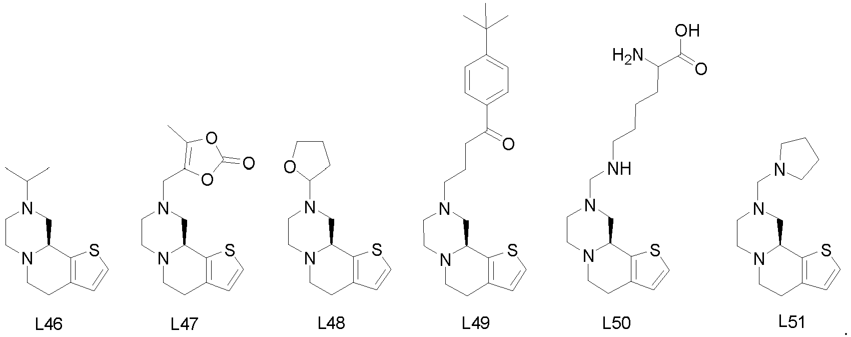

The present disclosure also provides a pharmaceutical composition comprising any one of the compounds or pharmaceutically acceptable salts thereof described above and a pharmaceutically acceptable carrier. The pharmaceutical composition can be prepared into various pharmaceutically acceptable dosage forms, such as tablets, capsules, oral liquid, granules, and injections. The pharmaceutical composition can be administered orally or parenterally (e.g., intravenously, subcutaneously, topically, etc.). The administration dose may be suitably adjusted depending on the age, sex, and type of disease of the patient, and is generally about 1-200 mg per day. The present disclosure also provides use of the compounds, the pharmaceutically acceptable salts thereof, the stereoisomers thereof, or the pharmaceutical composition described above in the preparation of a TAAR, particularly TAAR1 agonist medicament.

The present disclosure also provides use of the compounds, the pharmaceutically acceptable salts thereof, the stereoisomers thereof, or the pharmaceutical composition described above in the preparation of a medicament for treating TAAR, particularly TAAR1-mediated related diseases.

The present disclosure also provides use of the compounds, the pharmaceutically acceptable salts thereof, the stereoisomers thereof, or the pharmaceutical composition described above for preventing and/or treating TAAR, particularly TAAR1-mediated related diseases.

The present disclosure provides a method for treating, preventing and/or controlling various central nervous system (CNS)-related diseases or symptoms using the compounds, the pharmaceutically acceptable salts, the stereoisomers, or the compositions thereof provided by the present disclosure. The present disclosure also provides use of the compounds, the pharmaceutically acceptable salts, the stereoisomers, or the compositions thereof provided by the present disclosure in the preparation of a medicament for treating, preventing and/or controlling various central nervous system (CNS)-related diseases or symptoms. The central nervous system (CNS) diseases or symptoms include, but are not limited to: schizophrenia, schizophrenia spectrum disorder, acute schizophrenia, chronic schizophrenia, NOS schizophrenia, psychotic disorder, schizoid personality disorder, schizotypal personality disorder, delusional disorder, psychosis, mental disorder, brief psychotic disorder, shared psychotic disorder, psychotic disorder due to a physical disease, drug (e.g., cocaine, alcohol, and amphetamine)-induced psychosis, psychoaffective disorder, aggressive delirium, Parkinson psychosis, excitative psychosis, Tourette syndrome, organic or NOS psychosis, epilepsy, epileptic seizures, agitation, post-traumatic stress disorder, behavioral disorder, neurodegenerative disease, Alzheimer disease, Parkinson's disease, dyskinesias, Huntington disease, dementia, affective disorder, anxiety, affective psychosis (e.g., depression, e.g., major depressive disorder and dysthymia; bipolar disorder, e.g., bipolar depression; manic disorder; seasonal affective psychosis; attention deficit disorder (ADD), and attention deficit hyperactivity disorder (ADHD)), obsessive compulsive disorder, vertigo, pain (e.g., neuropathic pain, neuropathic pain susceptibility state, and inflammatory pain), fibromyalgia, migraine, cognitive impairment, movement disorder, restless leg syndrome (RLS), multiple sclerosis, substance (e.g., nicotine and cocaine) abuse, stress related disorder (e.g., acute stress disorder, post-traumatic stress disorder, and adjustment disorder), sexual dysfunction, eating disorder, disorders and malfunction of body temperature homeostasis, sleep disorder, sleep apnea, narcolepsy, excessive daytime sleepiness, jet lag, drowsiness side effects of medications, insomnia, sleep and circadian rhythm disorder, energy consumption and assimilation disorder, emesis, Lesche-Nyhane disease, Wilson's disease, autism, Huntington's chorea, and premenstrual dysphoria. The present disclosure provides a method for treating, preventing and/or controlling cardiovascular or metabolic diseases using the compounds, the pharmaceutically acceptable salts, the stereoisomers, or the compositions thereof provided by the present disclosure. The present disclosure also provides use of the compounds, the pharmaceutically acceptable salts, the stereoisomers, or the compositions thereof provided by the present disclosure in the preparation of a medicament for treating, preventing and/or controlling cardiovascular or metabolic diseases. The cardiovascular or metabolic diseases include, but are not limited to: diabetes, diabetic complications, obesity, dyslipidemia, and hypertension.

### BRIEF DESCRIPTION OF THE DRAWINGS

FIG. 1: pharmacodynamic evaluation of compounds 1-b and L27 in a mouse overactivity model.
FIG. 2: pharmacodynamic evaluation of compounds 1-b and 2-a in a mouse overactivity model.

### DEFINITION AND DESCRIPTION

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is described herein in terms of the compounds, compositions, and/or dosage forms, which are suitable for use in contact with human and animal tissues within the scope of reliable medical judgment, with no excessive toxicity, irritation, allergic response, or other problems or complications, and commensurate with a reasonable benefit/risk ratio.

The term "pharmaceutically acceptable salt" described herein refers to a salt of the compound of the present disclosure, which is prepared by reacting the compound having a specific substituent described herein with a relatively nontoxic acid or base. When the compound of the present disclosure contains a relatively basic functional group, an acid addition salt can be obtained by contacting the neutral form of such a compound with a sufficient amount of an acid in a pure solution or a suitable inert solvent. Examples of the pharmaceutically acceptable acid addition salts include inorganic acid salts and organic acid salts, and also include salts of amino acids such as arginine, and salts of organic acids such as glucuronic acid. Certain specific compounds of the present disclosure contain basic functional group that allow the compounds to be converted into any acid addition salt.

Certain compounds of the present disclosure may have asymmetric carbon atoms (optical centers) or double bonds. Racemates, diastereoisomers, geometric isomers, and individual isomers are all included within the scope of the present disclosure.

The compounds disclosed herein may be present in a specific geometric isomer or stereoisomer form. All such compounds are contemplated herein, including cis and trans isomers, (-)- and (+)-enantiomers, (R)- and (S)-enantiomers, diastereoisomers, (*D*)-isomers, (*L*)-isomers, and racemic mixtures and other mixtures thereof, such as a mixture enriched in enantiomer or diastereoisomer, all of which are encompassed within the scope of the present disclosure. Substituents such as alkyl may have an additional asymmetric carbon atom. All such isomers and mixtures thereof are included within the scope of the present disclosure.

The pharmaceutically acceptable salt of the present disclosure can be synthesized from the parent compound containing an acid or a basic moiety by conventional chemical methods. In general, the salt is prepared by reacting the free acid or base form of the compound with a stoichiometric amount of an appropriate base or acid in water or an organic solvent or a mixture thereof.

The term "pharmaceutically acceptable carrier" refers to any preparation or carrier medium representative of a carrier that is capable of delivering an effective amount of an active substance of the present disclosure, does not interfere with the biological activity of the active substance, and has no toxic side effects on the host or patient, including, but not limited to: binders, fillers, lubricants, disintegrants, wetting agents, dispersing agents, solubilizers, suspending agents, and the like.

The present disclosure is intended to include all isotopes of atoms present in the compounds of the present disclosure. Isotopes include those atoms having the same number of atoms but different mass numbers. By way of general example and without limitation, isotopes of hydrogen include deuterium and tritium. Isotopes of carbon include ¹³C and ¹⁴C. Isotopically-labeled compounds of the present disclosure can generally be prepared by conventional techniques known to those skilled in the art or by processes analogous to those described herein using an appropriate isotopically-labeled reagent in place of the non-labeled reagent otherwise used.

Unless otherwise specified, the term "alkyl" is used to refer to linear or branched saturated hydrocarbyl, which may be monosubstituted (e.g., -CH₂F) or polysubstituted (e.g., -CF₃), and may be monovalent (e.g., methyl), divalent (e.g., methylene), or multivalent (e.g., methine). For example, C₁-C₁₂ represents 1-12 carbons, and C₁₋₁₂ is selected from the group consisting of C₁, C₂, C₃, C₄, C₅, C₆, C₇, C₈, C₉, C₁₀, C₁₁, and C₁₂. Examples of alkyl include methyl (Me), ethyl (Et), propyl (e.g., n-propyl and isopropyl), butyl (e.g., n-butyl, isobutyl, s-butyl, and t-butyl), pentyl (e.g., n-pentyl, isopentyl, neopentyl, and 1-ethylpropyl), hexyl (e.g., n-hexyl, isohexyl, 1,1-dimethylbutyl, 2,2-dimethylbutyl, 3,3-dimethylbutyl, and 2-ethylbutyl), n-heptyl, n-octyl, n-nonyl, n-decyl, n-undecyl, n-dodecyl, and the like.

Unless otherwise specified, the term "halo" or "halogen" by itself or as part of another substituent refers to a fluorine, chlorine, bromine, or iodine atom.

Unless otherwise specified, "haloalkyl" is used to refer to monohalogenated/polyhalogenated linear or branched alkyl. Typical haloalkyl groups include C₁₋₆ haloalkyl, such as haloalkyl of C₁, C₂, C₃, C₄, C₅, and C₆. For example, examples of C₁-C₆ haloalkyl include, but are not limited to: fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, pentafluoroethyl, and pentachloroethyl.

Unless otherwise specified, "alkoxy" represents the above alkyl groups (including cycloalkyl or haloalkyl) having the specified number of carbon atoms connected through an oxygen bridge. Typical alkoxy groups include C₁₋₆ alkoxy, e.g., alkoxy of C₁, C₂, C₃, C₄, C₅, and C₆, cycloalkoxy of C₃, C₄, C₅, and C₆, and haloalkoxy of C₁, C₂, C₃, C₄, C₅, and C₆. Examples of alkoxy include, but are not limited to: methoxy, ethoxy, n-propoxy, isopropoxy, n-butoxy, s-butoxy, t-butoxy, n-pentyloxy, S-pentyloxy, hexyloxy, and 2-ethylbutoxy. Examples of cycloalkoxy include, but are not limited to: cyclopropoxy, cyclobutoxy, cyclopentyloxy, and cyclohexyloxy. Examples of haloalkoxy include, but are not limited to: fluoromethoxy, chloromethoxy, difluoromethoxy, dichloromethoxy, trifluoromethoxy, trichloromethoxy, 2-fluoroethoxy, 2,2-difluoroethoxy, 2,2-dichloroethoxy, 2,2,2-trifluoroethoxy, 2,2,2-trichloroethoxy, pentafluoroethoxy, and pentachloroethoxy.

Unless otherwise specified, "cycloalkyl" refers to a saturated or partially unsaturated monocyclic or polycyclic hydrocarbon substituent, which may be monosubstituted or polysubstituted and may be monovalent, divalent, or polyvalent. Examples of these cycloalkyl groups include, but are not limited to, cyclopropyl, cyclobutyl, cyclopentyl, cyclohexyl, and the like.

Unless otherwise specified, "aryl" refers to a 6- to 10-membered carbon monocyclic or fused polycyclic (rings sharing a pair of adjacent carbon atoms) group having a conjugated π-electron system such as phenyl and naphthyl, preferably phenyl.

Unless otherwise specified, the term "bond/single bond" refers to a chemical bond between atoms for interconnection or interaction, such as an ionic bond, a covalent bond, and a coordination bond; the "bond/single bond" in the molecular structure of an organic compound is usually a covalent bond. When one or more of the variables between two atoms/groups are defined as "bond/single bond", it is generally meant that the two atoms/groups are directly connected in a "bond/single bond".

"Prodrug" refers to a compound form that can be metabolized *in vivo* to become biologically active.

If the compound of the present disclosure relates to an absolute configuration, this absolute configuration may be confirmed by means of conventional techniques in the art. For example, in single crystal X-ray diffraction (SXRD), intensity data of diffraction of the single crystal grown are collected with a Bruker D8 venture diffractometer, with the light source being Cu-Kα radiation and the scanning mode being φ/ω scanning; after related data are collected, a direct method (Shelxs97) is further employed to analyze the crystal structure, and thus the absolute configuration can be confirmed. The instrument used for nuclear magnetic hydrogen spectrum detection of the compounds of the present disclosure is Bruker/AVANCE NEO 400MHz.

Compounds are named either manually or by ChemDraw^{®} software, and supplier's catalog names are given for commercially available compounds.

### DETAILED DESCRIPTION

The present disclosure will be further illustrated with reference to specific examples and test examples, but the scope of the present disclosure is not limited in any way.

### Example 1

### Synthetic route:

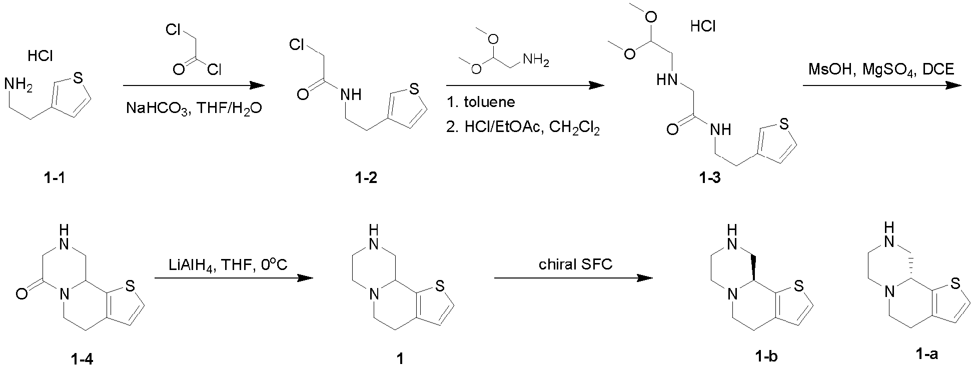

Compound 1-1 (5.00 g, 30.6 mmol, 1.00 eq) and NaHCO₃ (5.65 g, 67.2 mmol, 2.61 mL, 2.20 eq) were dissolved in THF (100 mL) and water (10.0 mL), and the mixture was cooled to 0-5 °C. Chloroacetyl chloride (5.31 g, 47.05 mmol, 1.10 eq) was added dropwise. After the dropwise addition was completed, the mixture was naturally warmed to 20 °C and stirred for 24 h. NaHCO₃ (2.57 g, 30.6 mmol, 1.00 eq) and compound chloroacetyl chloride (3.45 g, 30.6 mmol, 1.00 eq) were additionally added. After the addition was completed, the reaction mixture was stirred at 20 °C for 48 h. The reaction was completed as detected by LCMS. The reaction mixture was concentrated under reduced pressure to give a crude product, and the crude product was dissolved in EtOAc (150 mL) and water (100 mL). The organic phase was separated, and the aqueous phase was extracted once with EtOAc (100 mL). The organic phases were combined, washed once with brine (50.0 mL), dried over anhydrous Na₂SO₄, and concentrated under reduced pressure to give a crude product. The crude product was subjected to silica gel column chromatography (petroleum ether:ethyl acetate = 5:1) and concentrated to dryness to give compound 1-2 (3.00 g, 13.6 mmol, yield: 44.4%) as a white solid.

Compound 1-2 (3.00 g, 14.7 mmol, 1.00 eq) and aminoacetaldehyde dimethyl acetal (3.25 g, 30.9 mmol, 3.37 mL, 2.10 eq) were dissolved in anhydrous toluene (30.0 mL), and the mixture was heated to 115 °C and stirred for 2 h. The reaction was completed as detected by LCMS. The reaction mixture was cooled to 0-5 °C, and a large amount of solid was precipitated. The mixture was filtered, and the filter cake was washed with EtOAc (30.0 mL). The filtrate was washed with brine (30.0 mL). The mixture was dried over anhydrous Na₂SO₄ and concentrated under reduced pressure to leave about 30.0 mL of solvent. The remaining organic phase was cooled to 0-5 °C. HCl/EtOAc (4 M) was added dropwise to adjust the pH to 2-3, and a large amount of solid was precipitated. The mixture was filtered. The filter cake was washed with EtOAc (5.00 mL) and drained, and the solid was collected. The solid was concentrated under reduced pressure to remove the solvent to give compound 1-3 (3.20 g, 9.78 mmol, yield: 66.4%) as an off-white solid.

Compound 1-3 (2.63 g, 8.52 mmol, 1.00 eq) was suspended in DCE (130 mL). MgSO₄ (2.05 g, 17.0 mmol, 2.00 eq) and MsOH (4.91 g, 51.1 mmol, 3.64 mL, 6.00 eq) were added, and the reaction mixture was heated to 85 °C and stirred for 3 h. The reaction of compound 3 was completed as detected by LCMS. The reaction mixture was cooled to 20 °C. Water (100 mL) was added. The pH was adjusted to 9-10 with an aqueous Na₂CO₃ solution. Insoluble salts were removed by filtration, and the filter cake was washed with CH₂Cl₂ (150 mL) and water (100 mL). The filtrate was separated, and the aqueous phase was extracted twice with CH₂Cl₂ (150 mL). The organic phases were combined, washed with brine (50.0 mL), dried over anhydrous Na₂SO₄, and concentrated to dryness under reduced pressure to give a crude product of compound 1-4 (2.00 g) as a white solid.

Compound 1-4 (1.49 g, 7.15 mmol, 1.00 eq) was dissolved in anhydrous THF (31 mL). LiAlH₄ (543 mg, 14.3 mmol, 2.00 eq) was added in portions at 20 °C, and the reaction was warmed to 66 °C and stirred for 1 h. The MS of the product was detected by LCMS. The reaction mixture was quenched by dropwise adding water (1.50 mL) under nitrogen atmosphere, and the reaction mixture was concentrated to dryness by rotary evaporation. The crude product was subjected to silica gel column chromatography (dichloromethane:methanol = 100:0 - 96:4) to give a yellow oil. The product was separated by preparative chromatography and lyophilized to give compound 1. Chiral resolution (column: DAICEL CHIRALPAK AY-H (250 mm × 30 mm, 10 µm); mobile phase: A: CO₂, B:(0.1% NH₃H₂O EtOH); B%: 30%-30%, 4 min) was performed to give compound 1-a (retention time: 1.133 min, 265 mg, 1.35 mmol, yield: 18.9%) as a white solid, and compound 1-b (retention time: 1.368 min, 275 mg, 1.39 mmol, yield: 19.4%) as a white solid.

Compound 1-a: ¹H NMR:(400 MHz, CDCl₃) δ 7.11 (d*, J* = 5.2 Hz, 1H), 6.80 (d, *J* = 5.2 Hz, 1H), 3.34 - 3.28 (m, 2H), 3.06 - 2.89 (m, 5H), 2.83 - 2.80 (m, 1H), 2.66 - 2.62 (m, 2H), 2.53 - 2.48 (m, 1H). MS (ESI) m/z = 195.1 [M+H] ⁺.

Compound 1-b: ¹H NMR: (400 MHz CDCl₃)δ 7.11 (d, *J* = 5.2 Hz, 1H), 6.80 (d, *J* = 5.2 Hz, 1H), 3.36 - 3.29 (m, 2H), 3.07 - 2.90 (m, 5H), 2.83 - 2.80 (m, 1H), 2.66 - 2.62 (m, 2H), 2.54 - 2.48 (m, 1H). MS (ESI) m/z = 195.1 [M+H] ⁺.

### Example 2

Compound 2-a and compound 2-b were obtained using a synthesis method similar to that of compound 1 and the same resolution method. The characterization data for compounds 2-a (retention time: 0.707 min) and 2-b (retention time: 1.074 min) is listed in the table below:

| Compound No./structure | ¹H NMR | MS |
|---|---|---|
| 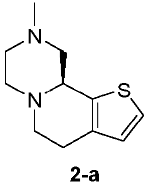 | ¹H NMR: (400 MHz, CDCl₃) δ 7.15 (d, J = 5.2 Hz, 1H), 6.82 (d, J = 5.2 Hz, 1H), 3.44 - 3.38 (m, 2H), 3.09 - 2.91 (m, 5H), 2.83 - 2.80 (m, 1H), 2.68 - 2.64 (m, 2H), 2.53 - 2.48 (m, 1H), 2.21(s, 3H). | MS (ESI) m/z = 209.1 [M+H]⁺ |
| 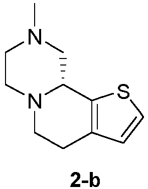 | ¹H NMR: (400 MHz, CDCl₃) δ 7.14 (d, J = 5.2 Hz, 1H), 6.82 (d, J = 5.2 Hz, 1H), 3.46 - 3.39 (m, 2H), 3.09 - 2.92 (m, 5H), 2.83 - 2.80 (m, 1H), 2.68 - 2.64 (m, 2H), 2.54 - 2.48 (m, 1H), 2.21(s, 3H). | MS (ESI) m/z = 209.1 [M+H]⁺ |

### Example 3

### Synthetic route:

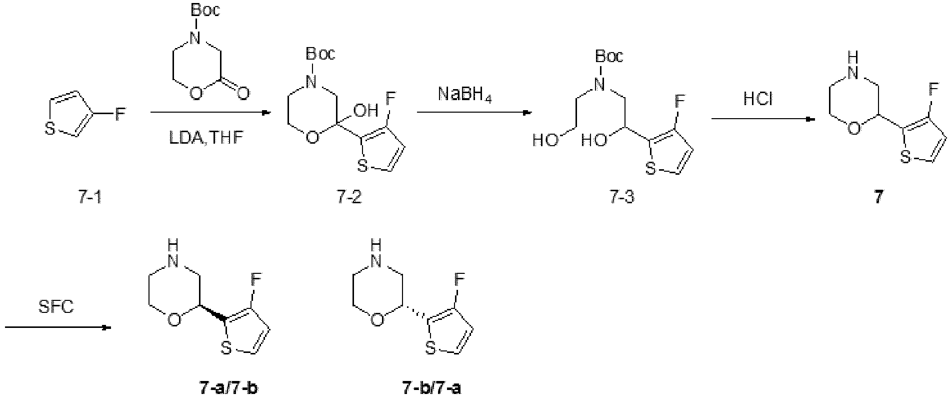

Compound 7-1 was dissolved in THF (20 mL), and LDA (2 M, 2.69 mL, 1.1 eq) was added dropwise to the reaction mixture described above at -70 °C. After the dropwise addition was completed, the reaction mixture was reacted at -70 °C for 3 h. The reaction was completed as monitored by LCMS, 5 mL of a saturated aqueous ammonium chloride solution was added to the reaction mixture to pH = 7.0, and the mixture was extracted twice with 20 mL of ethyl acetate. The extracts were combined and concentrated to dryness by rotary evaporation under vacuum to give compound 7-2 (0.8 g, 2.64 mmol, yield: 53.87%) as a colorless oily product, which was directly fed for the next reaction.

Compound 7-2 (0.8 g, 2.64 mmol, 1 eq) was dissolved in THF (10 mL), and sodium borohydride (200 mg, 5.29 mmol, 2.00 eq) was added to the reaction mixture at 25 °C. After the addition was completed, the mixture was stirred at room temperature for 2 h. The reaction was completed as monitored by LCMS. The reaction mixture was quenched with a saturated aqueous ammonium chloride solution (15 mL) and extracted with ethyl acetate (10 mL × 2 times). The organic phases were combined and concentrated to dryness under vacuum to give compound 7-3 (0.8 g, 2.52 mmol, yield: 95.37%) as a colorless oily product, which was directly fed for the next step.

Compound 7-3 was dissolved in H₂O (3 mL), and HCl (6 M, 5.46 mL, 50 eq) was added thereto. The mixture was stirred at 25 °C for 16 h. The reaction was completed as monitored by LCMS. 10 mL of a saturated aqueous sodium bicarbonate solution was added, and the mixture was extracted twice with ethyl acetate (10 mL × 2 times). The organic phase was concentrated to dryness under vacuum, and separated and purified by preparative liquid chromatography to give compound 7. Chiral resolution (column: Daicel ChiralPak IG (250 × 30 mm, 10 µm); mobile phase: A: CO₂, B:(0.1% NH₃H₂O EtOH); B%: 35%-35%, 4 min) was performed to give compound 7-a (retention time: 0.802 min, 15.9 mg, yield: 12.7%) as a yellow oil, and compound 7-b (retention time: 0.866 min, 27.7 mg, yield: 22.1%) as a yellow oil.

Compound 7-a: ¹H NMR (400MHz, CDCl₃) δ 7.13 (m, 1H), 6.75 (d, J = 5.6 Hz, 1H), 4.84 (dd, J = 2.4, 10.0 Hz, 1H), 3.99 (dd, J = 2.4, 10.0 Hz, 9.6 Hz, 1H), 3.76 (m, 1H), 3.15 (dd, J = 2.4 Hz, 10.0 Hz, 9.6 Hz, 1H), 2.99-2.87 (m, 3 H), 2.18-2.03(m, 1H). MS (ESI) m/z = 188.1 [M+H] ⁺.

Compound 7-b: ¹H NMR (400MHz, CDCl₃) δ 7.14 (m, 1H), 6.75 (d, J = 5.6 Hz, 1H), 4.86 (dd, J = 2.4, 10.0 Hz, 1H), 3.99 (dd, J = 2.4, 10.0 Hz, 9.6 Hz, 1H), 3.79 (m, 1H), 3.17 (dd, J = 2.4 Hz, 10.0 Hz, 9.6 Hz, 1H), 3.01-2.91 (m, 3 H), 2.16-2.02(m, 1H). MS (ESI) m/z = 188.1 [M+H] ⁺.

### Example 4

### Synthetic route:

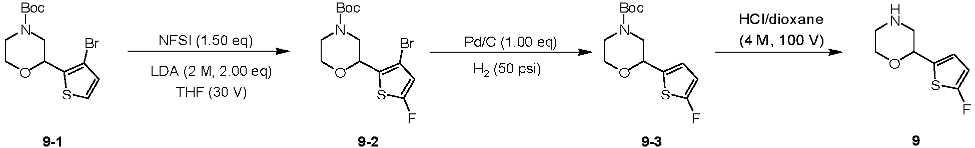

Compound 9-1 (1.00 g, 2.87 mmol, 1.00 eq) was dissolved in THF (25.0 mL). LDA (2 M, 2.87 mL, 2.00 eq) was added dropwise at -70 °C. The reaction mixture was stirred at -70 °C for 2 h. NFSI (1.36 g, 4.31 mmol, 1.50 eq) was dissolved in THF (10.0 mL) and added to the system, and the system was stirred at -70 to 20 °C for 13 h. The MS of the product was detected by LCMS. The LDA was quenched by dropwise adding an NH₄Cl solution to the reaction mixture at 0 °C, and then the reaction mixture was extracted 3 times with 40.0 mL of EtOAc. The organic phase was washed with saturated brine (20.0 mL), and finally dried over anhydrous Na₂SO₄. The organic phase was concentrated and subjected to column chromatography (petroleum ether:ethyl acetate = 95:5) to give intermediate 9-2 (450 mg, 1.05 mmol, yield: 36.7%) as an oily product.

Intermediate 9-2 (500 mg, 1.37 mmol, 1.00 eq) was dissolved in THF (35.0 mL), and Pd/C (820 mg, 1.37 mmol, 10% purity, 1.00 eq) was added. The reaction mixture was stirred at 50 °C for 20 h under nitrogen atmosphere. The MS of the product was detected by LCMS. The reaction mixture was filtered through celite to remove Pd/C and washed with THF. The organic phase was concentrated and separated by preparative chromatography to give intermediate 9-3 (30.0 mg, 103 µmol, yield: 7.57%) as an oily product.

Intermediate 9-3 (30.0 mg, 102 µmol, 1.00 eq) was dissolved in HCl/dioxane (3.00 mL) and stirred at 25 °C for 12 h. The MS of the product was detected by LCMS. The pH of the reaction mixture was adjusted to 7-8 with a NaHCO₃ solution, and the reaction mixture was extracted 3 times with 10.0 mL of CH₂Cl₂. The organic phase was washed with saturated brine (30.0 mL), and finally dried over anhydrous Na₂SO₄. The organic phase was concentrated to give compound 9 (16.0 mg, 79.2 µmol, yield: 77.5%) as an oily product.

¹H NMR (400MHz, CDCl₃)δ 6.52 - 6.63 (m, 1 H), 6.32 (dd, J = 3.94, 1.70 Hz, 1 H), 4.59 (dt, J = 9.98, 2.20 Hz, 1 H), 3.94 - 4.02 (m, 1 H), 3.75 (m, 1 H), 3.14 (dd, J = 12.26, 2.00 Hz, 1 H), 2.84 - 2.99 (m, 3 H). MS (ESI) m/z = 188.1 [M+H] ⁺.

### Example 5

### Synthetic route:

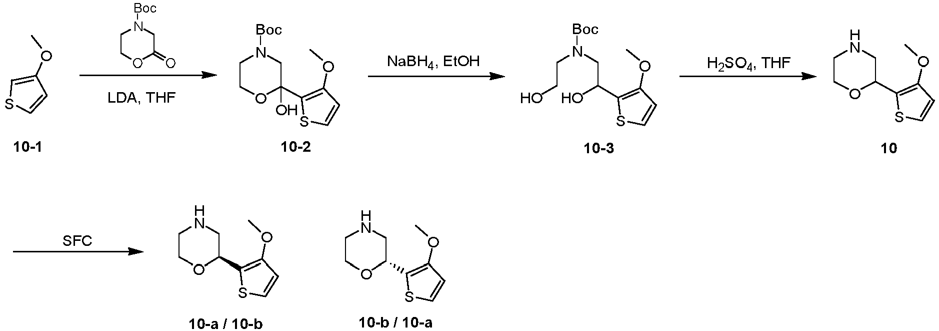

Compound 10-1 (1.00 g, 8.76 mmol, 1.00 *eq)* was dissolved in anhydrous THF (10.0 mL). The mixture was purged with nitrogen three times and cooled to -65 to -60 °C. Lithium diisopropylamide (2 M, 4.82 mL, 1.10 eq) was measured using a syringe, and added dropwise to the reaction mixture within 10 min with the temperature controlled at -65 °C to -60 °C. The reaction mixture was stirred for 30 min with the temperature controlled at - 65 °C to -60 °C. t-Butyl-2-oxopyran-4-carboxylate (1.59 g, 7.88 mmol, 0.90 eq) was weighed out, dissolved in anhydrous THF (4.00 mL), and added dropwise to the reaction mixture within 10 min with the temperature controlled at -65 °C to -60 °C. After the dropwise addition was completed, the reaction mixture was naturally warmed to 20 °C and stirred for 2 h. The reaction of compound 10-1 was completely as detected by LCMS. The reaction was quenched by dropwise adding an aqueous NH₄Cl solution (5.0 mL) under nitrogen atmosphere, diluted with water (20.0 mL), and extracted three times with EtOAc (30.0 mL). The organic phases were combined, washed with brine (30.0 mL), dried over anhydrous Na₂SO₄, and concentrated under reduced pressure to give a crude product. The crude product was subjected to column chromatography (petroleum ether:EtOAc = 2:1 to 1:1) and concentrated to dryness by rotary evaporation to give compound 10-2 (1.00 g, yield: 35.1%) as a yellow oil. Compound 10-2 (500 mg, 1.59 mmol, 1.00 eq) was dissolved in EtOH (5.00 mL) and cooled to 0 °C. NaBH₄ (240 mg, 6.34 mmol, 4.00 eq) was weighed out and added to the reaction mixture in portions. The reaction was naturally warmed to 20 °C and stirred for 2 h. The reaction of compound 10-2 was completed as detected by LCMS. The reaction mixture was slowly poured into water (5.00 mL) and extracted three times with EtOAc (15.0 mL). The organic phases were combined, washed with brine (45.0 mL), dried over anhydrous Na₂SO₄, and concentrated under reduced pressure to give compound 10-3 (436 mg, 1.15 mmol, yield: 72.8%) as a colorless oil. Compound 10-3 (250 mg, 788 µmol, 1.00 eq) was dissolved in anhydrous THF (10.0 mL) and cooled to 0 °C. H₂SO₄ (1.55 g, 15.8 mmol, 840 µL, 20.0 eq) was added dropwise. The reaction mixture was slowly warmed to 25 °C and stirred for 5 h. The reaction of compound 10-3 was completed as shown by LCMS detection. The MS of the product was detected. The reaction mixture was added slowly to ice water (20.0 mL) and extracted twice with EtOAc (20.0 mL). The organic phases were combined, washed once with brine (20.0 mL), dried over anhydrous Na₂SO₄, and filtered. The filtrate was concentrated to dryness under reduced pressure to give a crude product. The crude product was subjected to silica gel column chromatography (dichloromethane:methanol = 100:1 to 10:1) and concentrated to dryness by rotary evaporation to give compound 10. Chiral resolution (column: Daicel ChiralPak IG (250 × 30 mm, 10 µm); mobile phase: A: CO₂, B:(0.1% NH₃H₂O EtOH); B%: 35%-35%, 4 min) was performed to give compound 10-a (retention time: 0.933 min, 26.0 mg, yield: 14.9%) as a yellow oil, and compound 10-b (retention time: 1.080 min, 28.0 mg, yield: 16.6%) as a yellow oil.

Compound 10-a: ¹H NMR: (400 MHz DMSO-*d₆*) *δ* 7.34 (d, J = 5.6 Hz, 1H), 6.94 (d, J = 5.6 Hz, 1H), 4.62 (dd, J1 = 10.0 Hz, J2 = 2.4 Hz, 1H), 3.78 - 3.76 (s, 1H), 3.76 (s, 3H), 3.55 - 3.50 (m, 1H), 2.88 - 2.85 (m, 1H), 2.68 - 2.65 (m, 2H), 2.56 - 2.53 (m, 1H). MS (ESI) m/z = 200.1 [M+H] ⁺.

Compound 10-b: ¹H NMR: (400 MHz DMSO-*d₆*) *δ* 7.36 (d, J = 5.2 Hz, 1H), 6.96 (d, J = 5.6 Hz, 1H), 4.65 - 4.64 (m, 1H), 3.80 - 3.77 (m, 1H), 3.77 (s, 3H), 3.58 - 2.54 (m, 1H), 2.29 - 2.88 (m, 1H), 2.72 - 2.69 (m, 2H), 2.60 - 2.57 (m, 1H). MS (ESI) m/z = 200.1 [M+H] ⁺.

### Example 6

Compound 8, compound 11-a (retention time: 1.469 min), compound 11-b (retention time: 2.110 min), compound 12-a (retention time: 1.392 min) and compound 12-b (retention time: 1.701 min) were obtained using a synthesis method similar to that of compound 10 and the same resolution method. The characterization data for compound 8, compound 11-a, compound 11-b, compound 12-a, and compound 12-b is listed in the table below:

| Compound No./structure | ¹H NMR | MS |
|---|---|---|
| 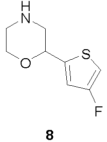 | ¹H NMR (400 MHz, CDCl₃) δ 7.13 (dd, J = 5.50, 3.74 Hz, 1 H), 6.75 (d, J = 5.50 Hz, 1 H), 4.85 (dd, J = 10.20, 2.20 Hz, 1 H), 3.95 - 4.03 (m, 1 H), 3.77 (m, 1 H), 3.16 (dd, J = 12.38, 2.24 Hz, 1 H), 2.87 - 3.03 (m, 3 H). | MS (ESI) m/z = 188.1 [M+H] ⁺ |
| 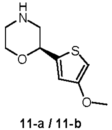 | ¹H NMR: (400 MHz CDCl₃) *δ* 6.64 (s, 1H), 6.17 (s, 1H), 4.62 (dd, *J₁* = 9.6 Hz, *J₂* = 2.0 Hz, 1H), 4.01 - 3.98 (m, 1H), 3.77 (s, 3H), 3.77 - 3.74 (m, 1H), 3.14 - 3.11 (m, 1H), 2.92 - 2.86 (m, 3H). | MS (ESI) m/z = 200.1 [M+H] ⁺ |
| 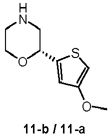 | ¹H NMR: (400 MHz CDCl₃) *δ* 6.64 (d, *J* = 1.2 Hz, 1H), 6.17 (d, *J* = 1.6 Hz, 1H), 4.64 (dd, *J₁* = 10.0 Hz, *J₂* = 2.0 Hz, 1H), 4.02 - 3.98 (m, 1H), 3.77 (s, 3H), 3.74 - 3.71 (m, 1H), 3.18 - 3.14 (m, 1H), 2.93 - 2.87 (m, 3H). | MS (ESI) m/z = 200.1 [M+H] ⁺ |
| 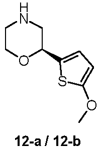 | ¹H NMR: (400 MHz CDCl₃) δ 6.61 (d, *J* = 4.0 Hz, 1H), 6.12 (s, *J* = 3.6 Hz, 1H), 4.42 (dd, *J₁* = 10.0 Hz, *J₂* = 2.4 Hz, 1H), 3.78 (s, 3H), 3.78 - 3.75 (m, 1H), 3.55 - 3.52 (m, 1H), 2.94 - 2.91 (m, 1H), 2.68 - 2.58 (m, 4H). | MS (ESI) m/z = 200.1 [M+H] ⁺ |
| 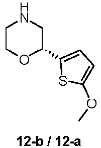 | ¹H NMR: (400 MHz CDCl₃) δ 6.61 (d, *J* = 3.6 Hz, 1H), 6.12 (s, *J* = 3.6 Hz, 1H), 4.42 (dd, *J₁* = 10.0 Hz, *J₂* = 2.0 Hz, 1H), 3.80(s, 3H), 3.80 - 3.75 (m, 1H), 3.55 - 3.52 (m, 1H), 2.95 - 2.92 (m, 1H), 2.68 - 2.56 (m, 4H). | MS (ESI) m/z = 200.1 [M+H] ⁺ |

### Example 7

### Synthetic route:

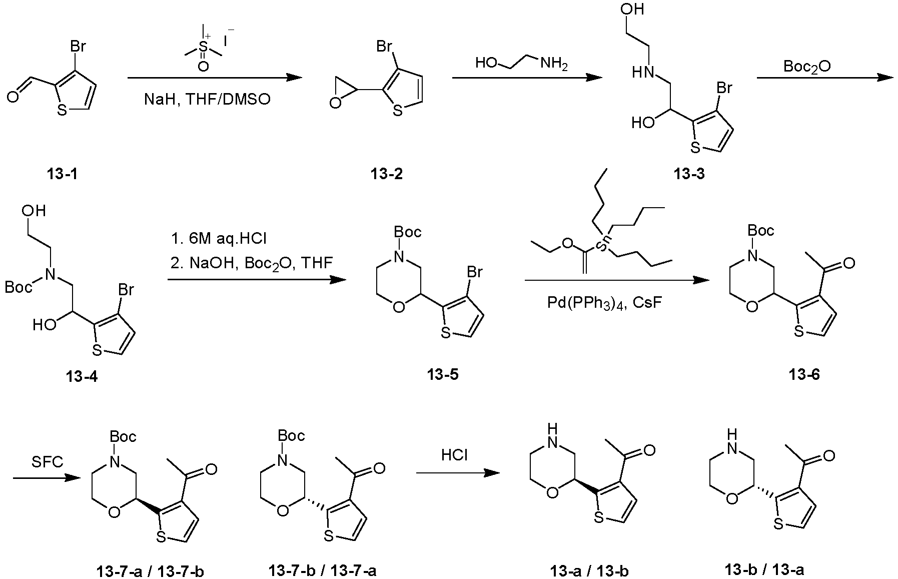

Trimethylsulfoxonium iodide (1.15 g, 5.23 mmol, 1.00 eq) was dissolved in dry DMSO (16.0 mL) and cooled to 0-5 °C. NaH (210 mg, 5.23 mmol, 60.0% purity, 1.00 eq) was added in portions, and the mixture was stirred for 1 h with the temperature controlled at 0 to 5 °C. Compound 13-1 (1.00 g, 5.23 mmol, 1.00 eq) was dissolved in anhydrous THF (8.00 mL) and added dropwise to the reaction mixture with the temperature controlled at 0 to 5 °C. The reaction mixture was naturally warmed to 20 °C and stirred for 2 h. The reaction of compound 13-1 was completed as detected by LCMS. The reaction mixture was slowly poured into ice water and extracted twice with petroleum ether (50 mL). The organic phases were combined, washed once with brine (30 mL), and dried over anhydrous Na₂SO₄ to give a solution of compound 13-2 in petroleum ether (100 mL). Ethanolamine (1.60 g, 26.2 mmol, 5.00 eq) was added to the solution of compound 13-2 in petroleum ether, and the reaction mixture was heated to 50 °C and stirred for 3 h. The reaction of compound 13-2 was completed as detected by LCMS. The reaction mixture was cooled to 20 °C, and Boc₂O (5.71 g, 26.2 mmol, 6.01 mL, 5.00 eq) was added in portions. The reaction mixture was stirred at 20 °C for 2 h. The reaction of compound 13-3 was completed as detected by LCMS. The reaction mixture was diluted with EtOAc (50.0 mL), washed once with water (50.0 mL), washed once with brine (30.0 mL), dried over anhydrous Na₂SO₄, and concentrated under reduced pressure to give a crude product. The crude product was purified by silica gel column chromatography to give compound 13-4 (300 mg, 819 µmol, yield: 15.7%) as a brown liquid.

Hydrochloric acid (6 M, 10.0 mL, 142 eq) was added to compound 13-4 (155 mg, 423 µmol, 1.00 eq), and the reaction was warmed to 110 °C and stirred for 3 h. The reaction of compound 13-4 was completed as shown by LCMS detection. The reaction mixture was adjusted to pH = 8-9 with an aqueous NaOH solution (2 M, 31.3 mL). Boc₂O (185 mg, 846 µmol, 194 µL, 2.00 eq) was added, and the reaction mixture was stirred at 20 °C for 2 h. The intermediate state reaction was completed as shown by LCMS detection. The reaction mixture was extracted three times with EtOAc (30 mL). The extracted organic phases were combined, washed once with brine (30 mL), dried once over anhydrous Na₂SO₄, and concentrated under reduced pressure to give a crude product. The crude product was subjected to silica gel column chromatography (eluent: petroleum ether:ethyl acetate = 5:1) and concentrated to dryness to give compound 13-5 (150 mg, 362 µmol, yield: 85.5%) as a yellow oil.

Compound 13-5 (150 mg, 431 µmol, 1.00 *eq*) and Pd(PPh₃)₄ (24.9 mg, 21.5 µmol, 0.05 *eq*) were dissolved in anhydrous toluene (5 mL), and dibutyl(1-ethoxyvinyl)pentylstannane (300 mg, 831 µmol, 280 µL, 1.93 *eq*) was added dropwise to the reaction mixture under nitrogen atmosphere. The reaction was warmed to 110 °C and stirred for 16 h. The reaction was not completed as detected by LCMS. The reaction was cooled to 20 °C, and CsF (393 mg, 2.58 mmol, 6.00 *eq*) and Pd(PPh₃)₄ (49.8 mg, 43.0 µmol, 0.10 eq) were added. The reaction was warmed to 110 °C and stirred for 16 h. The reaction of compound 13-5 was completed as detected by LCMS. The reaction mixture was filtered, the filter cake was washed with EtOAc (30.0 mL), and the filtrate was concentrated under reduced pressure to give a crude product. The crude product was subjected to silica gel column chromatography (eluent petroleum ether:ethyl acetate = 5:1) and chiral resolution (column: DaicelChiralPak IG (250 × 30 mm, 10um); mobile phase: A: CO₂, B: (0.1% NH₃H₂O MeOH); B%: 20%-20%, 4 min) to give compound 13-7-a (retention time: 0.901 min, 35.0 mg, 112 µmol, yield: 26.1%) as a white solid, and compound 13-7-b (retention time: 1.133 min, 35.0 mg, 112 µmol, yield: 26.1%) as a white solid.

Compound 13-7-a (30.0 mg, 96.3 µmol, 1.00 *eq*) was dissolved in MeOH (1.00 mL). HCl/dioxane (4 M, 241 µL, 10.0 *eq*) was added dropwise, and the reaction mixture was stirred at 20 °C for 16 h. The reaction of compound 13-7-a was completed as detected by LCMS. The reaction mixture was diluted with deionized water (20.0 mL). NaHCO₃ was added with stirring to adjust the pH to 7-8. The aqueous phase was extracted three times with CH₂Cl₂ (15.0 mL). The organic phases were combined, washed with brine (10.0 mL), dried over anhydrous Na₂SO₄, filtered, and concentrated under reduced pressure to give compound 13-a (20.0 mg, 90.9 µmol, yield: 94.3%) as a yellow solid.

Compound 13-7-b (35.0 mg, 112 µmol, 1.00 *eq*) was dissolved in MeOH (2.00 mL). HCl/dioxane (4 M, 281 µL, 10.0 *eq*) was added dropwise, and the reaction mixture was stirred at 20 °C for 16 h. The reaction of compound 13-7-b was completed as detected by LCMS. The reaction mixture was diluted with deionized water (20.0 mL). NaHCO₃ was added with stirring to adjust the pH to 7-8. The aqueous phase was extracted three times with CH₂Cl₂ (15.0 mL). The organic phases were combined, washed with brine (10.0 mL), dried over anhydrous Na₂SO₄, filtered, and concentrated under reduced pressure to give compound 13-b (20.0 mg, 90.7 µmol, yield: 80.7%) as a yellow solid.

Compound 13-a: ¹H NMR: (400 MHz CDCl₃) δ 7.36 (d, *J* = 5.6 Hz, 1H), 7.22 (d, *J* = 5.2 Hz, 1H), 5.35 (dd, *J₁* = 9.2 Hz, *J₁* = 2.4 Hz, 1H), 4.04 - 4.01 (m, 1H), 3.74 - 3.71 (m, 1H), 3.36 - 3.33 (m, 1H), 3.00 - 2.97 (m, 1H), 2.96 - 2.92 (m, 1H), 2.61 - 2.58 (m, 1H), 2.52 (s, 3H). MS (ESI) m/z = 212.1 [M+H] ⁺.

Compound 13-b: ¹H NMR: (400 MHz CDCl₃) δ 7.36 (d, *J* = 5.2 Hz, 1H), 7.22 (d, *J* = 5.2 Hz, 1H), 5.36 (dd, *J₁* = 9.6 Hz, *J₁* = 2.0 Hz, 1H), 4.05 - 4.01 (m, 1H), 3.75 - 3.73 (m, 1H), 3.37 - 3.34 (m, 1H), 3.00 - 2.97 (m, 1H), 2.97 - 2.93 (m, 1H), 2.61 - 2.56 (m, 1H), 2.52 (s, 3H). MS (ESI) m/z = 212.1 [M+H] ⁺.

### Example 8

### Synthetic route:

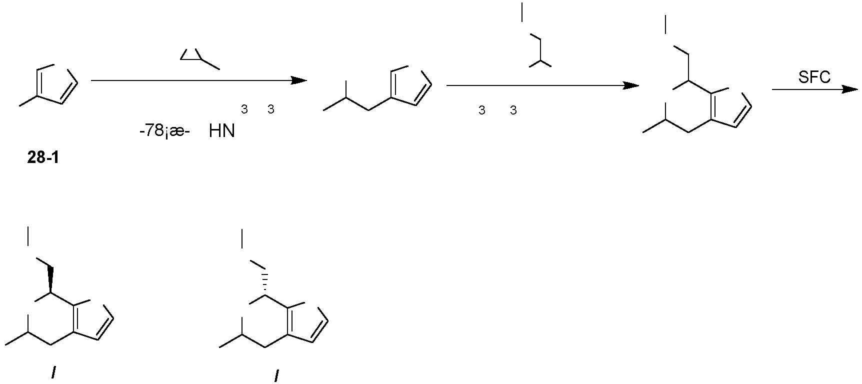

3-Bromothiophene (10.0 g, 61.3 mmol, 5.75 mL, 1 eq) was dissolved in tetrahydrofuran (150 mL). The solution was cooled to -78 °C, and n-butyllithium (4.32 g, 67.5 mmol, 27.0 mL, 1.10 eq) was then slowly added dropwise. After the addition, the mixture was stirred and reacted at this temperature for 1 h, 2-methyloxirane (7.12 g, 123 mmol, 8.60 mL, 2 eq) was then slowly added dropwise at -55 °C, and boron trifluoride diethyl ether (8.71 g, 61.3 mmol, 7.57 mL, 1 eq) was then added at -55 °C. The reaction mixture was stirred and reacted at -55 °C for 2 h. The reaction was completed as monitored by TLC. The reaction mixture was quenched with a saturated aqueous sodium bicarbonate solution at -78 °C and extracted with ethyl acetate. The organic phase was washed with a saturated aqueous sodium bicarbonate solution and saturated brine, dried over anhydrous sodium sulfate, filtered, and concentrated under reduced pressure to give a crude product. The crude product was purified by column chromatography (petroleum ether/ethyl acetate = 5/1 to 4/1). Compound 1-(thien-3-yl)propan-2-ol (compound 28-2) was obtained as a red oily compound (7.70 g, 54.1 mmol, yield: 88.3%).

The compound (methylamino)acetaldehyde dimethyl acetal was dissolved in a solution of 2-methyltetrahydrofuran (100 mL). Compound 1-(thien-3-yl)propan-2-ol (7.70 g, 54.1 mmol, 1 eq) was added at 25 °C. After the addition, the mixture was stirred at this temperature for 5 min, and trifluoromethanesulfonic acid (8.94 g, 59.6 mmol, 5.26 mL, 1.1 eq) was added. The resulting dark brown mixture was then heated to 80 °C and stirred and reacted for 1 h. The reaction was completed as detected by LCMS. The reaction mixture was quenched by slowly adding a saturated potassium hydroxide solution until pH > 7, and extracted with methyl t-butyl ether. The organic phase was washed with saturated brine, dried over anhydrous sodium sulfate, filtered, and concentrated under reduced pressure to give a crude product. The crude product was purified by high performance liquid chromatography to give compound 28, and resolved and purified by SFC (column: DAICEL CHIRALPAK AY-H (250 mm × 30 mm, 10 µm); mobile phase: A: CO₂, B: (0.1% NH₃H₂O MeOH); B%: 20%-20%, 4 min) to give 28-a (retention time: 0.812 min, 65.8 mg, 333 µmol, yield: 61.5%) as a yellow oily compound, and 28-b (0.954 min, 66.1 mg, 334 µmol, yield: 61.8%) as a yellow oily compound.

Compound 28-a: ¹H NMR: (400 MHz, DMSO-d6)δ 7.28 (d, *J* = 5.1 Hz, 1H), 6.93 (d, *J* = 5.3 Hz, 1H), 4.68 (td, *J* = 2.3, 4.6 Hz, 1H), 3.76 (ddd, *J* = 3.3, 6.2, 10.3 Hz, 1H), 3.49 - 3.39 (m, 1H), 2.92 (dd, *J* = 3.6, 12.2 Hz, 1H), 2.78 (td, *J* = 2.4, 15.9 Hz, 1H), 2.63 (dd, *J* = 7.8, 12.1 Hz, 1H), 2.53 (br dd, *J* = 2.3*,* 11.3 Hz, 1H), 2.32 (s, 3H), 1.28 (d, *J* = 6.1 Hz, 3H). MS (ESI) m/z = 198.1 [M+H]⁺.

Compound 28-b: ¹H NMR: (400 MHz, DMSO-d6)δ 7.28 (d, *J* = 5.1 Hz, 1H), 6.94 (d, *J* = 5.3 Hz, 1H), 4.72 - 4.65 (m, 1H), 3.76 (ddd, *J* = 3.2, 6.3, 10.3 Hz, 1H), 3.48 - 3.40 (m, 1H), 2.94 (dd, *J* = 3.6, 12.2 Hz, 1H), 2.79 (td, *J* = 2.4, 15.8 Hz, 1H), 2.64 (dd, *J* = 7.8, 12.3 Hz, 1H), 2.54 (br dd, *J* = 1.9, 10.7 Hz, 1H), 2.37 - 2.30 (m, 3H), 1.28 (d, *J* = 6.1 Hz, 3H). MS (ESI) m/z = 198.1 [M+H]⁺.

### Example 9

Compound 30, compound 39-a (retention time: 1.520 min), and compound 39-b (retention time: 2.120 min) were obtained using a synthesis method similar to that of compound 28 and the same resolution method. The characterization data for compound 30, compound 39-a, and compound 39-b is listed in the table below:

| Compound No./structure | ¹H NMR | MS |
|---|---|---|
| 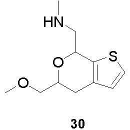 | ¹H NMR (400 MHz, CDCl₃) δ 7.12 (d, J = 4.8 Hz, 1H), 6.79 (d, J = 5.2 Hz, 1H), 4.90 - 4.86 (m, 1H), 3.95 - 3.92 (m, 1H), 3.63 - 3.61 (m, 1H), 3.57 - 3.54 (m, 1H), 3.44 (s, 3H), 3.08 - 3.07 (m, 1H), 2.89 - 2.87 (m, 1H), 2.77 - 2.76 (m, 2H) 2.49 (s, 3H). | MS (ESI) m/z = 228.1 [M+H] ⁺ |
| 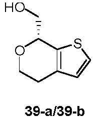 | ¹H NMR: (400 MHz, CDCl₃)δ 7.10 (d, *J* = 5.0 Hz, 1H), 6.77 (d, *J* = 5.0 Hz, 1H), 4.83 - 4.81 (m, 1H), 4.18 - 4.13 (m, 1H), 3.80 - 3.69 (m, 3H), 2.80 - 2.78 (m, 1H), 2.58 - 2.53 (m, 2H). | MS (ESI) m/z = 171.0 [M+H]⁺ |
| 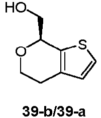 | ¹H NMR: (400 MHz, CDCl₃)δ 7.11 (d, *J* = 5.0 Hz, 1H), 6.77 (d, *J* = 5.0 Hz, 1H), 4.82 - 4.80 (m, 1H), 4.18 - 4.13 (m, 1H), 3.80 - 3.69 (m, 3H), 2.80 - 2.78 (m, 1H), 2.57 - 2.53 (m, 2H). | MS (ESI) m/z = 171.0 [M+H]⁺. |

### Example 10

### Synthetic route:

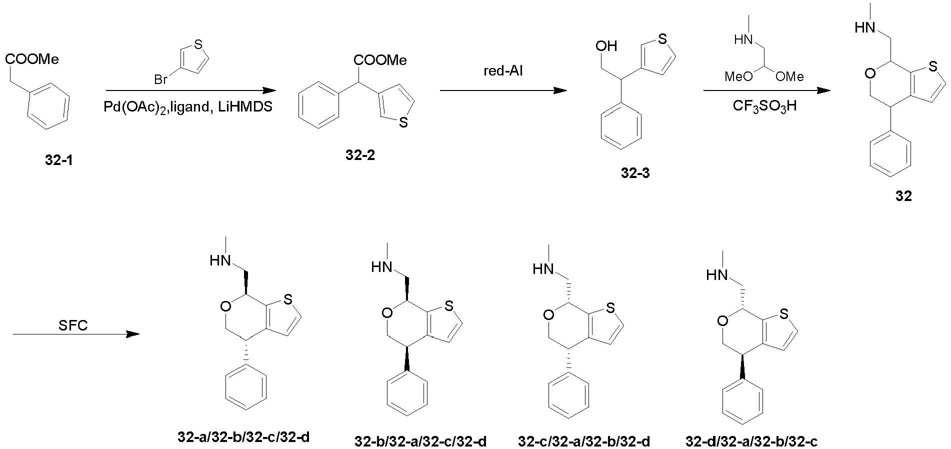

Pd(OAc)₂ (207 mg, 920 µmol, 0.03 eq) and DavePhos (724 mg, 1.84 mmol, 0.06 eq) were suspended in anhydrous toluene (100 mL) and purged with nitrogen three times. LiHMDS (1 M, 76.7 mL, 2.50 eq) was added dropwise to the reaction mixture at 20 °C within 10 min. Compound 32-1 (10.6 g, 70.5 mmol, 9.90 mL, 2.3 eq) and 3-bromothiophene (5.00 g, 30.7 mmol, 2.87 mL, 1.00 eq) were dissolved in toluene (20.0 mL), and added dropwise to the reaction mixture with the temperature controlled at -10 to 0 °C. The reaction was heated to 85 °C and stirred for 3 h. The reaction of the compound was completed as detected by LCMS. The reaction mixture was concentrated to dryness under reduced pressure. The crude product was dissolved in ethyl acetate (200 mL), washed with brine (50.0 mL), dried over anhydrous sodium sulfate, and concentrated under reduced pressure to give a crude product. The crude product was subjected to silica gel column chromatography (petroleum ether:ethyl acetate = 20:1) to give compound 32-2 (9.00 g, crude product) as a yellow solid.

Compound 32-2 (9.00 g, 38.7 mmol, 1.00 eq) was dissolved in anhydrous THF (100 mL) and cooled to 0-5 °C. Red-Al (33.6 g, 116 mmol, 32.3 mL, 70.0% purity, 3.0 eq) was added dropwise, and the reaction mixture was naturally warmed to 20 °C and stirred for 4 h. The reaction of compound 32-2 was completed as detected by LCMS. The reaction mixture was slowly poured into water (100 mL). The organic phase was washed with brine (50.0 mL), dried over anhydrous sodium sulfate, and concentrated under reduced pressure to give a crude product. The crude product was subjected to silica gel column chromatography (eluent: petroleum ether:ethyl acetate = 2:1) to give compound 32-3 (1.60 g, 3.84 mmol, 9.91% yield) as a colorless oil.

Compound 32-3 (1.60 g, 7.83 mmol, 1.00 eq) and 2,2-dimethoxy-N-methylethylamine (1.60 g, 13.4 mmol, 1.72 mL, 1.71 eq) were dissolved in 2-methyltetrahydrofuran (20.0 mL). TfOH (2.70 g, 18.0 mmol, 1.59 mL, 2.30 eq) was added dropwise, and the reaction mixture was warmed to 85 °C and stirred for 5 h. The reaction was completed as detected by LCMS. The reaction mixture was poured into water (50.0 mL), adjusted to pH = 7-8 with an aqueous sodium bicarbonate solution, and extracted twice with ethyl acetate (50.0 mL). The organic phases were combined, washed with brine (50.0 mL), dried over anhydrous sodium sulfate, and concentrated under reduced pressure to give a crude product. The crude product was subjected to silica gel column chromatography (dichloromethane:methanol = 10:1, Rf = 0.3) and resolved by SFC chiral resolution (column: DAICEL CHIRALPAK AY-H (250mm × 30mm, 10 µm); mobile phase: A: CO₂, B: (0.1% NH3H2O EtOH); B%: 20%-20%, 4 min) to give compound 32-a (retention time: 1.373 min, 251 mg, yield: 12.3%) as a brown oil, compound 32-b (retention time: 1.451 min, 56.0 mg, yield: 2.65%) as a brown oil, compound 32-c (retention time: 1.679 min, 51 mg, yield: 2.32%) as a brown oil, and compound 32-d (retention time: 1.850 min, 282 mg, yield: 13.6%) as a brown oil.

Compound 32-a: ¹ H NMR: (400 MHz CDCl₃) δ 7.34 - 7.27 (m, 3H), 7.18 - 7.16 (m, 2H), 7.13 (d, J = 4.8 Hz, 1H), 6.58 (d, J = 5.2 Hz, 1H), 5.05 - 5.02 (m, 1H), 4.29 - 4.25 (m, 1H), 4.21 - 4.18 (m, 1H), 3.67 - 3.61 (m, 1H), 3.01 - 2.98 (m, 2H), 2.54 (s, 3H). MS (ESI) m/z = 260.1 [M+H]⁺.

Compound 32-b: ¹H NMR: (400 MHz CDCl₃) δ 7.31 - 7.27 (m, 2H), 7.22 - 7.19 (m, 1H), 7.18 - 7.16 (m, 3H), 6.72 (d, J = 5.2 Hz, 1H), 4.99 - 4.96 (m, 1H), 4.14 - 4.07 (m, 2H), 3.99 - 3.97 (m, 1H), 3.08 - 2.92 (m, 2H), 2.54 (s, 3H). MS (ESI) m/z = 260.1 [M+H]⁺.

Compound 32-c: ¹H NMR: (400 MHz CDCl₃) δ 7.31 - 7.27 (m, 2H), 7.24 - 7.19 (m, 1H), 7.18 - 7.16 (m, 3H), 6.72 (d, J = 4.8 Hz, 1H), 4.98 - 4.95 (m, 1H), 4.14 - 4.07 (m, 2H), 3.99 - 3.97 (m, 1H), 3.08 - 2.98 (m, 2H), 2.53 (s, 3H). MS (ESI) m/z = 260.1 [M+H]⁺.

Compounds 32-d: ¹H NMR: (400 MHz CDCl₃) δ 7.34 - 7.30 (m, 3H), 7.18 - 7.16 (m, 2H), 7.13 (d, J = 4.8 Hz, 1H), 6.58 (d, J = 4.8 Hz, 1H), 5.05 - 5.02 (m, 1H), 4.29 - 4.25 (m, 1H), 4.21 - 4.18 (m, 1H), 3.67 - 3.61 (m, 1H), 3.04 - 2.95 (m, 2H), 2.54 (s, 3H). MS (ESI) m/z = 260.1 [M+H]⁺.

### Example 11

### Synthetic route:

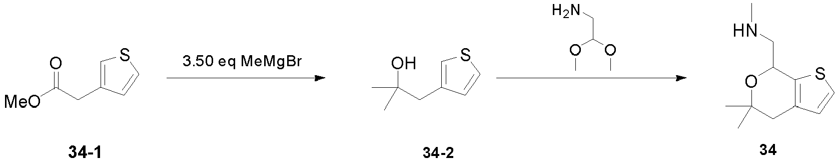

Compound 34-1 (2.00 g, 12.8 mmol, 1.00 eq) was dissolved in anhydrous THF (10.0 mL) and cooled to 0 °C. MeMgBr (3.00 M, 14.9 mL, 3.50 eq) was slowly added dropwise, and the reaction mixture was naturally warmed to 20 °C and stirred for 16 h. The reaction was completed as detected by TLC. The reaction was quenched with an aqueous ammonium chloride solution (300 mL) and extracted with a mixed solvent of dichloromethane:methanol = 10:1 (200 mL × 3). The resulting organic phase was washed with brine (100 mL × 1), dried over anhydrous sodium sulfate, and concentrated to dryness by rotary evaporation to give compound 34-2 (973 mg, 5.85 mmol, yield: 45.7%) as a colorless oil.

Compound 34-2 (476 mg, 3.05 mmol, 1.00 eq) and aminoacetaldehyde dimethyl acetal (363 mg, 3.05 mmol, 391 µL, 1.00 eq) were dissolved in anhydrous 2-MeTHF (5.00 mL). TfOH (549 mg, 3.66 mmol, 323 µL, 1.20 eq) was added dropwise, and the mixture was reacted at 55 °C for 3 h. LCMS showed that a product appeared and the starting material was completely consumed. The reaction mixture was directly concentrated to dryness by rotary evaporation and dried with 2-MeTHF (6.00 mL × 3) to give a crude product. The crude product was dissolved in 2-MeTHF (5.00 mL) and reacted at an internal temperature of 55 °C for 1 h. LCMS showed that the starting material disappeared and a product appeared. The reaction mixture was directly concentrated to dryness by rotary evaporation, and the crude product was adjusted to pH 9 with a sodium carbonate solution, and extracted five times with ethyl acetate (20.0 mL × 3) and dichloromethane (20.0 mL × 2). The organic phases were combined, dried over anhydrous sodium sulfate, and concentrated under reduced pressure to give a crude product. The crude product was subjected to column chromatography (dichloromethane:methanol = 100:0 to 93:7) and concentrated to dryness by rotary evaporation to give a crude product. The crude product was separated by preparative chromatography to give compound 34 (15.7 mg, 73.5 µmol, yield: 2.41%) as a brown oil.

¹H NMR (400 MHz CDCl₃) δ 7.09 (d, J = 4.8 Hz, 1H), 6.73 - 6.69 (m, 1H), 4.83 - 4.81 (m, 1H), 2.93 - 2.90 (m, 1H), 2.82- 2.81 (m, 1H), 2.60 - 2.59 (m, 1H), 2.47 - 2.43 (m, 4H), 1.31 (s, 3H), 1.15 (s, 3H). MS (ESI) m/z = 212.1 [M+H]⁺.

### Example 12

### Synthetic route:

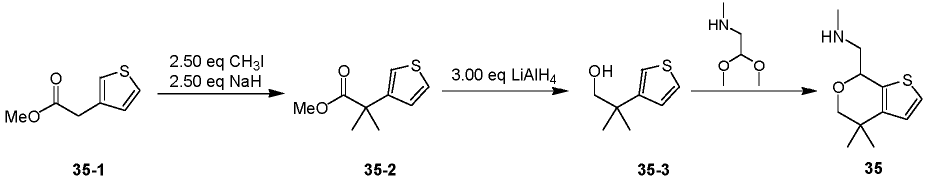

Compound 35-1 (5.00 g, 32.01 mmol, 1.00 eq) was dissolved in anhydrous THF (50 mL), and the reaction system was cooled to 0 °C. NaH (3.20 g, 80.0 mmol, 60.0% purity, 2.50 eq) was added in portions to the reaction system, and the reaction system was stirred at 0 °C for 0.5 h. Finally, CH₃I (11.3 g, 80.0 mmol, 4.98 mL, 2.50 eq) was slowly added dropwise to the reaction mixture at 0 °C and stirred at 20 °C for 15 h. TLC showed that the starting material disappeared and new spots were generated. The reaction mixture was cooled to 0 °C, quenched by dropwise adding H₂O (20.0 mL), and extracted 3 times with ethyl acetate (40.0 mL × 3). The organic phase was washed with NaCl (100 mL) and dried over anhydrous sodium sulfate to give a crude product. The crude product was subjected to column chromatography (petroleum ether:ethyl acetate = 100: 0 to 98:2) and concentrated to dryness by rotary evaporation to give compound 35-2 (3.94 g, 20.6 mmol, yield: 64.6%) as a colorless oil. Compound 35-2 (1.80 g, 9.77 mmol, 1.00 eq) was dissolved in anhydrous THF (18.0 mL), and the mixture was cooled to 0 °C. LiAlH₄ (1.11 g, 29.3 mmol, 3.00 eq) was added in portions, and the mixture was stirred at 20 °C for 2 h. LCMS showed that the starting material was consumed completely. TLC showed that the starting material was consumed completely and new spots were generated. The reaction mixture was cooled to 0 °C, quenched by dropwise adding H₂O (1.11 mL), 15% NaOH (1.11 mL) and H₂O (3.33 mL) sequentially, supplemented with H₂O (10.0 mL), and extracted 3 times with ethyl acetate (18.0 mL). The organic phase was dried over anhydrous sodium sulfate to give a crude product, and the crude product was subjected to column chromatography (petroleum ether:ethyl acetate = 100:0 to 92:8) and concentrated to dryness by rotary evaporation to give compound 35-3 (1.09 g, 6.82 mmol, yield: 69.8%) as a colorless oil.

Compound 35-3 (500 mg, 3.20 mmol, 1.00 eq) was dissolved in anhydrous 2-MeTHF (5.00 mL). (Methylamino)acetaldehyde dimethyl acetal (381 mg, 3.20 mmol, 411 µL, 1 eq) and TfOH (720 mg, 4.80 mmol, 339 µL, 1.50 eq) were added. The mixture was warmed to an internal temperature of 55 °C and reacted for 2 h. LCMS showed that a product appeared, and the reaction was performed for another 1.7 h. LCMS showed that a product appeared, and the reaction mixture was directly concentrated to dryness by rotary evaporation and dried with 2-MeTHF (6.00 mL × 3) to give a crude product. The crude product was dissolved in 2-MeTHF (5.00 mL) and reacted at an internal temperature of 55 °C for 1 h. LCMS showed that the starting material disappeared and a product appeared. The reaction mixture was directly concentrated to dryness by rotary evaporation, and the crude product was adjusted to pH 9 with a NaHCO₃ solution, and extracted three times with ethyl acetate (20.0 mL). The organic phases were combined, dried over anhydrous sodium sulfate, and concentrated under reduced pressure to give a crude product. The crude product was subjected to column chromatography (dichloromethane:methanol = 100:0 to 95:5) and concentrated to dryness by rotary evaporation to give compound 35 (496 mg, 2.16 mmol, yield: 67.4%) as a brown oil.

¹H NMR (400 MHz, CDCl₃) δ 7.16 (d, J = 5.2 Hz, 1H), 6.94 (d, J = 4.8 Hz, 1H), 4.93 - 4.90 (m, 1H), 3.73 (d, J = 9.6 Hz, 1H), 3.51 (d, J = 10.8 Hz, 1H), 3.02 - 2.92 (m, 2H), 2.55 (s, 3H), 2.42 (br.s, 1H), 1.27 (s, 3H), 1.19 (s, 3H). MS (ESI) m/z = 212.1 [M+H]⁺.

### Example 13

### Synthetic route:

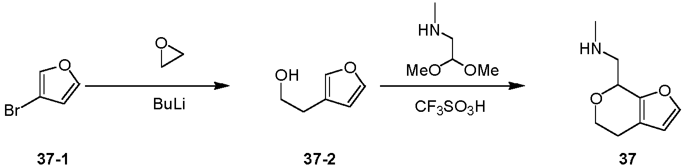

Compound 37-1 (3.50 g, 23.8 mmol, 2.13 mL, 1.00 eq) was dissolved in anhydrous tetrahydrofuran (30.0 mL). n-Butyllithium (2.50 M, 9.53 mL, 1.00 eq) was added dropwise to the reaction system at -78 °C, and the reaction system was reacted for 1 h with the temperature controlled at -60 °C. Ethylene oxide (3.00 M, 9.53 mL, 1.20 eq) was added dropwise to the reaction system. After the dropwise addition was completed, BF₃.Et₂O (4.06 g, 28.6 mmol, 3.53 mL, 1.20 eq) was added dropwise to the reaction system at -78 °C. After the dropwise addition was completed, the reaction system was gradually warmed and reacted for 3 h. TLC showed that starting material 37-1 was reacted completely and a new spot appeared. The mixture was slowly added dropwise to water (500 mL). The mixture was extracted twice with ethyl acetate (20.0 mL × 2), and water was removed with anhydrous sodium sulfate. The organic phase was concentrated to dryness by rotary evaporation to give a crude product, and the crude product was subjected to column chromatography (petroleum ether:ethyl acetate = 100: 1 to 10:1) and concentrated to dryness by rotary evaporation to give compound 37-2 (1.70 g, 10.5 mmol, yield: 43.9%) as a yellow oil.

Compound 37-2 (797 mg, 6.69 mmol, 860 µL, 1.50 eq) was dissolved in 2-MeTHF (10.0 mL). (Methylamino)acetaldehyde dimethyl acetal (500 mg, 4.46 mmol, 1.00 eq) was added. CF₃SO₃H (1.07 g, 7.13 mmol, 630 µL, 1.60 eq) was added dropwise to the reaction system at 0 °C. After the dropwise addition was completed, the reaction system was gradually warmed to 50 °C and reacted for 3 h. LCMS showed that starting material 37-2 was reacted completely and the target product was detected. The mixture was adjusted to pH = 7-8 by adding a sodium carbonate solution (20.0 mL). Dichloromethane (20.0 mL × 2) was added to the mixture, and the organic phase was dried over anhydrous sodium sulfate to give a crude product. The crude product was separated and purified by preparative HPLC to give compound 37 (23.0 mg, 129 µmol, yield: 1.93%) as a yellow oil.

¹H NMR (400MHz, CDCl₃)δ 7.30 (s, 1H), 6.27 (d, J = 1.6 Hz, 1H), 4.80 - 4.79 (m, 1H), 4.14 - 4.11 (m, 1H), 3.73 - 3.71 (m, 1H) 3.09 - 3.05 (m, 1H), 2.92 - 2.87 (m, 1H), 2.76 - 2.73 (m, 1H), 2.50 (s, 3H), 2.46 - 2.42 (m, 1H). MS (ESI) m/z =168.0 [M+H]⁺.

### Example 14

### Synthetic route:

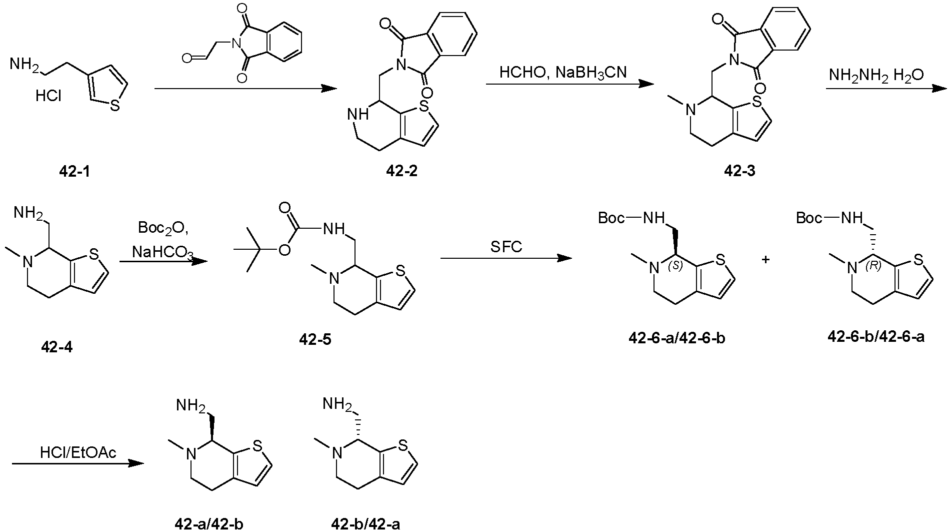

3-Aminoethylthiophene (1.10 g, 6.72 mmol, 1 eq, HCl), 2-(1,3-dioxoisoindolin-2-yl)acetaldehyde (1.27 g, 6.72 mmol, 1.00 eq) and trifluoroacetic acid (66.0 mg, 579 µmol, 42.9 µL, 2 eq) were dissolved in 1,2-dichloroethane (10 mL) under nitrogen atmosphere and purged with nitrogen three times, after which the mixture was warmed to 45 °C and stirred for 12 h. After the reaction was completed as monitored by LCMS, the reaction was quenched with a 2 M aqueous sodium bicarbonate solution (300 mL) and extracted with a mixed solvent of dichloromethane:methanol = 10:1 (200 mL × 3). The resulting organic phase was washed with brine (100 mL × 1), dried over anhydrous sodium sulfate, and concentrated by distillation under reduced pressure to give a crude product. The crude product was directly used in the next reaction without purification. Compound 42-2 (1.66 g, 5.56 mmol, yield: 82.8%) was obtained as a yellow solid.

Compound 42-2 (1.36 g, 4.56 mmol, 1.00 eq) was dissolved in a mixed solvent of methanol and 1,2-dichloroethane. Sodium cyanoborohydride (573 mg, 9.12 mmol, 2.00 eq), an aqueous formaldehyde solution (1.66 g, 18.2 mmol, 1.52 mL, purity: 33.0%, 4.00 eq), and sodium acetate (785 mg, 9.57 mmol, 2.10 eq) were added, and the reaction was stirred at 20 °C for 12 h. After the reaction was completed as monitored by TLC and LCMS, the reaction mixture was adjusted to pH = 8 with a 2 M aqueous sodium carbonate solution, diluted with brine (10 mL), and extracted with dichloromethane (20 mL × 3). The resulting organic phase was concentrated under reduced pressure to give a crude product. The crude product was purified by column chromatography (silica gel, petroleum ether:ethyl acetate = 1/0 to 1/1). Compound 42-3 (1.23 g, 3.70 mmol, yield: 81.2%) was finally obtained as a white solid.

Compound 42-3 (1.00 g, 2.60 mmol, 1.00 eq, 2 HCl) and hydrazine hydrate (800 mg, 16.0 mmol, 777 µL, 6.16 eq) were dissolved in absolute ethanol (10 mL) and purged with nitrogen 3 times, and then the system was warmed to 40 °C and stirred for 0.5 h under nitrogen atmosphere. After the reaction was completed as monitored by LCMS, the reaction mixture was quenched with a 2 M aqueous ammonium chloride solution (50 mL) at 0 °C and diluted with water (50 mL). The resulting reaction mixture was extracted with dichloromethane (15 mL × 2), washed with brine (50 mL × 1), dried over anhydrous sodium sulfate, and concentrated under reduced pressure to give a crude product. The crude product was purified by high performance liquid chromatography (hydrochloric acid system) to give compound 42-4 (crude product, hydrochloride) as a green gum product.

Compound 42-4 (560 mg, 2.56 mmol, 1.00 eq, HCl), di-t-butyl dicarbonate (1.68 g, 7.68 mmol, 1.76 mL, 3.00 eq) and sodium bicarbonate (215 mg, 2.56 mmol, 99.6 µL, 1.00 eq) were dissolved in a mixed solvent of water (100 mL) and ethyl acetate (50 mL), then purged with nitrogen 3 times, and stirred at 20 °C for 0.5 h. After the reaction was completed as monitored by LCMS, the aqueous phase was extracted with ethyl acetate (50 mL × 3). The organic phases were combined, washed with brine (50 mL × 1), dried over anhydrous sodium sulfate, and concentrated under reduced pressure. The resulting crude product was purified and separated by SFC (column: DAICEL CHIRALPAK AY-H (250 mm × 30 mm, 10 µm); mobile phase: A: CO₂, B:(0.1% NH₃H₂O EtOH); B%: 20%-20%, 4 min) to give compound 42-6-a (retention time: 1.592 min, 240 mg, 850 µmol, yield: 33.2%) and compound 42-6-b (retention time: 1.689 min, 340 mg, 1.20 mmol, yield: 47.0%) as yellow solid compounds. Compound 42-6-a (240 mg, 850 µmol, 1.00 eq) was dissolved in hydrogen chloride in ethyl acetate (4 M, 24 mL, 113 eq) and purged with nitrogen 3 times, and the reactant was reacted at 20 °C for 1 h. After the reaction was completed as monitored by LCMS, the reaction mixture was filtered, and the resulting filter cake was purified by high performance liquid chromatography (ammonium hydroxide solution system) to give compound 42-a (44.5 mg, 239 µmol, yield: 28.2%) as a yellow oily product. The same operation was performed to give compound 42-b (86.4 mg, 469 µmol, yield: 39.0%).

Compound 42-a: ¹H NMR: (400 MHz, DMSO-*d₆*) δ 7.30 (d, J = 5.0 Hz, 1H), 6.80 (d, J = 5.0 Hz, 1H), 3.01 - 2.92 (m, 1H), 2.75 (d, J = 5.4 Hz, 2H), 2.68 - 2.58 (m, 2H), 2.56 - 2.51 (m, 1H), 2.36 (s, 3H). MS (ESI) m/z = 183.1 [M+H] ⁺.

Compound 42-b: ¹H NMR: (400 MHz, DMSO-*d₆*) δ 7.30 (d, J = 5.0 Hz, 1H), 6.80 (d, J = 5.0 Hz, 1H), 3.01 - 2.92 (m, 1H), 2.75 (d, J = 5.5 Hz, 2H), 2.67 - 2.58 (m, 2H), 2.36 (s, 3H), 1.64 - 1.30 (m, 2H). MS (ESI) m/z = 183.0 [M+H] ⁺.

### Example 15

Compound 38-a (retention time: 2.261 min), compound 38-b (retention time: 2.427 min), compound 41-a (retention time: 1.798 min) and compound 41-b (retention time: 1.903 min) were obtained using a synthesis method similar to that of compound 42 and the same resolution method. The characterization data for compound 38-a, compound 38-b, compound 41-a, and compound 41-b is listed in the table below:

| Compound No./structure | ¹H NMR | MS |
|---|---|---|
| 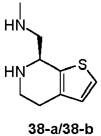 | ¹H NMR: (400 MHz, METHANOL-*d₄*) δ 7.25 (d, J = 5.0 Hz, 1H), 6.80 (d, J = 5.1 Hz, 1H), 3.97 (td, J = 1.9, 3.8 Hz, 1H), 3.21 - 3.12 (m, 1H), 2.78 - 2.59 (m, 5H), 2.33(s, 3H). | MS (ESI) m/z = 183.1 [M+H] ⁺. |
| 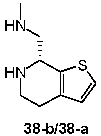 | ¹H NMR:(400 MHz, METHANOL-*d₄*) δ 7.24 (d, J = 5.0 Hz, 1H), 6.80 (d, J = 5.0 Hz, 1H), 3.97 (br d, J = 4.1 Hz, 1H), 3.21 - 3.12 (m, 1H), 2.78 - 2.58 (m, 5H), 2.33(s, 3H). | MS (ESI) m/z = 183.1 [M+H] ⁺ |
| 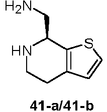 | ¹H NMR (400 MHz, METHANOL-*d₄*) δ 7.25 - 7.18 (m, 1H), 6.82 (d, J = 5.1 Hz, 1H), 4.01 (td, J = 1.9, 3.8 Hz, 1H), 3.23 (td, J = 4.9, 12.8 Hz, 1H), 3.04 - 2.83 (m, 3H), 2.76 - 2.59 (m, 2H). | MS (ESI) m/z = 169.0 [M+H] ⁺ |
| 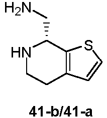 | ¹H NMR (400 MHz, METHANOL-*d₄*) δ 7.22 (d, J *=* 5.0 Hz, 1H), 6.82 (d, J = 5.0 Hz, 1H), 4.01 (br d, J = 4.1 Hz, 1H), 3.22 (td, J = 4.9, 12.8 Hz, 1H), 3.04 - 2.83 (m, 3H), 2.75 - 2.58 (m, 2H). | MS (ESI) m/z = 169.0 [M+H] ⁺ |

### Example 16

### Synthetic route:

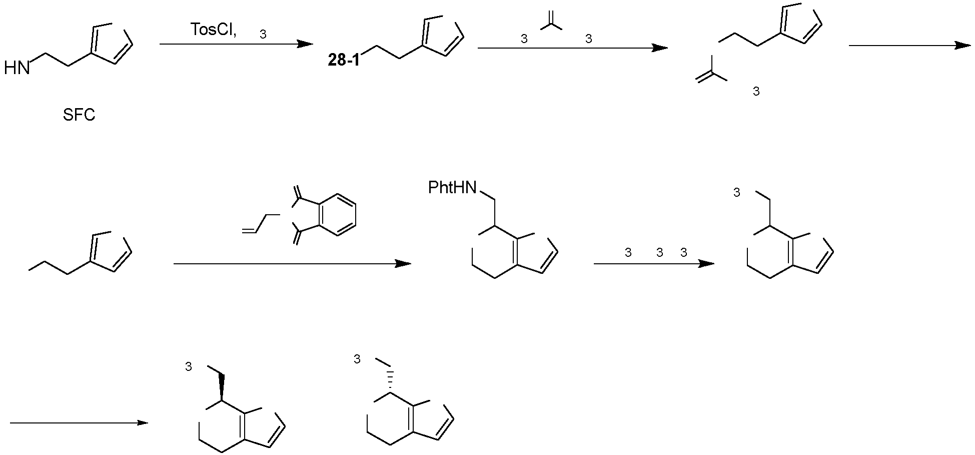

2-(Thien-3-yl)ethanol (7.00 g, 54.6 mmol, 6.03 mL, 1 eq) was dissolved in dichloromethane (100 mL), and 4-methylbenzenesulfonyl chloride (12.5 g, 65.5 mmol, 1.2 eq) and triethylamine (6.63 g, 65.5 mmol, 9.12 mL, 1.2 eq) were added. The resulting mixture was then stirred at 15 °C for 12 h. The reaction was completed as monitored by TLC. The reaction mixture was quenched by adding 150 mL of water at 0 °C, and extracted with dichloromethane. The organic phase was washed with saturated brine, dried over anhydrous sodium sulfate, filtered and concentrated under reduced pressure to give a crude product. The crude product was purified by column chromatography (petroleum ether/ethyl acetate = 7/1 to 6/1). Compound 43-2 (11.7 g, 41.5 mmol, yield: 76.0%) was obtained as a pale yellow oil.

Compound 43-2 (11.7 g, 41.4 mmol, 1 eq), thiourea (3.15 g, 41.4 mmol, 1 eq), and water (747 mg, 41.4 mmol, 747 µL, 1 eq) were dissolved in ethanol (50.0 mL), purged with nitrogen three times, and then the refluxed at 100 °C for 5 h. The reaction was completed as monitored by LCMS. The reaction mixture was directly used in the next step.

Sodium hydroxide (3.20 g, 80.0 mmol, 1.86 eq) was dissolved in pure water H₂O (15 mL), added to the reaction mixture in the previous step, purged with nitrogen three times, and reacted at 100 °C for 1.5 h. The reaction was completed as monitored by LCMS. The reaction mixture was concentrated to about 5 mL, diluted with water (2 × 5 mL), made neutral with hydrochloric acid (37%), and extracted with dichloromethane. The organic phase was dried over anhydrous sodium sulfate, filtered and concentrated under reduced pressure to give compound 43-4 (5.70 g, crude product) as a yellow oil.

Compound 43-4 (4.70 g, 32.6 mmol, 1 eq), 2-(1,3-dioxoisoindol-2-yl)acetaldehyde (6.16 g, 32.6 mmol, 1 eq), and trifluoromethanesulfonic acid (7.43 g, 65.2 mmol, 4.82 mL, 2 eq) were dissolved in 1,2-dichloroethane (100 mL), and purged with nitrogen three times. The mixture was reacted at 50 °C for 12 h. The reaction was completed as monitored by LCMS. The reaction mixture was filtered and concentrated under reduced pressure to give compound 43-5 (10.0 g, crude product).

Compound 43-5 (2.00 g, 6.34 mmol, 1 eq), hydrazine hydrochloride hydrate (869 mg, 12.7 mmol, 2 eq), and potassium carbonate (1.75 g, 12.7 mmol, 2 eq) were dissolved in methanol (30 mL), and purged with nitrogen three times. The mixture was then reacted at 65 °C for 4 h. The reaction was completed as monitored by LCMS. The reaction mixture was filtered and concentrated to give a crude product, and the filtrate was quenched with a dilute aqueous sodium hypochlorite solution. The mixture was purified by high performance liquid chromatography to give compound 43 as a brown solid. Chiral resolution (DAICEL CHIRALPAK AY-H (250 mm × 30 mm, 10 µm); mobile phase: A: CO₂, B:(0.1% NH₃H₂O EtOH); B%: 30%-30%, 4 min) was performed to give compound 43-a (retention time: 1.753 min, 133 mg, 0.69 mmol, yield: 10.9%) as a yellow oil, and compound 43-b (retention time: 1.908 min, 143 mg, 0.74 mmol, yield: 11.7%) as a yellow oil.

Compound 43-a: ¹H NMR (400 MHz, CDCl₃) δ 7.13 (d, J = 5.1 Hz, 1H), 6.77 (d, J = 5.1 Hz, 1H), 3.94 (dd, J = 4.4, 8.3 Hz, 1H), 3.11 (br d, J = 4.5 Hz, 1H), 3.04 - 2.94 (m, 4H), 2.92 - 2.77 (m, 1H), 1.86-1.82(m, 2H). MS (ESI) m/z = 185.9 [M+H] ⁺.

Compound 43-b: ¹H NMR (400 MHz, CDCl₃) δ 7.11 (d, J = 5.1 Hz, 1H), 6.76 (d, J = 5.1 Hz, 1H), 3.94 (dd, J = 4.4, 8.3 Hz, 1H), 3.11 (br d, J = 4.5 Hz, 1H), 3.02 - 2.93 (m, 4H), 2.92 - 2.77 (m, 1H), 1.95-1.92(m, 2H). MS (ESI) m/z = 185.9 [M+H] ⁺.

### Example 17

Compound 40-a (retention time: 2.918 min) and compound 40-b (retention time: 3.491 min) were obtained using a synthesis method similar to that of compound 43 and the same resolution method. The characterization data for compound 40-a and compound 40-b is listed in the table below:

| Compound No./structure | ¹H NMR | MS |
|---|---|---|
| 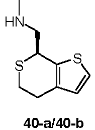 | ¹H NMR: (400 MHz, CDCl₃)δ 7.31 (d, J = 5.1 Hz, 1H), 6.81 (d, J = 5.1 Hz, 1H), 4.08 (dd, J = 4.4, 8.3 Hz, 1H), 2.89 - 2.76 (m, 6H), 2.33 (s, 3H), 1.84(s, 1H). | MS (ESI) m/z = 200.0 [M+H] ⁺ |
| 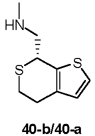 | ¹H NMR: (400 MHz, CDCl₃)δ 7.31 (d, J = 5.1 Hz, 1H), 6.81 (d, J = 5.1 Hz, 1H), 4.09 (dd, J = 4.4, 8.3 Hz, 1H), 2.89 - 2.76 (m, 6H), 2.33 (s, 3H), 1.84(s, 1H). | MS (ESI) m/z = 200.0 [M+H] ⁺. |

### Example 18

### Synthetic route:

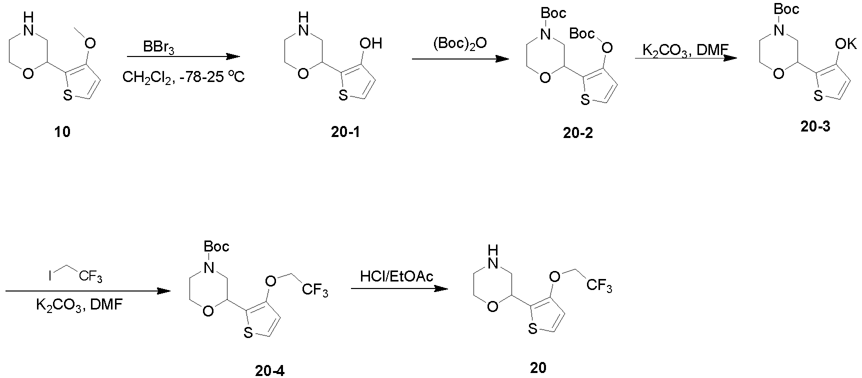

Compound 10 (100 mg, 502 µmol, 1.00 eq) was dissolved in DCM (10.0 mL), and the mixture was cooled to - 75 °C. Compound BBr₃ (377 mg, 1.51 mmol, 145 µL, 3.00 eq) was added dropwise. After the dropwise addition was completed, the mixture was naturally warmed to 20 °C and stirred for 2 h. The reaction of compound 10 was completed as shown by LCMS detection. The reaction mixture was cooled to -20 °C, quenched by slowly dropwise adding MeOH (10.0 mL), and stirred at low temperature for 10 min. The reaction mixture was directly used in the next step without concentration and purification.

Et₃N was added dropwise to a solution of compound 20-1 in methanol (1.00 eq) at -20 °C to adjust the pH to 8, and (Boc)₂O (6.00 eq) was added. The reaction mixture was gradually warmed to room temperature and reacted for 12 h. The reaction of compound 20-1 was completed as detected by TLC. The reaction mixture was cooled to 0-5 °C, and water (50.0 mL) was added. The mixture was washed with EtOAc (30.0 mL). The filtrate was washed with brine (30.0 mL). The mixture was dried over anhydrous sodium sulfate and concentrated under reduced pressure. The crude product was subjected to silica gel column chromatography (eluent petroleum ether:EtOAc = 5:1) to give compound 20-2 (400 mg, 1.04 mmol, yield: 55.9%) as a yellow oil.

Compound 20-2 (100 mg, 259 µmol, 1.00 eq) was suspended in MeOH (2.00 mL), K₂CO₃ (179 mg, 1.30 mmol, 5.00 eq) was added, and the reaction mixture was heated to 25 °C and stirred for 6 h. The reaction of compound 20-2 was completed as detected by LCMS. The reaction mixture was filtered, and the filtrate was collected and directly concentrated to dryness by rotary evaporation, which was used in the next step. The product 20-3 (93 mg) was obtained, and the crude product was a yellow oil.

Compound 20-3 (100 mg, 259 µmol, 1.00 eq) was dissolved in anhydrous DMF (2.00 mL). DIPEA (223 mg, 1.73 mmol, 300 µL, 6.00 eq) was added, and trifluoroiodoethane (200 mg, 862 µmol, 3.00 eq) was added. The reaction mixture was stirred at room temperature for 5 h. The starting material was reacted completely and a product was generated as detected by LCMS. The reactant was directly concentrated to dryness by rotary evaporation and purified by column chromatography (PE:EtOAC = 5:1) to give compound 20-4 (70 mg) as a yellow oil.

Compound 20-4 (70.0 mg, 190 µmol, 1.00 eq) was added to hydrogen chloride in ethyl acetate (4 M) (5.0 mL). The reaction mixture was stirred at 25 °C for 5 h. The starting material was reacted completely and a product was generated as detected by LCMS. The pH of the product was adjusted to about 8 with NaHCO₃, and the mixture was extracted three times with ethyl acetate (20 mL). The organic phase was concentrated to dryness by rotary evaporation to give compound 20 (27.0 mg, 95.9 µmol, yield: 50.4%) as a yellow oil. ¹H NMR (400 MHz, CDCl₃) δ 7.20 - 7.19 (d,J=4.0 Hz 1H), 6.77-6.75(d, J = 8Hz, 1H), 4.93-4.90(dd, J = 8.0 Hz,4.0 Hz 1H), 4.38-4.32.(m, 2H), 4.00-.399 (d,J=4.0 Hz 1H), 3.99-3.82 (m, 1H), 3.20-3.17(m, 1H), 3.02-2.89(m, 3H). MS (ESI) m/z = 268.1 [M+H] ⁺.

### Example 19

Compound 15, compound 19 and compound 60 were obtained using a synthesis method similar to that of compound 20. The characterization data for compound 15, compound 19, and compound 60 is listed in the table below:

| Compound No./structure | ¹H NMR | MS |
|---|---|---|
| 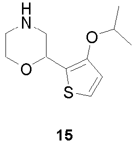 | ¹H NMR (400 MHz, CDCl₃) δ 7.13 (d, J=5.20 Hz, 1 H) 6.76 (d, J=5.20 Hz, 1 H) 4.85 (dd, J=10.0, 2.60 Hz, 1 H) 4.31 - 4.40 (m, 1 H) 3.99 (dd, J=11.6, 2.20 Hz, 1 H) 3.69 - 3.79 (m, 1 H) 3.11 (dd, J=12.4, 2.20 Hz, 1 H) 2.94 - 3.05 (m, 1 H) 2.82 - 2.92 (m, 2 H) 1.30 (dd, J=6.0, 3.20 Hz, 6 H). | MS (ESI) m/z = 228.1 [M+H] ⁺ |
| 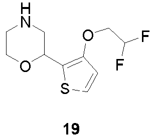 | ¹H NMR (400 MHz, CDCl₃) δ 2.84 - 2.93 (m, 2 H), 2.94 - 3.03 (m, 1 H), 3.12 (d, J=12.4 Hz, 1 H), 3.77 (t, J=11.6 Hz, 1 H), 3.98 (br d, J=11.2 Hz, 1 H), 4.18 (td, J=13.2, 3.60 Hz, 2 H), 4.87 (br d, J=10.01 Hz, 1 H), 5.86 - 6.19 (m, 1 H), 6.76 (d, J=5.20 Hz, 1 H), 7.17 (d, J=5.20 Hz, 1 H). | MS (ESI) m/z = 250.1 [M+H] ⁺ |
| 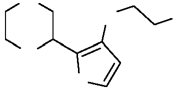 | ¹H NMR (400 MHz MeOD-d₆) δ 7.32 - 7.31 (d, J=4.0 Hz, 1 H), 6.93- 6.91 (d, J=8.0 Hz, 1 H), 5.07-5.05 (m, 1H), 4.73-4.72 (m, 1H), 4.59 - 4.62 (m, 1 H) 4.28 - 4.33 (m, 1 H) 4.22 - 4.26 (m, 1 H) 4.03 (m, 1 H) 3.90 (m,1 H) 3.64 (s, 1 H) 3.34 (s, 2 H) 2.99 - 3.18 (m, 3 H) 2.42 - 2.43 (m, 1 H) 2.38 - 2.39 (m, 1 H). | MS (ESI) m/z = 232.1 [M+H] ⁺ |

### Example 20

### Synthetic route:

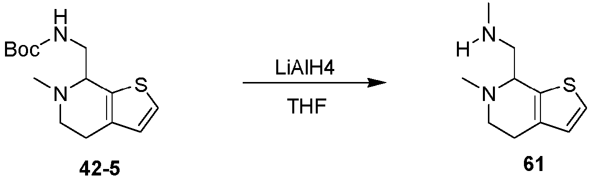

Anhydrous THF (10.0 mL) was added to compound 42-5 (300 mg, 1.06 mmol, 1.00 eq), and LiAlH₄ was added in portions at 0 °C under N₂ atmosphere. The reaction was warmed to 75 °C and stirred for 3 h. The reaction of compound 42-5 was completed as shown by LCMS detection. The reaction mixture was slowly poured into water. The reaction mixture was extracted three times with EtOAc (30 mL). The extracted organic phases were combined, washed once with brine (30 mL), dried once over anhydrous sodium sulfate, and concentrated under reduced pressure to give a crude product. The crude product was purified by HPLC chromatography column (column: Xtimate C18 150 × 40 mm × 10 um; mobile phase: [water(NH₄HCO₃)-ACN]; B%: 2%-42%, 25 min) to give compound 61 (139 mg, 637 µmol, yield: 41.7%) as a yellow oil. ¹H NMR (400 MH, CDCl₃) δ 7.14 (d, J=5.20 Hz, 1 H), 6.78 (d, J=5.20 Hz, 1 H), 3.75 (t, J=12.0 Hz, 8.0 Hz 1 H), 3.03 - 3.11 (m, 1 H), 2.87 - 2.90 (m, 2 H), 2.69 - 2.80 (m, 2 H), 2.55 - 2.62 (m, 1 H), 2.49 (d, J=2.40Hz, 6 H). MS (ESI) m/z = 197.1 [M+H] ⁺.

### Example 21

### Synthetic route:

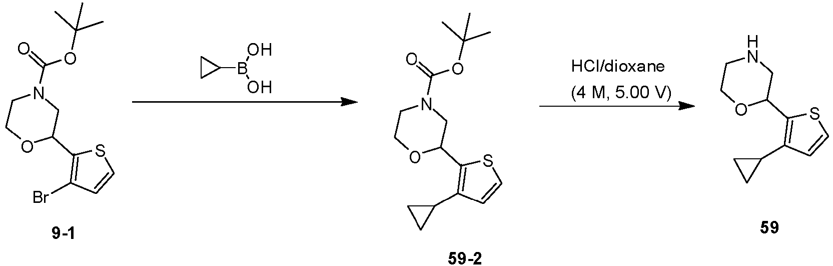

Compound 9-1 (100 mg, 287 µmol, 1.00 eq) was dissolved in dioxane (1.00 mL) and water (0.10 mL). Cyclopropylboronic acid (49.3 mg, 574 µmol, 2.00 eq), potassium carbonate (79.4 mg, 574 µmol, 2.00 eq) and Pd(PPh₃)₄ (33.2 mg, 28.7 µmol, 0.10 eq) were sequentially added to the system, and the system was stirred at 100 °C for 3 h. The MS of the product was detected by LCMS. Water (10.0 mL) was added dropwise to the reaction mixture at 25 °C while the reaction mixture was extracted 3 times with 3.0 mL of EtOAc. The organic phase was washed with saturated brine (7.00 mL), and finally dried over anhydrous sodium sulfate. The organic phase was concentrated and subjected to column chromatography (petroleum ether/ethyl acetate = 93/7) to give intermediate 59-2 (110 mg) as a white oily product.

Intermediate 59-2 (110 mg, 356 µmol, 1.00 eq) was dissolved in HCl/dioxane (0.50 mL), and the reaction mixture was stirred at 25 °C for 3 h. The MS of the product was detected by LCMS. The pH of the reaction mixture was adjusted to 7-8 with a saturated NaHCO₃ solution. The reaction mixture was extracted 3 times with 1.0 mL of DCM. The organic phase was washed with saturated brine (3.0 mL), and finally dried over anhydrous sodium sulfate. The organic phase was concentrated and subjected to prep-HPLC (Welch Xtimate C18 150 × 30 mm × 5 µm; mobile phase: [water(NH₄HCO₃) - ACN]; B%: 10% - 50%, 36 min) to give compound 59 (33.0 mg, 155 µmol, yield: 43.6%) as a white solid. ¹H NMR (400 MH, CDCl₃) δ 7.12 (d, J = 5.14 Hz, 1 H), 6.57 (d, J = 5.14 Hz, 1 H), 5.00 (dd, J = 10.20, 2.56 Hz, 1 H), 4.03 (dd, J = 11.44, 2.70 Hz, 1 H), 3.80 (m, 1 H), 3.16 (dd, J = 12.20, 1.80 Hz, 1 H), 2.88 - 3.06 (m, 3 H), 1.81 - 1.92 (m, 1 H) 0.89 - 0.97 (m, 2 H), 0.68 - 0.74 (m, 1 H), 0.56 - 0.65 (m, 1 H). MS (ESI) m/z = 210.1 [M+H] ⁺.

### Example 22

### Synthetic route

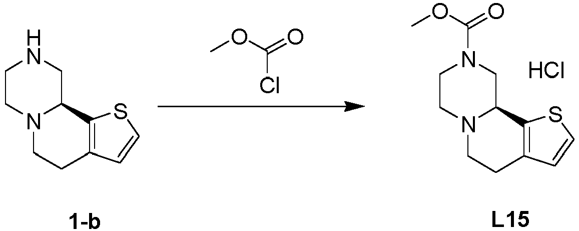

Compound 1-b (500 mg, 2.57 mmol, 1.00 eq) was dissolved in anhydrous dichloromethane (5.00 mL). Et₃N (390 mg, 3.86 mmol, 537 µL, 1.50 eq) was then added, and methyl chloroformate (1.22 g, 12.8 mmol, 994 µL, 5.00 eq) was then added dropwise to the reaction mixture. After the dropwise addition was completed, the mixture was stirred at 20 °C for 5 h. The reaction of compound 1-b was completed as shown by LCMS detection. To the reaction mixture was added 10.0 mL of water, and the mixture was extracted 2 times with DCM (20.0 mL). The organic phase was collected, dried over Na₂SO₄, and concentrated to dryness by rotary evaporation to give a crude product. The crude product was adjusted to pH = 2 with HCl/EtOAC (1 M), and a solid was precipitated in the reaction mixture. The reaction mixture was filtered to collect the solid, and the solid was concentrated to dryness by rotary evaporation to give the hydrochloride of compound L15 (150 mg, 582 µmol, yield: 22.6%) as a yellow solid. ¹H NMR (400 MHz, DMSO) *δ* 7.61-7.60 (d, J=4.00 Hz, 1 H), 7.00-6.99 (d, J=4.00 Hz, 1 H), 4.7 (m, 1 H), 4.36-4.16(m, 2 H), 3.68(s, 5 H), 3.50-3.47(m, 2 H), 3.21-3.20(m, 3 H), 2.96-2.92(m, 1 H). MS (ESI) m/z = 253.1 [M+H] ⁺.

### Example 23

### Synthetic route:

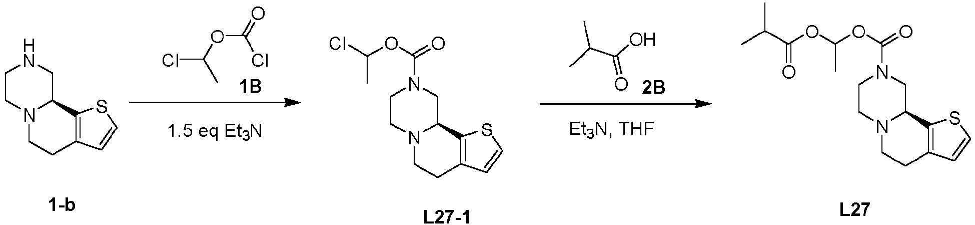

Compound 1-b (500 mg, 2.57 mmol, 1.00 eq) was dissolved in anhydrous dichloromethane (5.00 mL). Et₃N (390 mg, 3.86 mmol, 537 µL, 1.50 eq) was then added, and compound 1B (1.84 g, 12.8 mmol, 5.00 eq) was then added dropwise to the reaction mixture. After the dropwise addition was completed, the mixture was stirred at 20 °C for 5 h. The reaction of compound 1-b was completed as shown by LCMS detection. To the reaction mixture was added 10.0 mL of water, and the mixture was extracted 2 times with DCM (20.0 mL). The organic phase was collected, dried over Na₂SO₄, and concentrated to dryness by rotary evaporation to give crude compound L27-1 (1.00 g) as a yellow solid.

Compound L27-1 (370 mg, 1.23 mmol, 1.00 eq) was dissolved in THF (3.00 mL), and DIEA (477 mg, 3.69 mmol, 0.64 mL, 3.00 eq) was added. Compound 2B (650 mg, 7.38 mmol, 0.684 mL, 6.00 eq) was then added dropwise to the reaction mixture, and the reaction mixture was heated to 55 °C and stirred for 12 h. The reaction of compound L27-1 was completed as detected by LCMS. To the reaction mixture was added 5.00 mL of water, and the mixture was extracted 2 times with DCM (10.0 mL). The organic phase was collected, dried over Na₂SO₄, and concentrated to dryness by rotary evaporation to give a crude product. The crude product was purified by column chromatography to give compound L27 (240 mg, 0.78 mmol, yield: 63.3%). ¹H NMR (400 MHz, CDCl₃) *δ* 7.15 (d, J=5.00 Hz, 1 H), 6.77 - 6.92 (m, 2 H), 3.98 - 4.47 (m, 2 H), 2.83 - 3.39 (m, 6 H), 2.44 - 2.71 (m, 4 H), 1.54 (s, 3 H), 1.19 (dd, J=6.80, 2.00 Hz, 6 H). MS (ESI) m/z = 353.2 [M+H]⁺.

L27 (100 mg, 0.29 mmol) was dissolved in ethyl acetate (5 mL), and a 4 M hydrochloric acid/dioxane solution (0.5 mL) was added dropwise at 0 °C. The mixture was stirred at room temperature for 2 h, and a solid was precipitated. The mixture was filtered to give the hydrochloride of L27 (120 mg).

### Test Example 1. In Vitro Evaluation of Intrasynaptic and Extrasynaptic Functional Activities in Cells

Cell: cAMP Hunter^{™} CHO-K1 TAAR1 Gs cell line
Assay reagent: HitHunter cAMP Assay Detection Kit
Experimental instrument: PerkinElmer Envision^{™} chemiluminescence signal detector
Experimental method: cell treatment: The cAMP Hunter cell strain was cultured at 37 °C with 5% CO₂ to 70-80% confluence. The cells were harvested, and the cell density was adjusted to 1.5 × 10⁴ cells/mL. The cells were seeded in a white-wall 384-well microwell plate at a volume of 20 µL, and incubated at 37 °C with 5% CO₂ overnight. The cell medium was discarded, and 15 µL of 2:1 HBSS/10 mM Hepes:cAMP XS + Ab reagent was added to each well. The mother solution of the sample was diluted with an assay buffer in a concentration gradient to a 4X sample, and 5 µL of the 4X sample was added to each well. The plate was incubated at 37 °C or room temperature for 30 or 60 min. 20 µL of cAMP XS + ED/CL mixed lysis buffer was added to each well, and the plate was incubated for 1 h. 20 µL of cAMP XS + EA reagent was added to each well, and the plate was incubated at room temperature for 3 h. The chemiluminescence signal was read using a PerkinElmer Envision^{™} detector.

Data analysis: EC₅₀ was calculated using CBIS data analysis software (ChemInnovation, CA). The activity percentage was calculated using the following formula: %activity = 100% × (test sample average RLU - solvent control average RLU)/(maximum control average RLU - solvent control average RLU). The results are shown in Table 1.

**Table 1. Results of in vitro activity test for compound TAAR1**

| Compound | EC₅₀ (µM) |
|---|---|
| 1-a | 9.121 |
| 1-b | 0.062 |
| 2-a | 4.402 |
| 2-b | >10 |
| 7-a | 4.137 |
| 7-b | 0.344 |
| 8 | 0.295 |
| 9 | 0.734 |
| 10-a | 0.028 |
| 10-b | 0.386 |
| 11-a | >10 |
| 11-b | 2.162 |
| 12-a | >10 |
| 12-b | 1.592 |
| 13-a | >10 |
| 13-b | >10 |
| 15 | >10 |
| 19 | >10 |
| 20 | 4.715 |
| 28-a | >10 |
| 28-b | 1.842 |
| 30 | >10 |
| 32-a | >10 |
| 32-b | >10 |
| 32-c | >10 |
| 32-d | >10 |
| 34 | 1.253 |
| 35 | >10 |
| 37 | 1.228 |
| 38-a | >10 |
| 38-b | 7.961 |
| 39-a | >10 |
| 39-b | >10 |
| 40-a | >10 |
| 40-b | 0.522 |
| 41-a | 9.102 |
| 41-b | 0.424 |
| 42-a | >10 |
| 42-b | 0.244 |
| 43-a | >10 |
| 43-b | 0.031 |
| 59 | 2.913 |
| 60 | 9.803 |
| 61 | >10 |

| | |
|---|---|
| Conclusion: The compounds in the present disclosure show good TAAR1 agonistic activity. | |

### Test Example 2. Pharmacokinetic Study in Rats

Rat: SD rats weighing 200-250 g
Reagent: acetonitrile, MERCK; methanol, MERCK; formic acid, Sigma; tolbutamide, Sigma.

Instrument: water purification equipment, Millipore; electronic balance, METTLER TOLEDO; high-speed benchtop centrifuge, Thermo; water bath thermostatic oscillator, Shanghai Yiheng Technical Co., Ltd.; vortex oscillator, Thermo; liquid chromatography-mass spectrometry, AB SCIEX.

Method: The SD rats were dosed intravenously (IV) and intragastrically (IG), 3 SD rats in each group. Compounds 1-b, 2-a and L27 were separately administered, and the vehicles were DMSO and Tween 80. Administration dose: 2 mg/kg (compound 1-b) or 1 mg/kg (compound 2-a) for intravenous administration, and 5 mg/kg (compound 1-b and compound 2-a) or 9 mg/kg (compound L27) for intragastric administration. Whole blood samples were separately collected at 0, 5 min (intravenous), 15 min, 30 min, 1 h, 2 h, 4 h, 6 h, 8 h, 12 h, and 24 h. The whole blood was placed in a heparinized EP tube and centrifuged at 13,500 rpm for 10 min to separate the plasma. After pretreatment, the plasma was analyzed by LC-MS/MS to determine the concentration of the test compound in the plasma. The results are shown in Table 2 and Table 3:

**Table 2. Pharmacokinetic parameters in SD rats after intravenous and intragastric administration of 2-a and 1-b**

| Subject | 1-b IV 2mg/kg | 2-a IV 1mg/kg | | 1-b IG 5mg/kg | 2-a IG 5mg/kg | |
|---|---|---|---|---|---|---|
| | | 2-a | 1-b | | 2-a | 1-b |
| Tₘₐₓ(h) | / | 0.083±0 | 0.667±0.289 | 0.417±0.144 | 0.139±0.096 | 0.500±0.433 |
| Cₘₐₓ or C₀ (nM) | 7541±2278 | 3113±81 | 118±3 | 3730±846 | 3747±2011 | 465±143 |
| AUCₗₐₛₜ(h* nM) | 5971±642 | 1906±46 | 339±11 | 9945±1213 | 4582±1321 | 1743±480 |
| T_{1/2} (h) | 1.76±0.344 | 0.528±0.054 | 1.24±0.16 | 1.85±0.217 | 3.46±4.30 | 1.94±0.53 |
| Vss_obs(L/kg) | 2.91±0.68 | 1.46±0.06 | / | / | / | |
| Cl_obs(L/h/kg) | 1.73±0.195 | 2.51±0.06 | / | / | / | |
| MRTₗₐₛₜ(h) | 1.60±0.203 | 0.566±0.005 | 1.83±0.04 | 2.33±0.465 | 1.89±1.15 | 2.71±0.46 |
| F(%) | / | / | / | 66.6±8.12 | 48.1±10.3 | |

**Table 3. Pharmacokinetic parameters in SD rats after intragastric administration of L27**

| Test compound | HL_Lambda_z h | Tₘₐₓ h | Cₘₐₓ nM | AUCₗₐₛₜ h*nM | MRTₗₐₛₜ h |
|---|---|---|---|---|---|
| L27 | L27 was not detected | | | | |
| 1-b | 1.61±0.35 | 0.667±0.289 | 1977±200 | 5993±1341 | 2.40±0.42 |

| | | | | | |
|---|---|---|---|---|---|
| Conclusion: The results show that compounds 1-b and 2-a have a relatively high exposure and bioavailability in rats after intravenous and intragastric administration. After being intragastrically administered in rats, compound L27 can be metabolized to active compound 1-b. L27 was not detected. | | | | | |

### Test Example 3. Pharmacokinetic Study in Dogs

Dog: Beagle dogs weighing 11-13 kg
Reagent: acetonitrile, MERCK; methanol, MERCK; formic acid, Sigma; tolbutamide, Sigma.

Instrument: water purification equipment, Millipore; electronic balance, METTLER TOLEDO; high-speed benchtop centrifuge, Thermo; water bath thermostatic oscillator, Shanghai Yiheng Technical Co., Ltd.; vortex oscillator, Thermo; liquid chromatography-mass spectrometry, AB SCIEX.

Method: L15 hydrochloride was administered intragastrically at a dose of 2 mg/kg (calculated based on free base), 2 Beagle dogs in each group. The vehicles were DMSO and Tween 80. Whole blood samples were separately collected at 0, 5 min, 15 min, 30 min, 1 h, 2 h, 3 h, 4 h, 6 h, 8 h, 12 h, and 24 h. The whole blood was placed in a heparinized EP tube and centrifuged at 13,500 rpm for 10 min to separate the plasma. After pretreatment, the plasma was analyzed by LC-MS/MS to determine the concentration of the test compound in the plasma. The results are shown in Table 4:

**Table 4. Pharmacokinetic parameters in Beagle dogs after intragastric administration of L15 hydrochloride**

| Test compound | HL_Lambda_z h | Tₘₐₓ h | Cₘₐₓ nM | AUCₗₐₛₜ h*nM | MRTₗₐₛₜ h |
|---|---|---|---|---|---|
| L15 | 2.09 | 0.25 | 217 | 197 | 1.44 |
| 1-b | NA | 1.50 | 22.3 | 54.7 | 1.68 |

| | | | | | |
|---|---|---|---|---|---|
| Conclusion: After being intragastrically administered in Beagle dogs, L15 hydrochloride can be partially metabolized to compound 1-b. | | | | | |

### Test Example 4. Pharmacodynamic Study in Mice

Mouse: C57 mice weighing 20-22 g
Reagent: normal saline.

Instrument: electronic balance, METTLER TOLEDO; TopScan monitoring system.

4.1. model preparation: MK-801 induces overactivity in mice and is commonly used for preclinical evaluation of antipsychotics. The modeling method in this test was: The mice were injected with MK-801 intraperitoneally in at single dose of 0.8 mg/kg.

4.2. Experimental grouping: The mice were randomly divided into model group, 1-b group, and L27 group, 10 animals in each group, as shown in Table 5.

**Table 5. Animal grouping and dosing**

| Group | Type of drug | Administration dose (mg/kg) | Drug concentration (mg/mL) | Administration route | Administration volume (mL/kg) | Animal Number |
|---|---|---|---|---|---|---|
| Model group | -- | -- | -- | -- | -- | 10 |
| 1-b group | 1-b | 10 | 1 | Intragastric | 10 | 10 |
| L27 group | L27 hydrochloride | 16 | 1.6 | Intragastric | 10 | 10 |

| | | | | | | |
|---|---|---|---|---|---|---|
| Note: The L27 group was the equimolar dose as 1-b. | | | | | | |

### 4.3. Test method:

The mice were randomly divided into groups. At the start of the test, the test samples were administered intragastrically at an single dose according to the doses shown in Table 5, and the mice were placed in an activity chamber. After 30 min, each mouse was injected intraperitoneally with 0.8 mg/kg of MK-801 for modeling, and placed in the activity chamber again. The movement distance (mm) of the mice after modeling (i.e., within 30-60 min after administration) was recorded and analyzed using a TopScan monitoring system.

### 4.4. Observation of indexes:

The movement distance (mm) of the mice after modeling (within 30-60 min after administration) was recorded and analyzed using a TopScan monitoring system. The total movement distance of the mice after modeling (30-60 min after administration) was recorded.

### 4.5. Statistical analysis:

Data analysis and processing were carried out using Graphpad 5.0 software. ANOVA test was used to compare the total movement distance between each administration group and model group within 30-60 min. All tests were bilateral tests. *p* < 0.05 indicates that the difference has statistical significance.

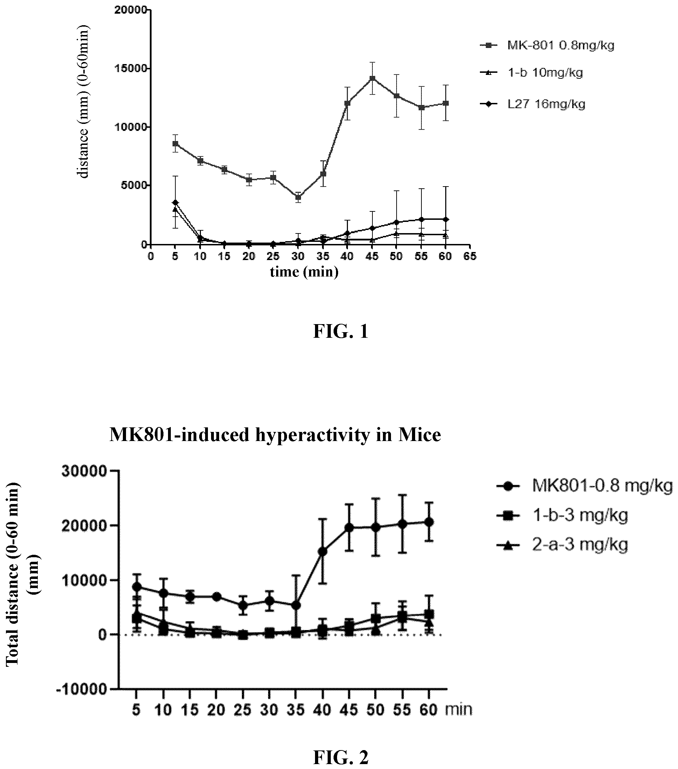

### 4.6. Test results (see FIG. 1):

The test results show that the total movement distance of the animals in the groups 1-b and L27 after modeling was 6493.12 ± 6228.87 mm and 8100.56 ± 7787.37 mm, respectively, which was significantly reduced (*p* < 0.05) compared with the total movement distance of the animals in the model group after modeling (63052.14 ± 21618.80 mm).

### 4.7. Conclusion of test:

Under the experimental conditions, 1-b (10 mg/kg) and equimolar L27 hydrochloride (16 mg/kg) have a good inhibition effect on MK801-induced overactivity.

### Test Example 5. Pharmacodynamic Study in Mice

5.1. Experimental grouping: The mice were randomly divided into model group, 1-b group, and 2-a group, 5 animals in each group, as shown in Table 6. The model preparation, test method, observation of indexes and statistical method were the same as those in Test Example 4.

**Table 6. Animal grouping and dosing**

| Group | Type of drug | Administration dose (mg/kg) | Drug concentration (mg/mL) | Administration route | Administration volume (mL/kg) | Animal Number |
|---|---|---|---|---|---|---|
| Model group | -- | -- | -- | -- | -- | 5 |
| 1-b group | 1-b | 3 | 0.75 | Intragastric | 4 | 5 |
| 2-a group | 2-a | 3 | 0.75 | Intragastric | 4 | 5 |

### 5.2. Test results (see FIG. 2):

The test results show that the total movement distance of the animals in the groups 1-b and 2-a after modeling was 13644.54 ± 10814.85 mm and 9161.61 ± 4711.10 mm, respectively, which was significantly reduced (*p* < 0.05) compared with the total movement distance of the animals in the model group after modeling (101535.71 ± 26076.11 mm).

### 5.3. Conclusion of test:

Under the experimental conditions, 1-b (3 mg/kg) and 2-a (3 mg/kg) have a good inhibition effect on MK801-induced overactivity.

## Claims

1. A compound of formula (IA), a pharmaceutically acceptable salt thereof or a stereoisomer thereof, 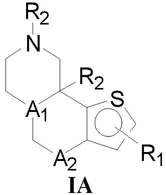 wherein,
A₁ is selected from the group consisting of CR₂ or N;
A₂ is selected from the group consisting of CR₂ or O;
R₁ is selected from the group consisting of H, halogen, C₁₋₆ alkyl, or C₁₋₆ alkoxy;
each R₂ is independently selected from the group consisting of H or C₁₋₆ alkyl.

2. A compound of formula (IA-1) or (IA-2), or a pharmaceutically acceptable salt thereof, wherein 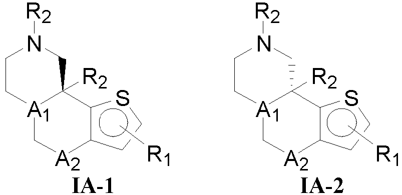
A₁ is selected from the group consisting of CR₂ or N;
A₂ is selected from the group consisting of CR₂ or O;
R₁ is selected from the group consisting of H, F, methyl, or methoxy;
each R₂ is independently selected from the group consisting of H or methyl.

3. A compound of formula (IA-3) or (IA-4), or a pharmaceutically acceptable salt thereof, wherein 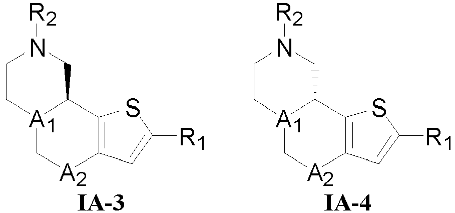
A₁ is selected from the group consisting of CH or N;
A₂ is selected from the group consisting of CH₂ or O;
R₁ is selected from the group consisting of H, halogen, C₁₋₆ alkyl, or C₁₋₆ alkoxy;
R₂ is selected from the group consisting of H, C₁₋₆ alkyl, or the following formula: 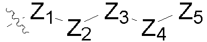 wherein,
Z₁ is selected from the group consisting of a bond or C₁₋₃ alkyl;
Z₂ is selected from the group consisting of a bond, -O-, -NH-, -S-, -(C=O)-, -O(C=O)-, or -(C=O)O-;
Z₃ is selected from the group consisting of a bond, C₁₋₆ alkyl, phenyl, -CH(NHAc)-CH₂CH₂-, -CH(CH₃)-NHC(=O)-CH(CH₃)-, -CH(CH₃)-OC(=O)-, or -CH₂CH₂CH₂CH₂CH(NH₂)-;
Z₄ is selected from the group consisting of a bond, -O-, -NH-, -S-, -(C=O)-, -O(C=O)-, or -(C=O)O-;
Z₅ is selected from the group consisting of H, C₁₋₁₂ alkyl, -P(=O)(OH)₂,

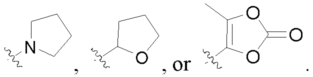

4. The compound or the pharmaceutically acceptable salt thereof according to claim 3, wherein,
A₁ is selected from the group consisting of CH or N;
A₂ is selected from the group consisting of CH₂ or O;
R₁ is selected from the group consisting of H, F, methyl, or methoxy;
R₂ is selected from the group consisting of H, methyl, isopropyl, OH,

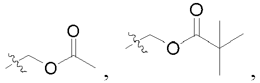

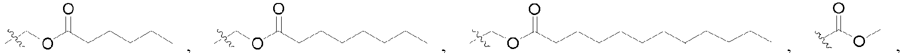

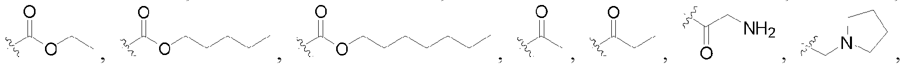

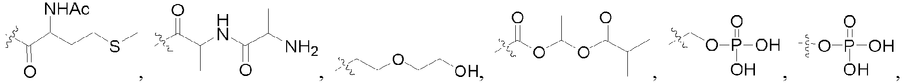

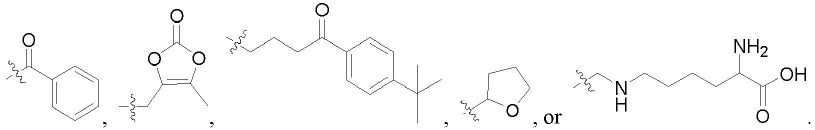

5. A compound of formula (IB), a pharmaceutically acceptable salt thereof or a stereoisomer thereof, 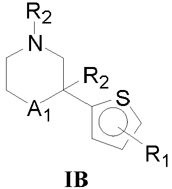 wherein,
(1) A₁ is selected from the group consisting of O or S;
R₁ is selected from the group consisting of F, C₁₋₆ alkoxy, C₃₋₆ cycloalkoxy, C₁₋₆ haloalkoxy, C₃₋₆ cycloalkyl, or C₁₋₆ alkyl-(C=O)-;
each R₂ is independently selected from the group consisting of H or C₁₋₆ alkyl;
or
(2) A₁ is selected from the group consisting of NH;
R₁ is selected from the group consisting of C₁₋₆ alkoxy, C₃₋₆ cycloalkoxy, C₁₋₆ haloalkoxy, C₃₋₆ cycloalkyl, or C₁₋₆ alkyl-(C=O)-;
each R₂ is independently selected from the group consisting of H or C₁₋₆ alkyl;
or
(3) A₁ is selected from the group consisting of CH₂;
R₁ is selected from the group consisting of C₂₋₆ alkoxy, C₃₋₆ cycloalkoxy, C₁₋₆ haloalkoxy, C₃₋₆ cycloalkyl, or C₁₋₆ alkyl-(C=O)-;
each R₂ is independently selected from the group consisting of H or C₁₋₆ alkyl.

6. A compound of formula (IB-1) or (IB-2), or a pharmaceutically acceptable salt thereof, 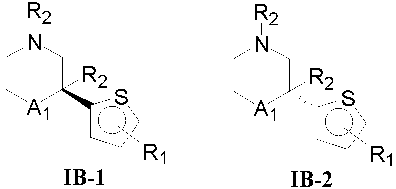 wherein,
(1) A₁ is selected from the group consisting of O or S;
R₁ is selected from the group consisting of F, methoxy, ethoxy, n-propoxy, isopropoxy, cyclopropoxy, cyclobutoxy, fluoromethoxy, chloromethoxy, difluoromethoxy, dichloromethoxy, trifluoromethoxy, trichloromethoxy, 2-fluoroethoxy, 2,2-difluoroethoxy, 2,2-dichloroethoxy, 2,2,2-trifluoroethoxy, 2,2,2-trichloroethoxy, pentafluoroethoxy, pentachloroethoxy, cyclopropyl, cyclobutyl, cyclopentyl, cyclohexyl, methyl-(C=O)-, or ethyl-(C=O)-;
each R₂ is independently selected from the group consisting of H or methyl;
or
(2) A₁ is selected from the group consisting of NH;
R₁ is selected from the group consisting of methoxy, ethoxy, n-propoxy, isopropoxy, cyclopropoxy, cyclobutoxy, fluoromethoxy, chloromethoxy, difluoromethoxy, dichloromethoxy, trifluoromethoxy, trichloromethoxy, 2-fluoroethoxy, 2,2-difluoroethoxy, 2,2-dichloroethoxy, 2,2,2-trifluoroethoxy, 2,2,2-trichloroethoxy, pentafluoroethoxy, pentachloroethoxy, cyclopropyl, cyclobutyl, cyclopentyl, cyclohexyl, methyl-(C=O)-, or ethyl-(C=O)-;
each R₂ is independently selected from the group consisting of H or methyl;
or
(3) A₁ is selected from the group consisting of CH₂;
R₁ is selected from the group consisting of ethoxy, n-propoxy, isopropoxy, cyclopropoxy, cyclobutoxy, fluoromethoxy, chloromethoxy, difluoromethoxy, dichloromethoxy, trifluoromethoxy, trichloromethoxy, 2-difluoroethoxy, 2,2-difluoroethoxy, 2,2-dichloroethoxy, 2,2,2-trifluoroethoxy, 2,2,2-trichloroethoxy, pentafluoroethoxy, pentachloroethoxy, cyclopropyl, cyclobutyl, cyclopentyl, cyclohexyl, methyl-(C=O)-, or ethyl-(C=O)-;
each R₂ is independently selected from the group consisting of H or methyl.

7. A compound of formula (II), a pharmaceutically acceptable salt thereof or a stereoisomer thereof, 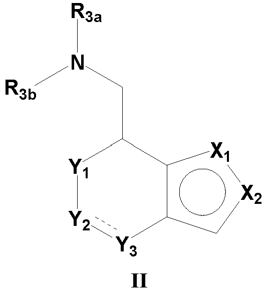 wherein,
X₁ is selected from the group consisting of S or O, and X₂ is selected from the group consisting of CH, or X₂ is selected from the group consisting of S or O, and X₁ is selected from the group consisting of CH;
(1) Y₁ is selected from the group consisting of O; 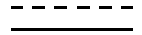
optionally represents a single bond or a double bond; when 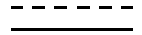
represents a single bond, Y₂ is selected from the group consisting of C(R₅ₐR_{5b}), and Y₃ is selected from the group consisting of C(R₆ₐR_{6b}); when 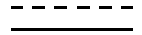 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of C₁₋₆ alkyl, or R₃ₐ and R_{3b} are both selected from the group consisting of C₁₋₆ alkyl;
R₅ₐ, R_{5b}, and R₆ₐ are independently selected from the group consisting of H, C₁₋₆ alkyl, C₁₋₃ alkoxy C₁₋₃ alkyl, or C₆₋₁₀ aryl, R_{6b} is independently selected from the group consisting of H, C₂₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O), and at least one of R₅ₐ, R_{5b}, R₆ₐ, and R_{6b} is not hydrogen;
or R₅ₐ and R_{5b} are independently selected from the group consisting of H, C₁₋₆ alkyl, C₁₋₃ alkoxy C₁₋₃ alkyl, or C₆₋₁₀ aryl, and R₆ₐ and R_{6b} are both selected from the group consisting of C₁₋₆ alkyl;
or R₅ₐ and R_{5b} are both selected from the group consisting of C₁₋₆ alkyl, and R₆ₐ and R_{6b} are independently selected from the group consisting of H, C₁₋₆ alkyl, C₁₋₃ alkoxy C₁₋₃ alkyl, or C₆₋₁₀ aryl;
or
(2) Y₁ is selected from the group consisting of NR₄ or S; 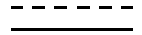
optionally represents a single bond or a double bond; when 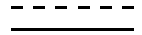 represents a single bond, Y₂ is selected from the group consisting of C(R₅ₐR_{5b}), and Y₃ is selected from the group consisting of C(R₆ₐR_{6b}); when 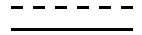 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of C₁₋₆ alkyl;
R₄ is independently selected from the group consisting of H or C₁₋₆ alkyl;
R₅ₐ, R_{5b}, R₆ₐ, and R_{6b} are independently selected from the group consisting of H, C₁₋₆ alkyl, C₁₋₃ alkoxy C₁. ₃ alkyl, or C₆₋₁₀ aryl, or R₅ₐ and R_{5b}, and R₆ₐ and R_{6b} together form oxo (-C=O);
or
(3) Y₃ is selected from the group consisting of O, NR₆, or S; 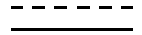
represents a single bond; Y₁ is selected from the group consisting of C(R₄ₐR_{4b}) or Y₁ is absent, and Y₂ is selected from the group consisting of C(R₅ₐR_{5b});
R₃ₐ and R_{3b} are independently selected from the group consisting of H or C₁₋₆ alkyl;
R₄ₐ, R_{4b}, R₅ₐ, and R_{5b} are independently selected from the group consisting of H, C₁₋₆ alkyl, C₁₋₃ alkoxy C₁₋₃ alkyl, or C₆₋₁₀ aryl, or R₄ₐ and R_{4b} together form oxo (-C=O), and at least one of R₄ₐ, R_{4b}, R₅ₐ, and R_{5b} is not hydrogen;
R₆ is independently selected from the group consisting of H or C₁₋₆ alkyl.

8. A compound of formula (IIA) or (IIB), or a pharmaceutically acceptable salt thereof, wherein 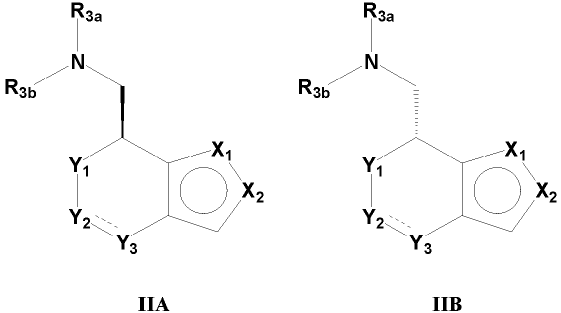 X₁ is selected from the group consisting of S or O, and X₂ is selected from the group consisting of CH, or X₂ is selected from the group consisting of S or O, and X₁ is selected from the group consisting of CH;
(1) Y₁ is selected from the group consisting of O; 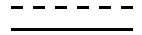
optionally represents a single bond or a double bond; when 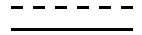 represents a single bond, Y₂ is selected from the group consisting of C(R₅ₐR_{5b}), and Y₃ is selected from the group consisting of C(R₆ₐR_{6b}), when 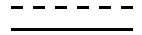 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of C₁₋₆ alkyl, or R₃ₐ and R_{3b} are both selected from the group consisting of C₁₋₆ alkyl;
R₅ₐ, R_{5b}, and R₆ₐ are independently selected from the group consisting of H, C₁₋₆ alkyl, C₁₋₃ alkoxy C₁₋₃ alkyl, or C₆₋₁₀ aryl, R_{6b} is independently selected from the group consisting of H, C₂₋₆ alkyl, C₁₋₃ alkoxy C₁. ₃ alkyl, or C₆₋₁₀ aryl, or R₅ₐ and R_{5b}, and R₆ₐ and R_{6b} together form oxo (-C=O), and at least one of R₅ₐ, R_{5b}, R₆ₐ, and R_{6b} is not hydrogen;
or R₅ₐ and R_{5b} are independently selected from the group consisting of H, C₁₋₆ alkyl, C₁₋₃ alkoxy C₁₋₃ alkyl, or C₆₋₁₀ aryl, and R₆ₐ and R_{6b} are both selected from the group consisting of C₁₋₆ alkyl;
or R₅ₐ and R_{5b} are both selected from the group consisting of C_{1 -6} alkyl, and R₆ₐ and R_{6b} are independently selected from the group consisting of H, C₁₋₆ alkyl, C₁₋₃ alkoxy C₁₋₃ alkyl, or C₆₋₁₀ aryl;
or
(2) Y₁ is selected from the group consisting of NR₄ or S; 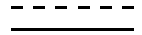
optionally represents a single bond or a double bond; when 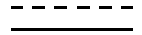 represents a single bond, Y₂ is selected from the group consisting of C(R₅ₐR_{5b}), and Y₃ is selected from the group consisting of C(R₆ₐR_{6b}); when 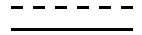 represents a double bond, Y₂ is selected from the group consisting of CR₅ₐ, and Y₃ is selected from the group consisting of CR₆ₐ;
one of R₃ₐ and R_{3b} is selected from the group consisting of H, and the other is selected from the group consisting of C₁₋₆ alkyl;
R₄ is independently selected from the group consisting of H or C₁₋₆ alkyl;
R₅ₐ, R_{5b}, R₆ₐ, and R_{6b} are independently selected from the group consisting of H, C₁₋₆ alkyl, C₁₋₃ alkoxy C₁₋₃ alkyl, or C₆₋₁₀ aryl, or R₅ₐ and R_{5b}, and R₆ₐ and R_{6b} together form oxo (-C=O);
or
(3) Y₃ is selected from the group consisting of O, NR₆, or S; 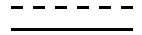
represents a single bond; Y₁ is selected from the group consisting of C(R₄ₐR_{4b}) or Y₁ is absent, and Y₂ is selected from the group consisting of C(R₅ₐR_{5b});
R₃ₐ and R_{3b} are independently selected from the group consisting of H or C₁₋₆ alkyl;
R₄ₐ, R_{4b}, R₅ₐ, and R_{5b} are independently selected from the group consisting of H, C₁₋₆ alkyl, C₁₋₃ alkoxy C₁₋₃ alkyl, or C₆₋₁₀ aryl, or R₄ₐ and R_{4b} together form oxo (-C=O), and at least one of R₄ₐ, R_{4b}, R₅ₐ, and R_{5b} is not hydrogen;
R₆ is independently selected from the group consisting of H or C₁₋₆ alkyl.

9. A compound of formula (III), a pharmaceutically acceptable salt thereof or a stereoisomer thereof, 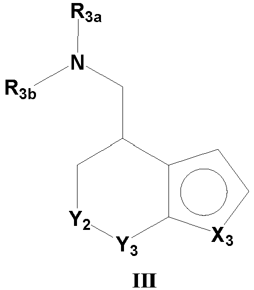 wherein,
X₃ is selected from the group consisting of S or O;
Y₂ is selected from the group consisting of C(R₅ₐR_{5b}), and Y₃ is selected from the group consisting of O, NR₆, or S;
R₃ₐ and R_{3b} are independently selected from the group consisting of H or C₁₋₆ alkyl;
R₅ₐ is independently selected from the group consisting of H, C₁₋₆ alkyl, C₁₋₃ alkoxy-C₁₋₃ alkyl, or C₆₋₁₀ aryl, R_{5b} is independently selected from the group consisting of C₁₋₆ alkyl, C₁₋₃ alkoxy C₁₋₃ alkyl, or C₆₋₁₀ aryl, or R₅ₐ and R_{5b} together form oxo (-C=O);
R₆ is independently selected from the group consisting of H or C₁₋₆ alkyl.

10. A compound of formula (IIIA) or (IIIB), or a pharmaceutically acceptable salt thereof, 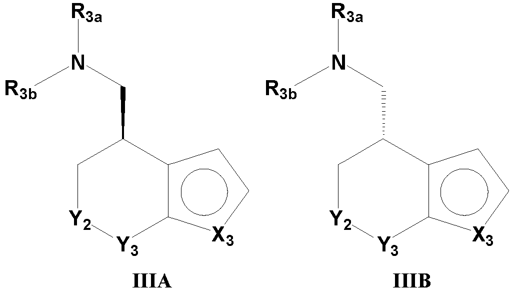 wherein,
X₃ is selected from the group consisting of S or O;
Y₂ is selected from the group consisting of C(R₅ₐR_{5b}), and Y₃ is selected from the group consisting of O, NR₆, or S;
R₃ₐ and R_{3b} are independently selected from the group consisting of H or C₁₋₆ alkyl;
R₅ₐ is independently selected from the group consisting of H, C₁₋₆ alkyl, C₁₋₃ alkoxy-C₁₋₃ alkyl, or C₆₋₁₀ aryl, R_{5b} is independently selected from the group consisting of C₁₋₆ alkyl, C₁₋₃ alkoxy C₁₋₃ alkyl, or C₆₋₁₀ aryl, or R₅ₐ and R_{5b} together form oxo (-C=O);
R₆ is independently selected from the group consisting of H or C₁₋₆ alkyl.

11. The compound or the pharmaceutically acceptable salt thereof or the stereoisomer thereof according to any one of claims 7-10,
wherein,
one of R₃ₐ and R_{3b} is selected from the group consisting of H, and the other is selected from the group consisting of methyl, or R₃ₐ and R_{3b} are both selected from the group consisting of methyl;
R₄ is independently selected from the group consisting of H or methyl;
R₄ₐ, R_{4b}, R₅ₐ, R_{5b}, and R₆ₐ are independently selected from the group consisting of H, methyl, ethyl, n-propyl, isopropyl, methoxymethyl, and phenyl, R_{6b} is independently selected from the group consisting of H, ethyl, n-propyl, isopropyl, methoxymethyl, or phenyl, or R₄ₐ and R_{4b}, R₅ₐ and R_{5b}, and R₆ₐ and R_{6b} together form oxo (-C=O);
R₆ is independently selected from the group consisting of H or methyl.

12. A compound, a pharmaceutically acceptable salt thereof or a stereoisomer thereof, selected from the group consisting of:

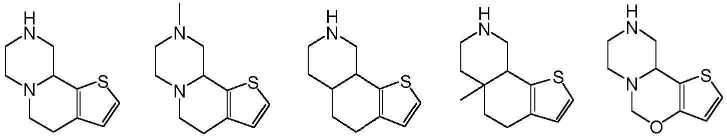

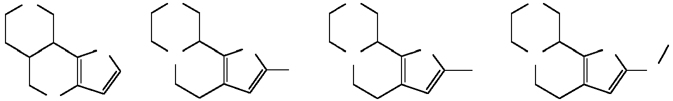

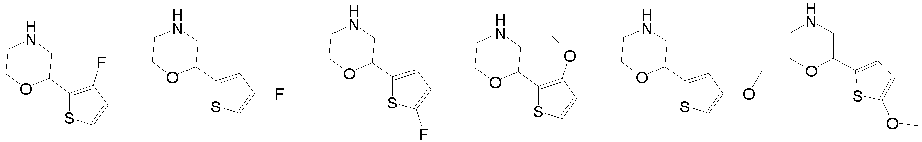

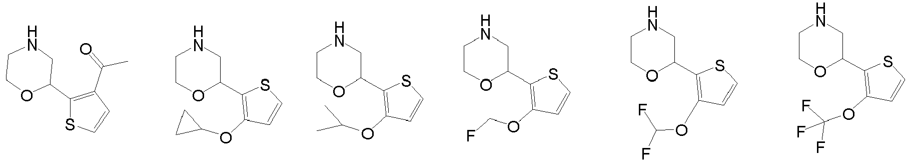

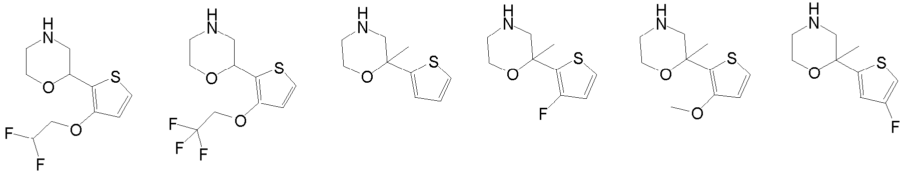

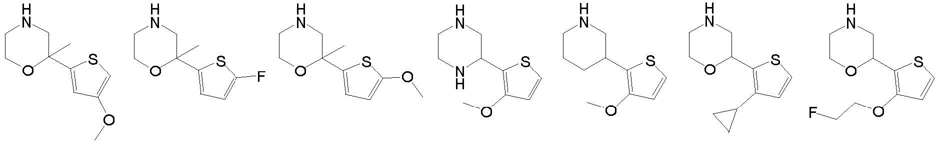

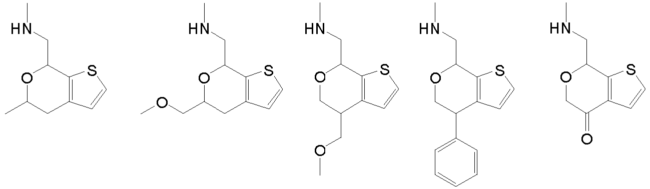

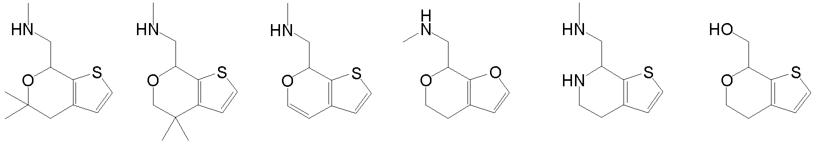

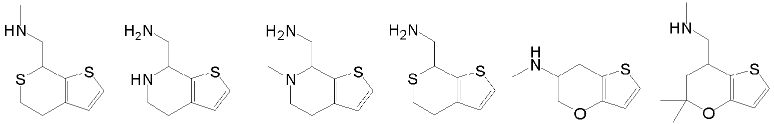

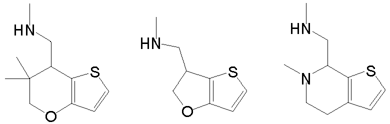

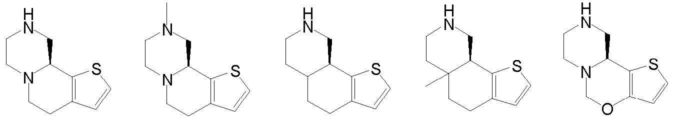

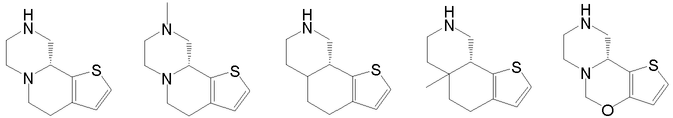

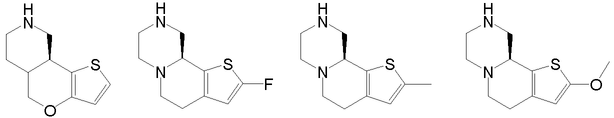

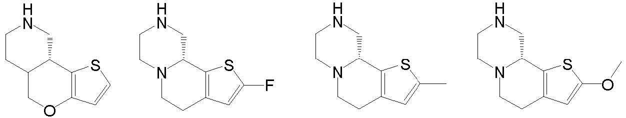

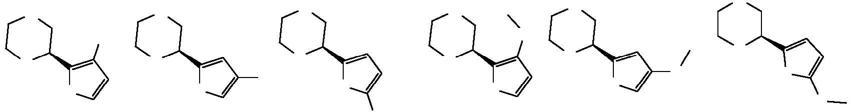

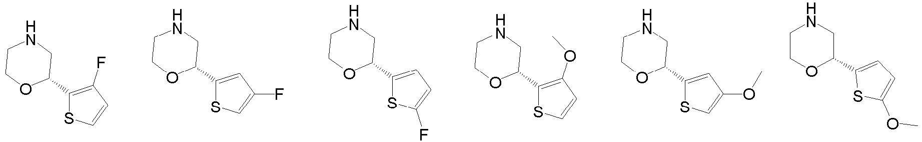

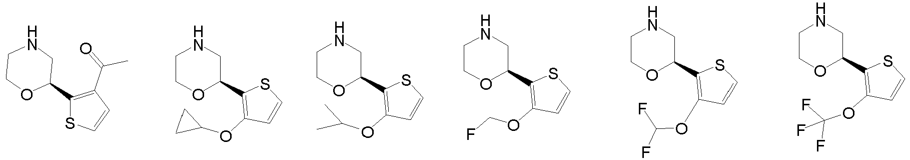

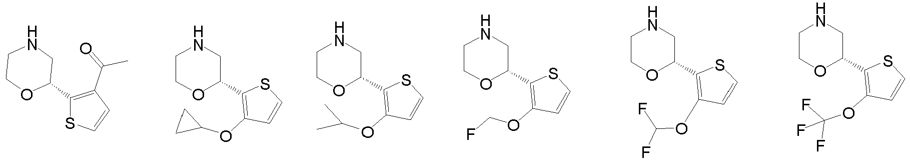

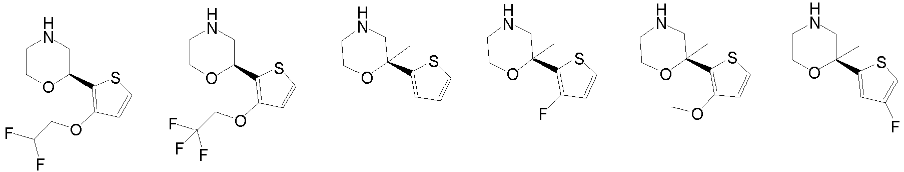

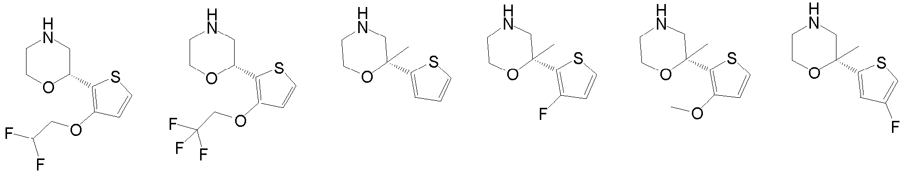

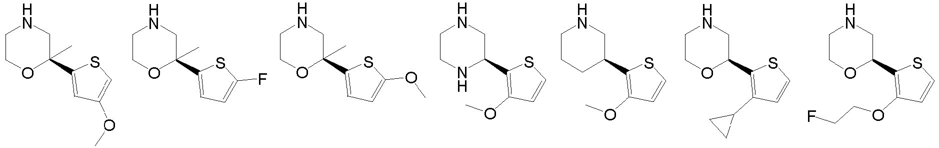

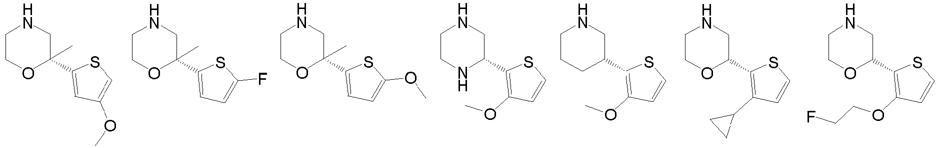

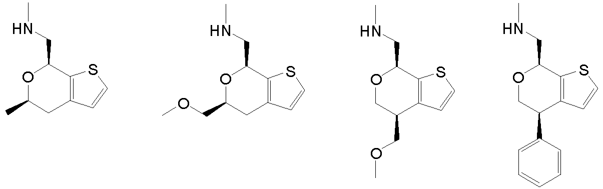

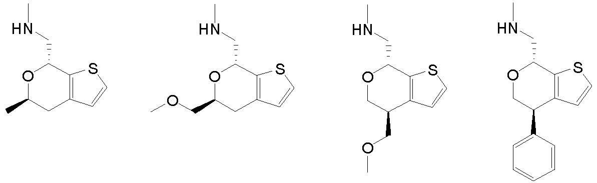

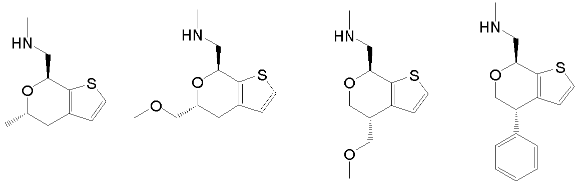

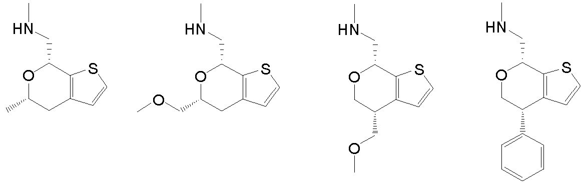

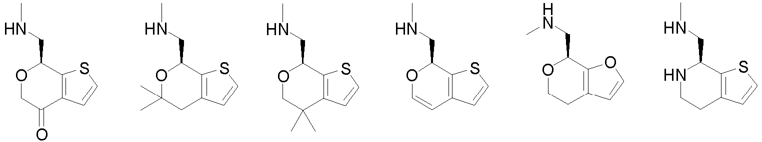

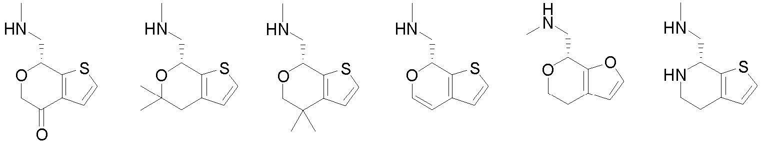

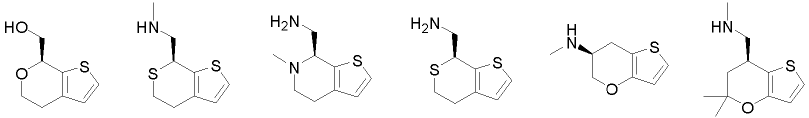

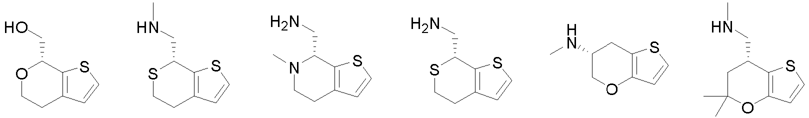

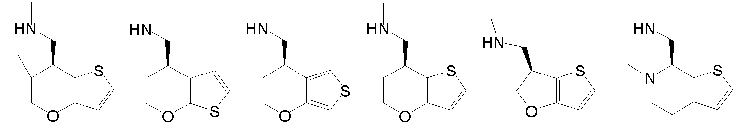

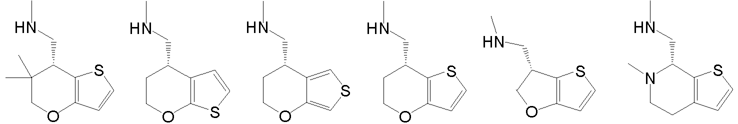

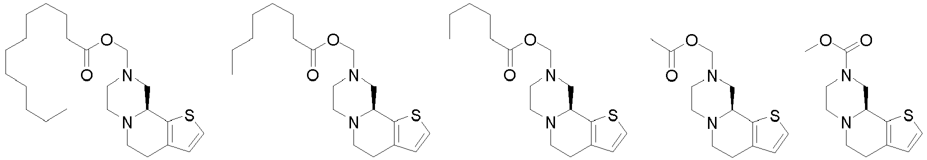

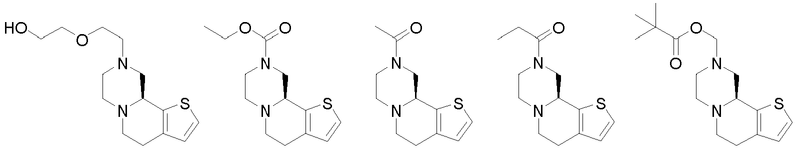

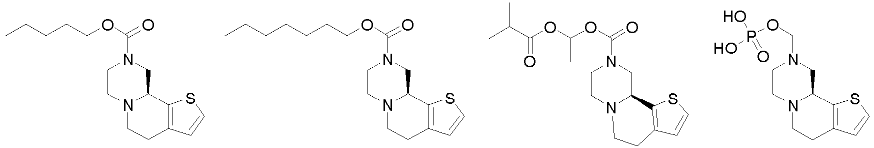

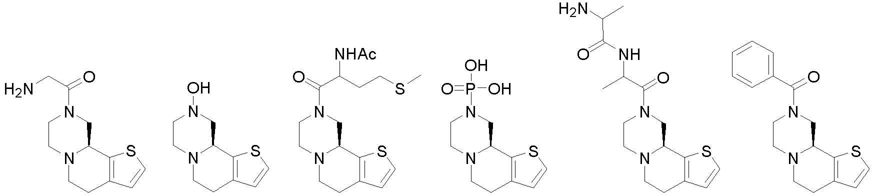

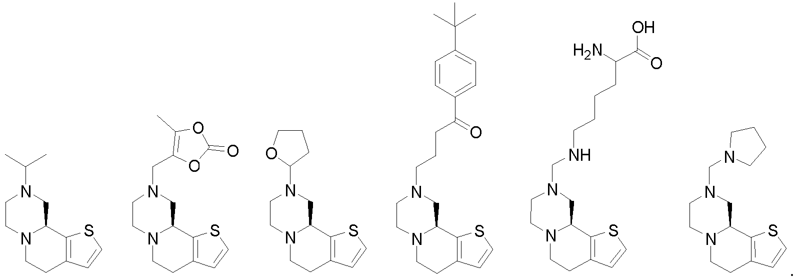

13. A pharmaceutical composition, comprising the compound, the pharmaceutically acceptable salt thereof or the stereoisomer thereof according to any one of claims 1-12, and a pharmaceutically acceptable carrier.

14. Use of the compound, the pharmaceutically acceptable salt thereof or the stereoisomer thereof according to any one of claims 1-12 or the pharmaceutical composition according to claim 13 in the preparation of a TAAR1 agonist medicament.

15. Use of the compound, the pharmaceutically acceptable salt thereof or the stereoisomer thereof according to any one of claims 1-12 or the pharmaceutical composition according to claim 13 in the preparation of a medicament for treating, preventing and/or controlling central nervous system (CNS)-related diseases or symptoms.

16. The use according to claim 15, wherein the central nervous system (CNS) diseases or symptoms comprise: schizophrenia, schizophrenia spectrum disorder, acute schizophrenia, chronic schizophrenia, NOS schizophrenia, psychotic disorder, schizoid personality disorder, schizotypal personality disorder, delusional disorder, psychosis, mental disorder, brief psychotic disorder, shared psychotic disorder, psychotic disorder due to a physical disease, drug-induced psychosis, psychoaffective disorder, aggressive delirium, Parkinson psychosis, excitative psychosis, Tourette syndrome, organic or NOS psychosis, epilepsy, epileptic seizures, agitation, post-traumatic stress disorder, behavioral disorder, neurodegenerative disease, Alzheimer disease, Parkinson's disease, dyskinesias, Huntington disease, dementia, affective disorder, anxiety, affective psychosis, obsessive compulsive disorder, vertigo, pain, fibromyalgia, migraine, cognitive impairment, movement disorder, restless leg syndrome (RLS), multiple sclerosis, substance abuse, and stress related disorder.

17. The use according to claim 16, wherein the affective psychosis comprises: depression, major depressive disorder and dysthymia, bipolar disorder, bipolar depression, manic disorder, seasonal affective psychosis, attention deficit disorder (ADD), and attention deficit hyperactivity disorder (ADHD); the pain comprises: neuropathic pain, neuropathic pain susceptibility state, and inflammatory pain; the stress related disorder comprises: acute stress disorder, post-traumatic stress disorder, and adjustment disorder.

18. Use of the compound, the pharmaceutically acceptable salt thereof or the stereoisomer thereof according to any one of claims 1-12 or the pharmaceutical composition according to claim 13 in the preparation of a medicament for treating, preventing and/or controlling cardiovascular or metabolic diseases.

19. The use according to claim 18, wherein the cardiovascular or metabolic diseases comprise: diabetes, diabetic complications, obesity, dyslipidemia, and hypertension.
